# EUROPEAN PATENT SPECIFICATION

(11) **EP 3 217 787 B1**
(45) Date of publication and mention of the grant of the patent: **17.04.2019**
(21) Application number: 15798303.2
(22) Date of filing: 10.11.2015
(51) Int. Cl.: A01K 67/027, C12N 15/85, C07K 16/18, C07K 14/775, A61K 49/00

(54) **ANIMAL MODEL FOR NEPHROPATHY AND AGENTS FOR TREATING THE SAME**
TIERMODELL FÜR NEPHROPATHIE UND MITTEL ZUR BEHANDLUNG DAVON
MODÈLE ANIMAL POUR LA NÉPHROPATHIE ET AGENTS DE TRAITEMENT CORRESPONDANTS

(30) Priority: 10.11.2014 US 201462077774 P
(43) Date of publication of application: 20.09.2017
(60) Divisional application: 19152929.6
(73) Proprietor: F. Hoffmann-La Roche AG, 4070 Basel (CH)
(72) Inventor: WILSON, Deanna Grant, South San Francisco, California 94080 (US); FOREMAN, Oded, South San Francisco, California 94080 (US); PETERSON, Andrew, South San Francisco, California 94080 (US); WEN, Xiaohui, South San Francisco, California 94080 (US); SCALES, Suzanna J., South San Francisco, California 94080 (US)
(74) Representative: Brodbeck, Michel
(86) International application number: PCT/US2015/059987
(87) International publication number: WO 2016/077369

(56) References cited:
- WO-A1-2011/020865
- WO-A2-2012/162394
- US-A1- 2011 030 078
- THOMSON R ET AL: "Evolution of the primate trypanolytic factor APOL1", PROCEEDINGS OF THE NATIONAL ACADEMY OF SCIENCES, vol. 111, no. 20, 7 May 2014 (2014-05-07), pages E2130-E2139, XP055243608, US ISSN: 0027-8424, DOI: 10.1073/pnas.1400699111
- MOLINA-PORTELA M P ET AL: "Distinct roles of apolipoprotein components within the trypanosome lytic factor complex revealed in a novel transgenic mouse model", THE JOURNAL OF EXPERIMENTAL MEDICINE, ROCKEFELLER UNIVERSITY PRESS, US, vol. 205, no. 8, 1 August 2008 (2008-08-01), pages 1721-1728, XP002584510, ISSN: 0022-1007, DOI: 10.1084/JEM.20071463 [retrieved on 2008-08-04]

## Description

### FIELD OF THE INVENTION

This invention relates to a non-human transgenic animal model for nephropathy and methods for identifying therapeutic agents.

### BACKGROUND

Apolipoprotein L1 (ApoL1) is the only member in a 6-gene family that includes a signal peptide, and with apolipoprotein A1, is secreted into a particularly dense subspecies (HDL₃) of high-density lipoprotein (HDL) particles (Duchateau et al., 1997). ApoL1 is a major component of the human innate immune response against African trypanosomes, *Trypanosoma brucei brucei,* that cause of African sleeping sickness. A subspecies of the trypanosome, *T.b. rhodesiense,* is resistant to wild-type ApoL1 (also referred to as G0). However, two distinct alleles of ApoL1 (termed G1 and G2) which are common in African chromosomes but absent in European chromosomes, confer protection against infection with *T.b. rhodesiense* (see US 7,585,511, US 2012/0128682). The G1 and G2 alleles also increase the risk for developing nephropathy and variants are associated for example with focal segmental glomerulosclerosis (FSGS), Hypertension associated nephropathy (HTN) and HIV associated nephropathy (HIVAN) (see US 2012/0195902, US 2012/0003644). In African Americans FSGS occurs earlier and progresses 4-5 times more rapidly to end-stage renal disease (ESRD) as compared with Caucasians (Hsu et al., 2003; Kopp et al., 2011; Parsa et al., 2013). ApoL1 variants are associated with seven-fold higher odds for "hypertension-attributed" ESRD, regardless of diabetes status (Freedman et al., 2010; Freedman et al., 2011; Parsa et al., 2013), 17-fold higher
odds for idiopathic FSGS (Kopp et al., 2011), and 29-fold higher odds for HIV-associated nephropathy (Kopp et al., 2011). The genetic association is one of the strongest ever reported for a common disease and provides an explanation for the higher rates of nephropathy in African-Americans relative to caucasians. These diseases account for much suffering and lead to expenditures in the tens of billions of dollars in the United States. No targeted therapies to protect filter barrier function and prevent nephropathy are presently available.

Therefore, it would be highly advantageous and desirable to have therapeutic agents for treatment of such diseases. Furthermore, an animal model for nephropathy which is suitable for the use in a method to identify agents as potential therapeutics would be valuable.

### SUMMARY

The invention provides a non-human transgenic animal expressing ApoL1, wherein the non-human transgenic animal is a rodent and wherein the non-human transgenic animal has a nepropathy as well as a method for generating the same. Also provided is a method for identifying an agent capable of reducing the progression of an ApoL1 mediated nephropathy. Furthermore, an isolated antibody is disclosed which binds to the human variants of ApoL1.

In one aspect, the disclosure provides a non-human transgenic animal expressing human ApoL1. In some embodiments, the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1. In some embodiments, the rodent is a mouse. In some embodiments, the non-human transgenic animal has a nephropathy. In some embodiments, the nephropathy is a doxorubicin-induced nephropathy.

In another aspect, the disclosure provides a cell or a tissue derived from the non-human transgenic animal as described herein.

In yet another aspect, the invention provides a method of determining whether an agent is capable of reducing the serum concentration of human ApoL1 the method comprising the steps of measuring the serum concentration of human ApoL1 in a non-human transgenic animal, wherein the non-human transgenic animal is a rodent, administering the agent to the non-human transgenic animal, and measuring the serum concentration of human ApoL1 in the non-human transgenic animal, wherein a reduction in the serum concentration of human ApoL1 in the non-human transgenic animal indicates that the

agent is capable of reducing the serum concentration of human ApoL1. In some embodiments, the non-human transgenic animal is a non-human transgenic animal expressing human ApoL1. In some embodiments, the non-human transgenic animal expresses i) G0 variant of ApoL1 (SEQ ID NO:01), ii) G1 variant of ApoLl (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1. In some embodiments, the rodent is a mouse. In some embodiments, the non-human transgenic animal has a nephropathy. In some embodiments, the nephropathy is a HIV-associated nephropathy. In some embodiments, the nephropathy is a doxorubicin-induced nephropathy.

In yet another aspect, the invention provides a method of identifying an agent capable of reducing the progression of nephropathy the method comprising the steps of inducing a nephropathy in a non-human transgenic animal, wherein the non-human transgenic animal is a rodent, administering the agent to the non-human transgenic animal, and assessing the progression of nephropathy based on the pathological phenotype of the kidneys of the non-human transgenic animal (as described in the Examples), wherein a less advanced nephropathy as compared to non-human transgenic animals not administered with the agent identifies the agent to be capable of reducing the progression of nephropathy. The non-human transgenic animal is a non-human transgenic animal expressing human ApoL1. In some embodiments, the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1. In some embodiments, the rodent is a mouse. In some embodiments, the nephropathy is induced by administration of doxorubicin. In some embodiments, the nephropathy is induced by expressing a transgene containing a portion of the human immunodeficiency virus in the non-human transgenic animal. In some embodiments, the agent binds to i) human G0 variant of ApoL1 (SEQ ID NO:01), ii) human G0 variant of ApoL1 and human G1 variant of ApoL1 (SEQ ID NO:02), iii) human G0 variant of ApoL1 and human G2 variant of ApoL1 (SEQ ID NO:03), or iv) human G0 variant of ApoL1, human G1 variant of ApoL1 and human G2 variant of ApoL1. In some embodiments, the agent is an antibody.

In yet another aspect, the invention provides a method of generating an animal model for nephropathy, the method comprising inducing a nephropathy in a non-human transgenic animal
expressing human ApoL1, wherein the non-human transgenic animal is a rodent. In some embodiments, the nephropathy is induced by expressing a transgene containing a portion of the human immunodeficiency virus in the non-human animal. In some embodiments, the nephropathy is induced by administration of doxorubicin. In some embodiments, doxorubicin is administered at a concentration from 15 mg/kg to 40 mg/kg. In some embodiments, doxorubicin is administered at a concentration from 20 mg/kg to 30 mg/kg. In some embodiments, doxorubicin is administered at a concentration from 24 mg/kg to 26 mg/kg. In some embodiments, doxorubicin is administered at a concentration of 25 mg/kg. In some embodiments, doxorubicin is administered in a single dose. In some embodiments, doxorubicin is administered into the tail vein. In some embodiments, the non-human animal is treated daily with subcutaneous fluids to prevent dehydration. In some embodiments, the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1. In some embodiments, the rodent is a mouse.

In another aspect, the disclosure provides an isolated antibody which binds to the human G0 variant of ApoL1 (SEQ ID NO:01) and to one or both of the human G1 variant of ApoL1 (SEQ ID NO:02) and the human G2 variant of ApoL1 (SEQ ID NO:03). In some embodiments, the antibody is a monoclonal antibody. In some embodiments, the antibody is a human, humanized, or chimeric antibody. In some embodiments, the antibody is a full length IgG1 antibody. In some embodiments, the antibody is capable of blocking multimerization of ApoL1 variants. In some embodiments, the antibody is capable of reducing the serum concentration of human ApoL1. In some embodiments, the antibody is capable of reducing the progression of a nephropathy. In some embodiments, the nephropathy is an ApoL1 mediated nephropathy. In some embodiments, the ApoL1 mediated nephropathy is selected from the group consisting of HIV-associated nephropathy, focal segmental glomerular sclerosis associated nephropathy, sickle cell nephropathy, nephropathy associated with allograft loss following transplantation, and lupus nephritis associated nephropathy. In some embodiments, the ApoL1 mediated nephropathy is hypertension associated nephropathy. In some embodiments, the ApoL1 mediated nephropathy is diabetic nephropathy.

In another aspect, the disclosure provides an isolated nucleic acid encoding the antibody described herein. In yet another aspect, the disclosure provides a host cell comprising the nucleic acid mentioned above. In yet another aspect, the disclosure provides a method of producing an
antibody comprising culturing the host cell mentioned above so that the antibody is produced.

In yet another aspect, the disclosure provides a pharmaceutical formulation comprising the antibody as described herein and a pharmaceutically acceptable carrier. In yet another aspect, the disclosure provides the antibody as described herein for use as a medicament. In yet another aspect, the disclosure provides the use of the antibody described herein in the manufacture of a medicament. In yet another aspect, the disclosure provides a method for treating an individual having a nephropathy by administering to the individual the antibody described herein. In yet another aspect, the disclosure provides a method of reducing the progression of a nephropathy in a subject comprising administering to the subject an effective amount of the antibody described herein.

### BRIEF DESCRIPTION OF THE FIGURES

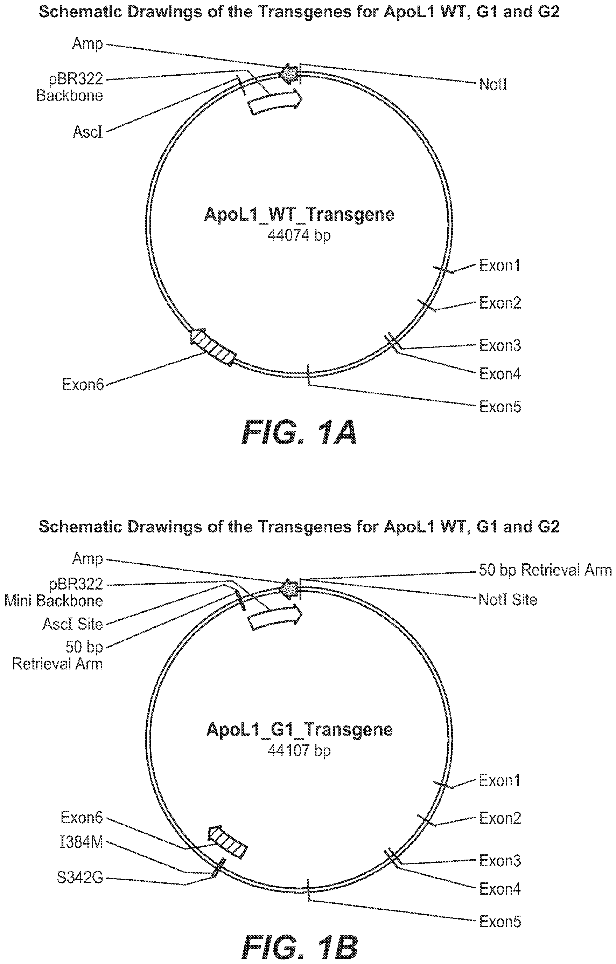

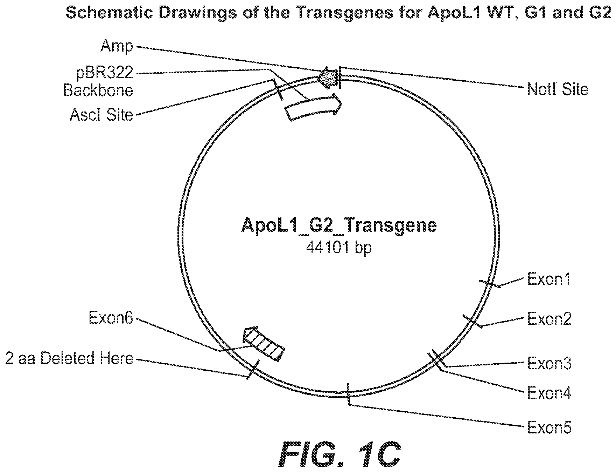

**Figure** 1 shows schematic drawings of the transgenes for G0 variant of ApoL1 (Fig. 1A), G1 variant of ApoL1 (Fig. 1B) and G2 variant of ApoL1 (Fig. 1C).

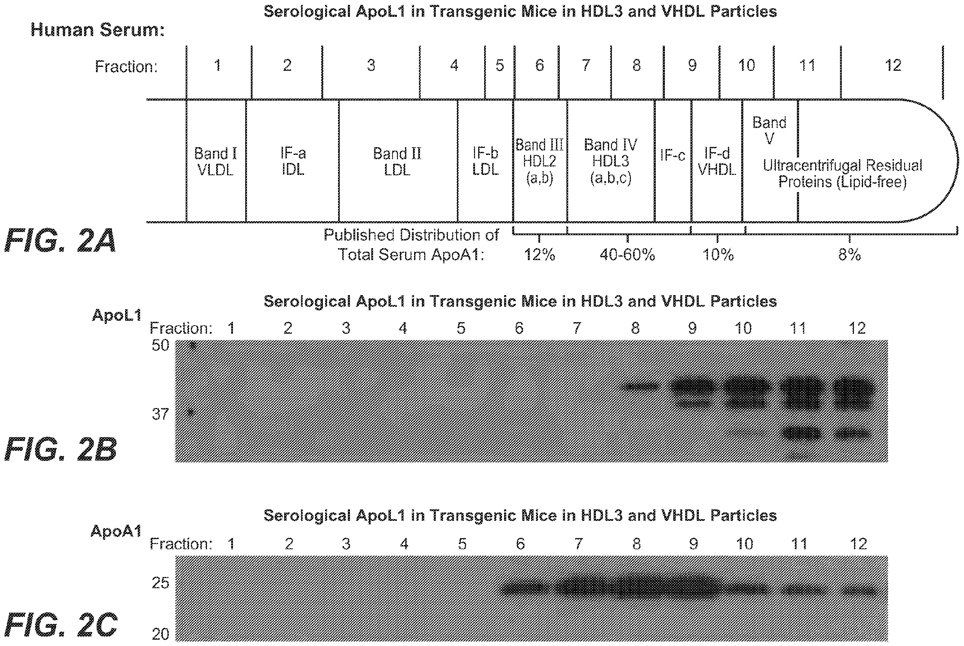

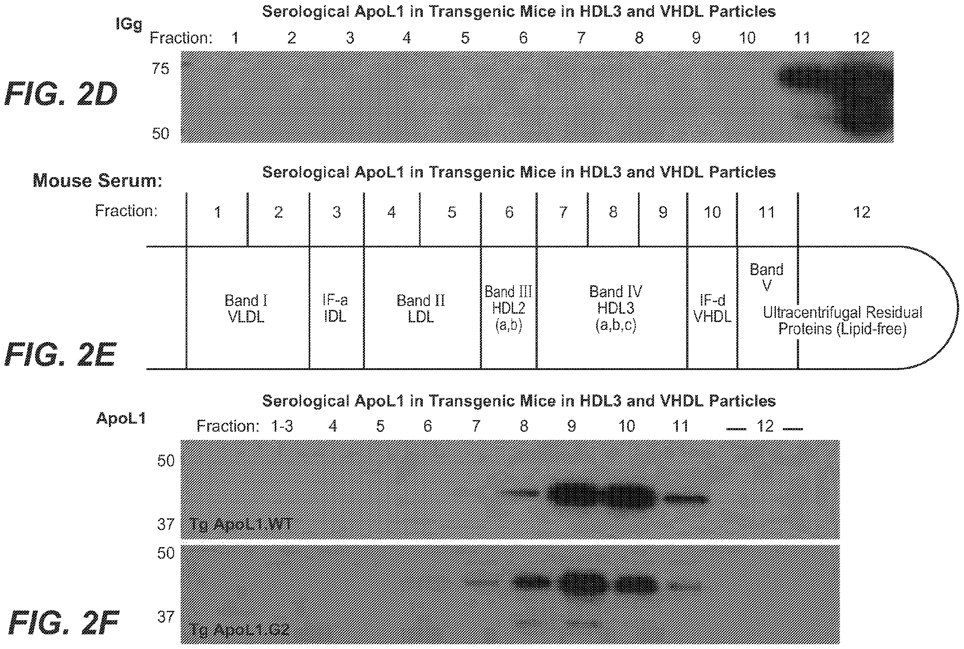

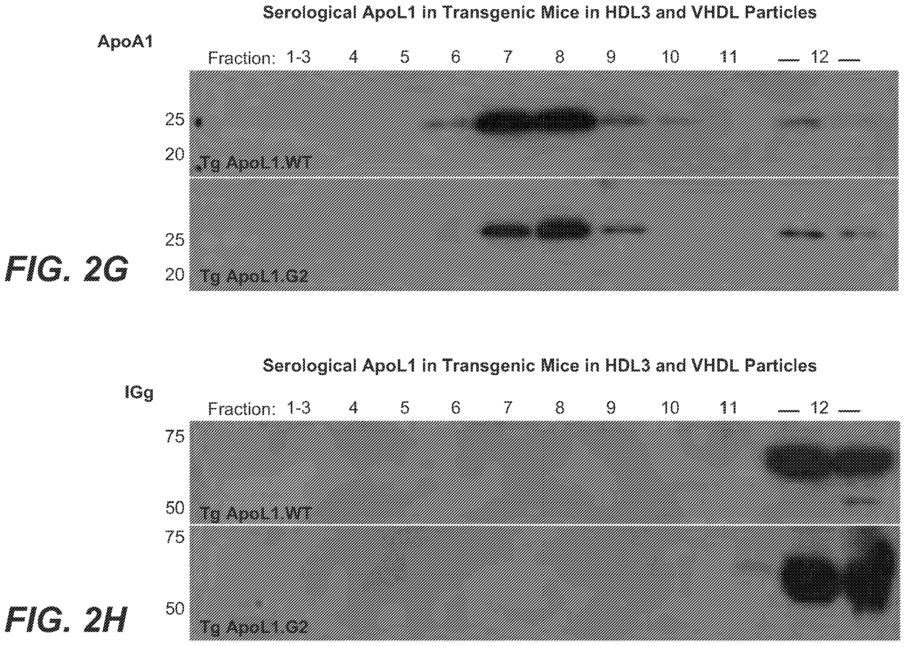

**Figure 2** **(A-H)** shows that the detection of serological ApoL1 in transgenic mice can be detected in HDL3 and VHDL fractions of major lipoprotein classes using ultracentrifugal density gradients. Abbreviations: VLDL is very low density lipoprotein, IF is Intermediate Fractions, HDL is high density lipoprotein, VHDL is very high density lipoprotein.

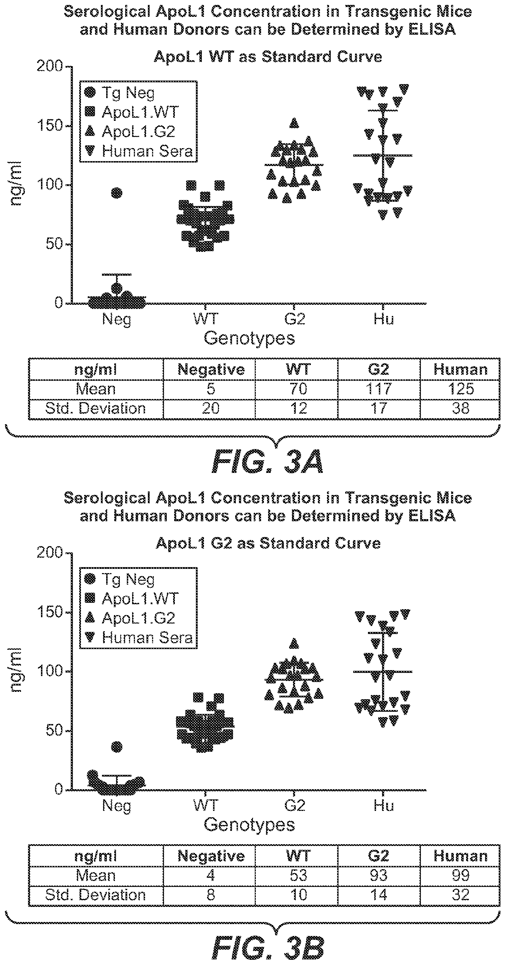

**Figure 3** illustrates the serological ApoL1 concentrations in transgenic mice and human donors determined by ELISA using serum from either G0 variant of ApoL1 (wt) (Fig. 3A) or the G2 variant of ApoL1 (Fig. 3B) for the standard curve.

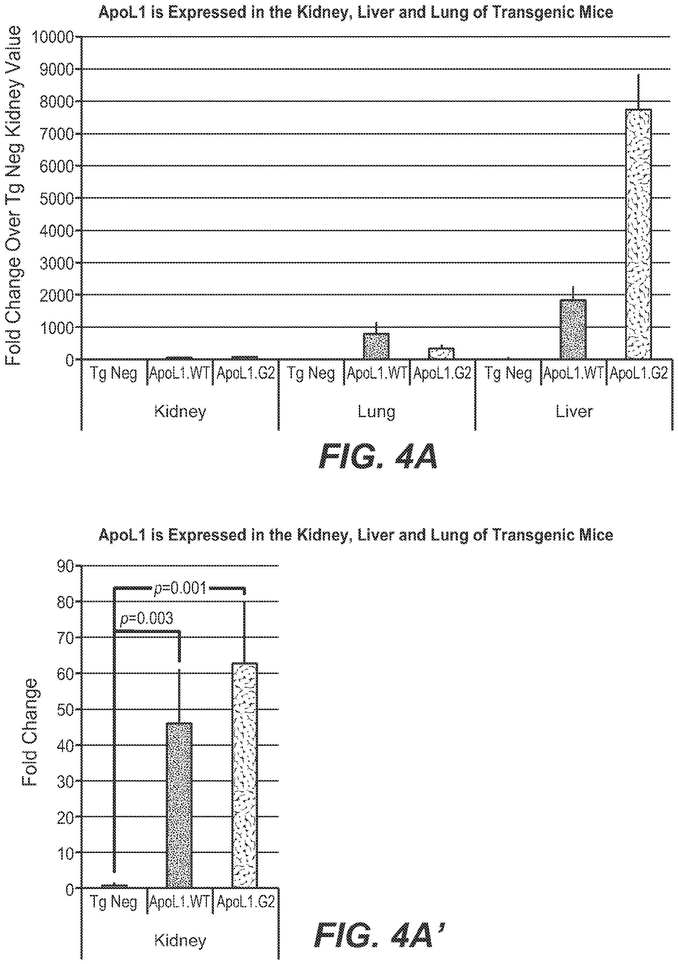

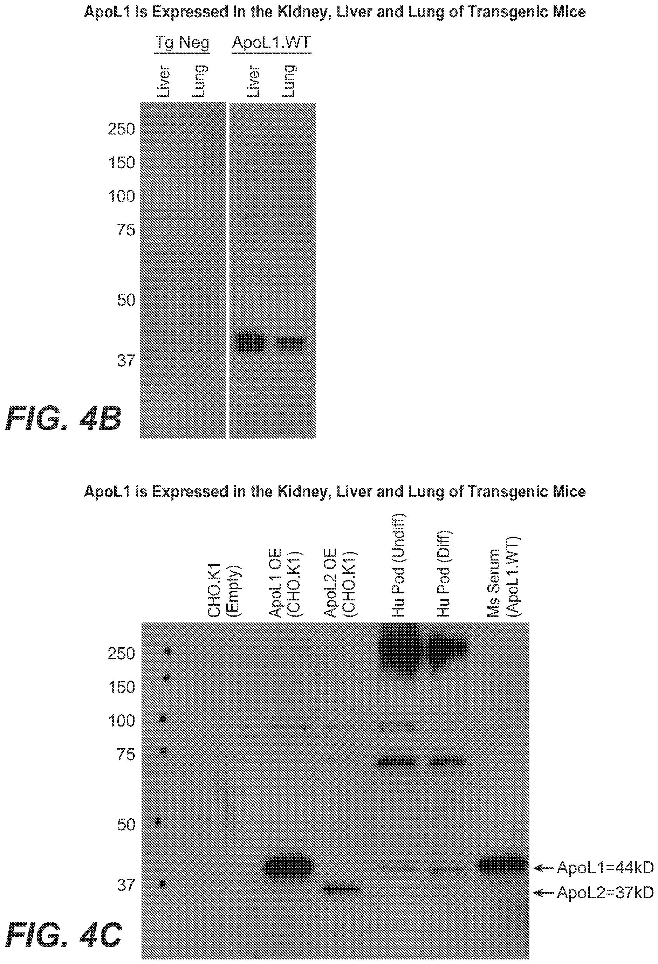

**Figure 4** shows the expression of ApoL1 in kidney, liver, and lung as determined by quantitative PCR (Fig. 4A, Fig. 4A'). Western blots were performed using a polyclonal antibody to ApoL1 on homogenates of liver and lung from PBS perfused mice (Fig. 4B). Western blots were performed using a rabbit polyclonal antibody using lysates of ApoL1, ApoL2, or control transient transfections in CHO-1K cells (Fig. 4C).

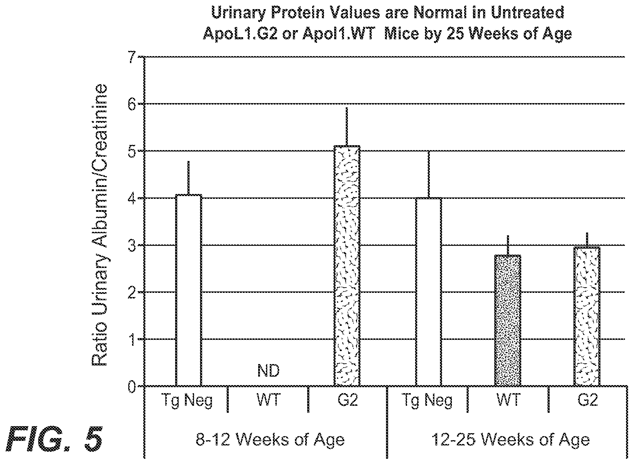

**Figure 5** shows the urinary protein values in untreated transgenic negative mice and mice expressing mice the G0 variant of ApoL1 (wt) and the G2 variant of ApoL1.

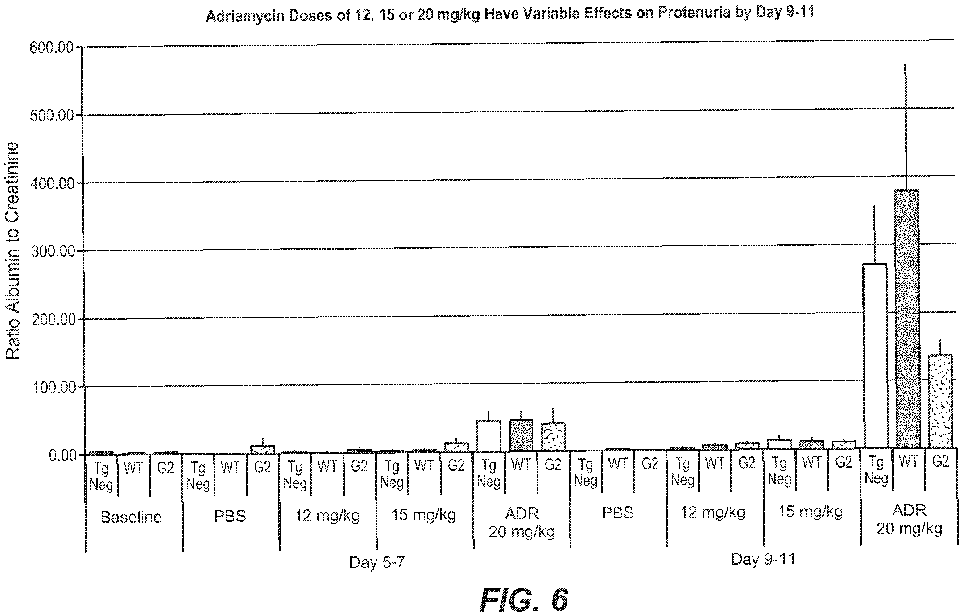

**Figure 6** illustrates the effects of different doxorubicin doses on urinary protein by days 5-7 and 9-11, respectively (n = 3-5 per genotype and means are ± SEM).

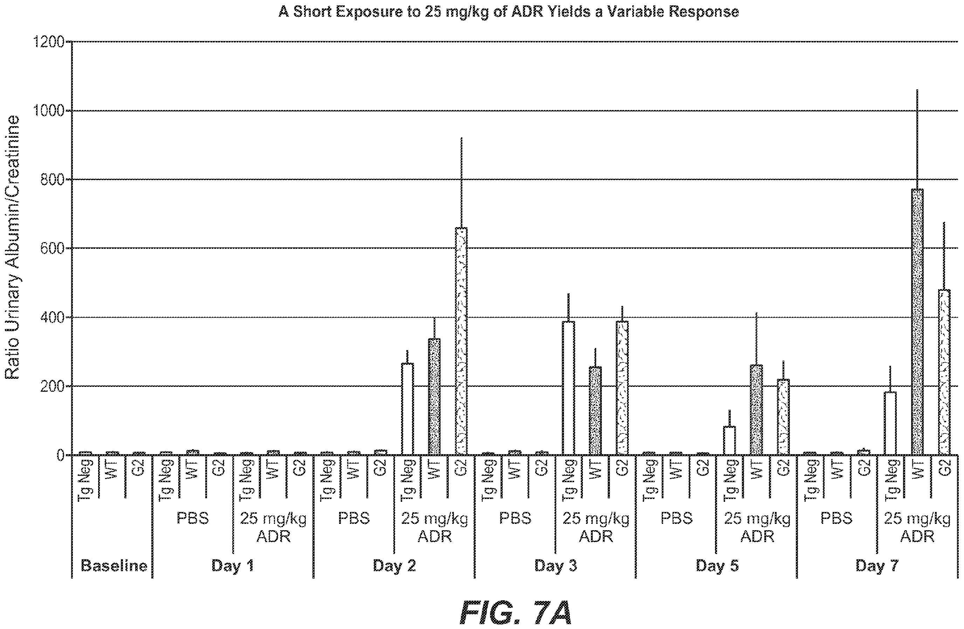

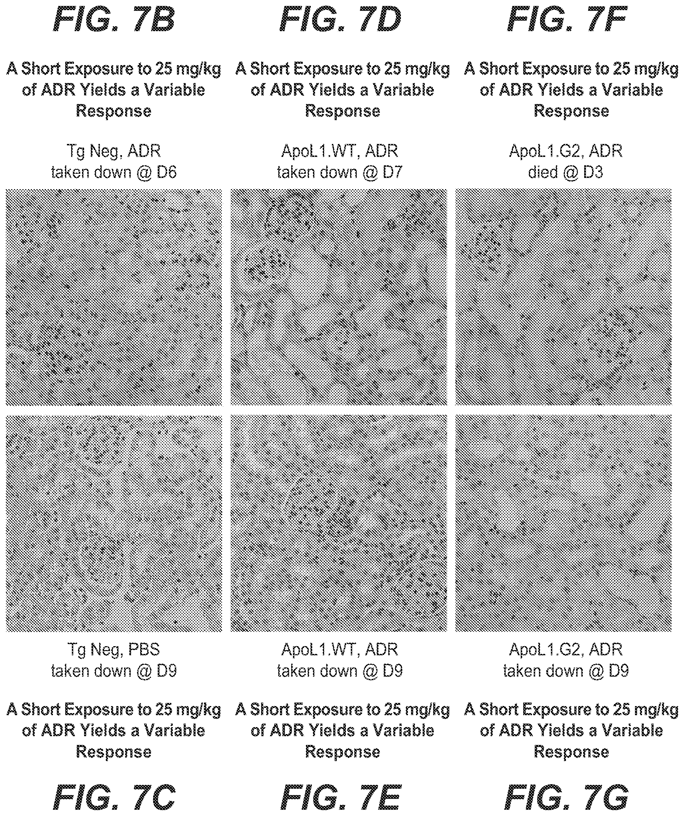

**Figure 7** illustrates the effect of a short exposure of up to 7 days to 25 mg/kg of doxorubicin (Fig. 7A, n = 3-6 per genotype treated with 25mg/kg doxorubicin, and means are ± SEM. P-values refer to a two-tailed t test with equal variance). H&E stained kidney tissue of transgenic negative animals treated with doxorubicin (Fig. 7B), transgenic negative animals treated with PBS (Fig. 7C), transgenic mice expressing G0 variant of ApoL1 (wt) (Fig. 7D, Fig. 7E) or G2 variant of ApoL1 (Fig. 7F, Fig. 7G).

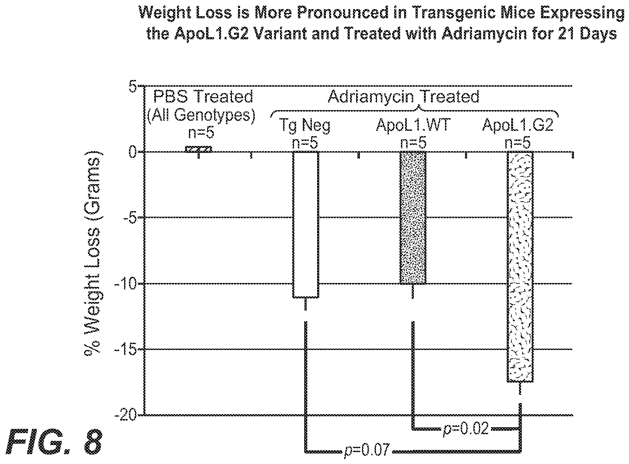

**Figure 8** shows weight loss in doxorubicin treated transgenic mice expressing the ApoL1 G2 variant after 21 days (n = 5 per genotype, and means are ± SEM, P-values refer to a two-tailed t test with equal variance).

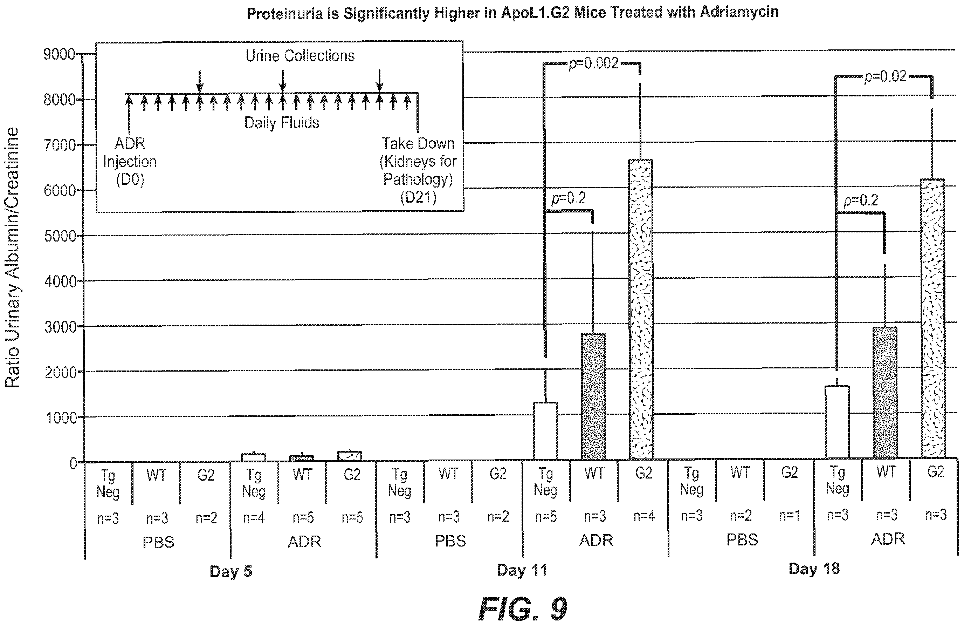

**Figure 9** shows proteinuria in mice expressing G2 variant of ApoL1 treated with doxorubicin. Albumin concentrations are normalized to creatinine levels (n = 3-5 per genotype treated with 25mg/kg doxorubicin (Adriamycin®), and means are ± SEM. P-values refer to a two-tailed t test with equal variance).

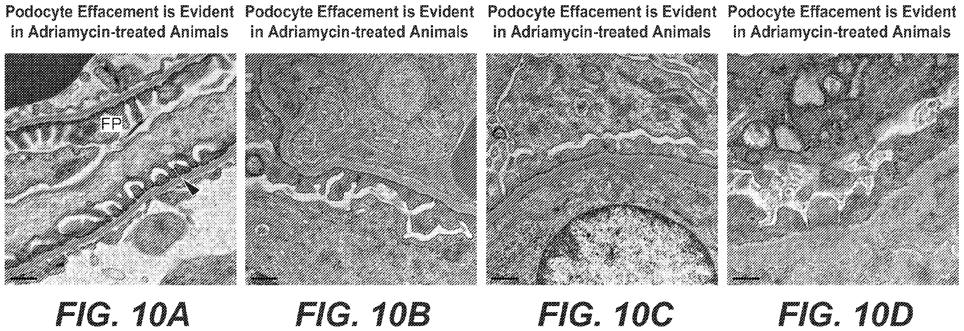

**Figure 10** shows transmission electron microscopy images at 5000x of podocytes in PBS-treated mice (Fig. 10A), doxorubicin-treated transgenic negative mice (Fig. 10B), mice expressing G0 variant of ApoL1 (Fig. 10C) and mice G2 variant of ApoL1 (Fig. 10D). Further depicted are multiple foot processes (FP) and intervening slit diaphragms (arrowhead).

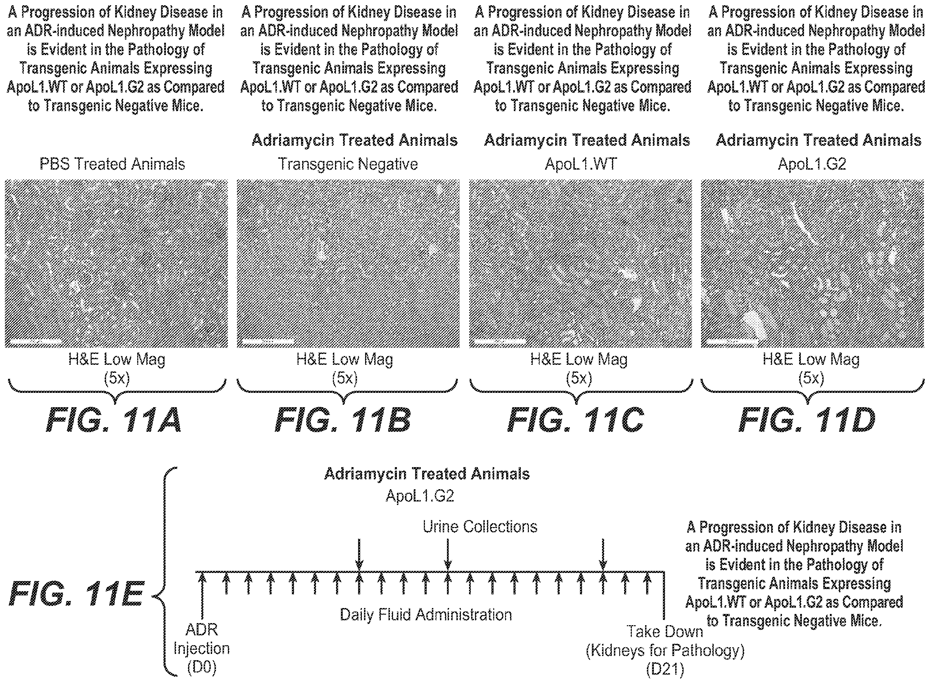

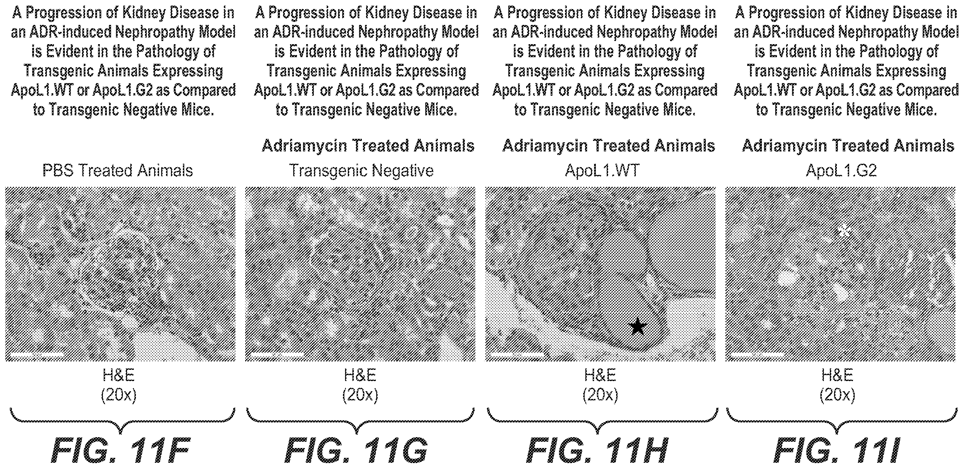

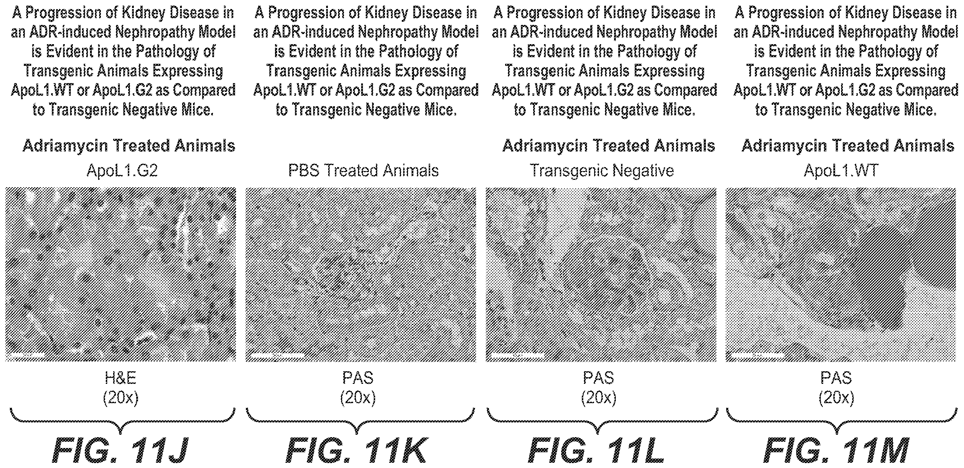

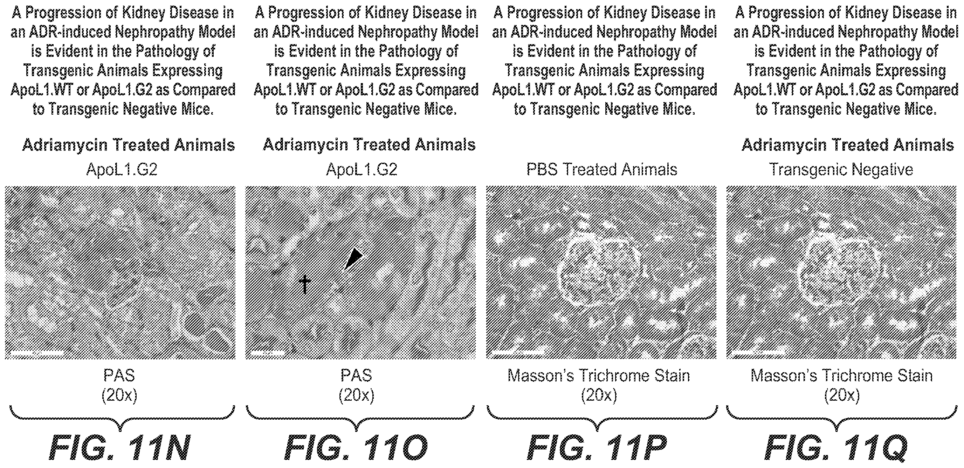

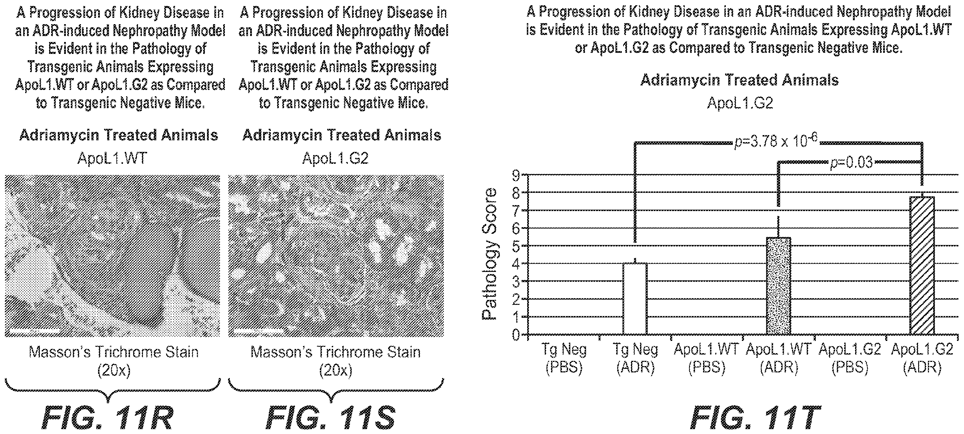

**Figure 11** **(A-T)** shows progression in kidney damage in different magnifications (5x, 20x) and different stainings (H&E, PAS, Masson's Trichrome stain) in transgenic animals treated with doxorubicin or PBS, respectively.

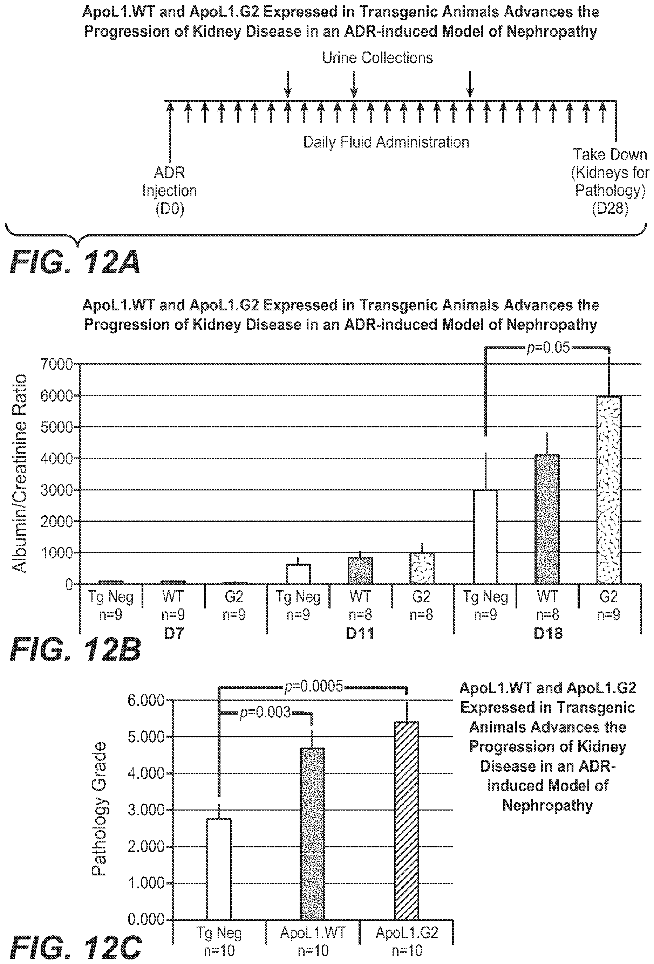

**Figure 12** **(A-C)** shows the effect of G0 variant of ApoL1 and G2 variant of ApoL1 expressed in transgenic animals on the progression of kidney disease in a doxorubicin-induced model of nephropathy (n = 9-10 per genotype, means are ± SEM, and p-values refer to a two-tailed t test with equal variance).

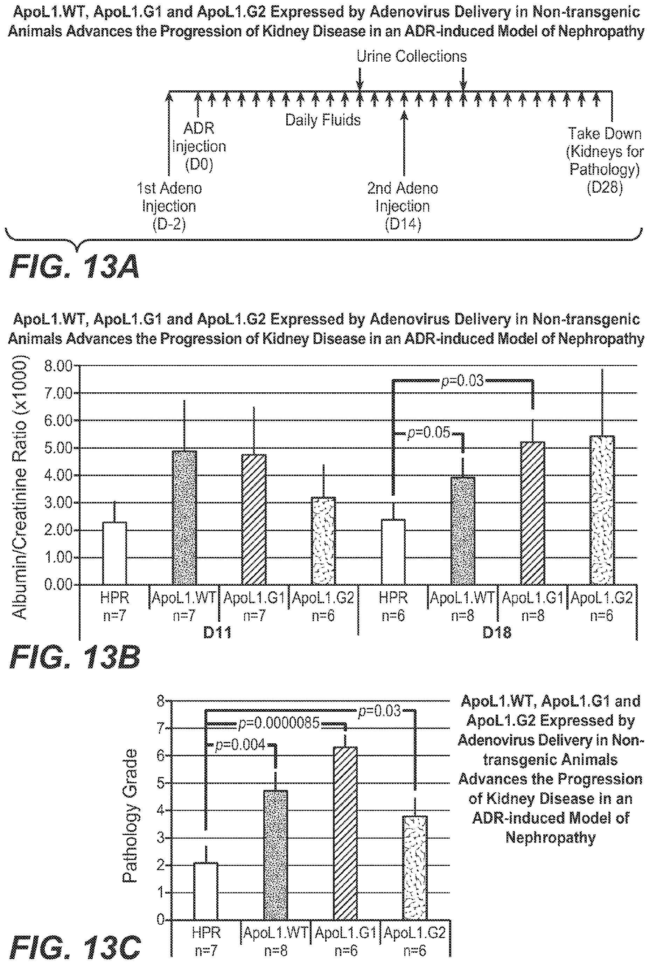

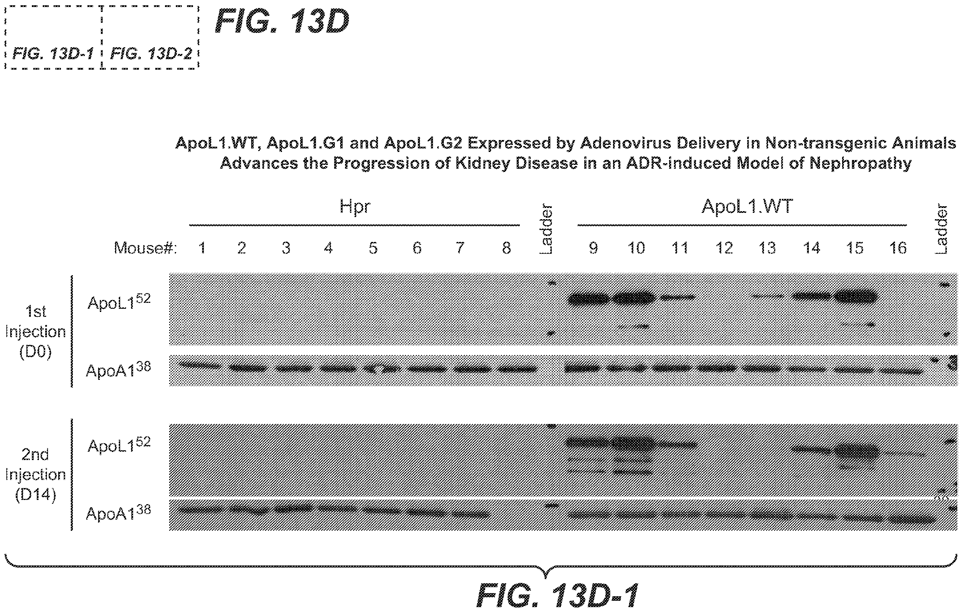

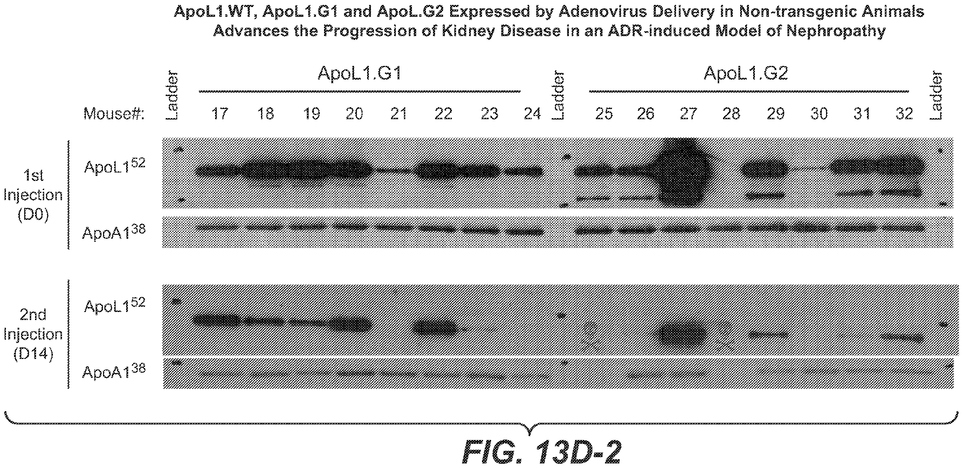

**Figure 13** **(A-D)** shows the effect of G0 variant of ApoL1, G1 variant of ApoL1 and G2 variant of ApoL1 expressed by adenovirus delivery in non-transgenic animals advances on the progression of kidney disease in a doxorubicin-induced model of nephropathy. The effect is shown relative to a control (mice expressing haptoglobin related protein (HPR)); (n= 6-8 per genotype, means are ± SEM, and p-values refer to a two-tailed t test with equal variance, skull and cross bones symbol indicates animals that died before the end-of-study).

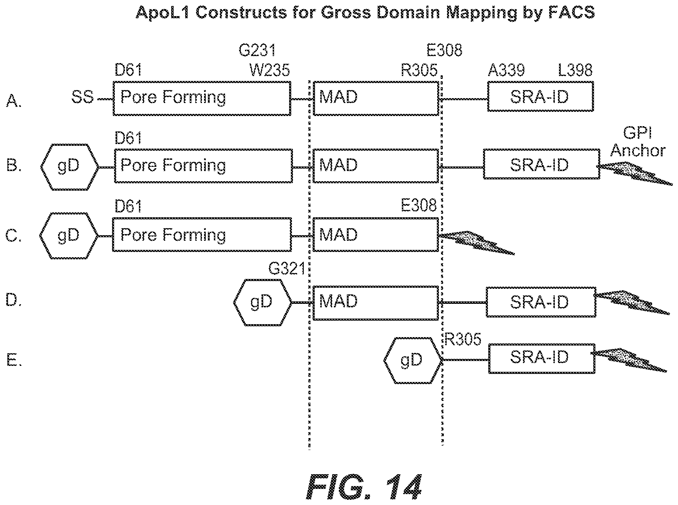

**Figure 14** shows a schematic representation of the APOL1 constructs. PFD: Pore forming domain, MAD: Membrane addressing domain, SRA-ID: Serum resistance associated protein-Interacting domain, GPI: Glycosylphosphatidylinositol anchor, gD: herpes simplex virus glycoprotein D anchor.

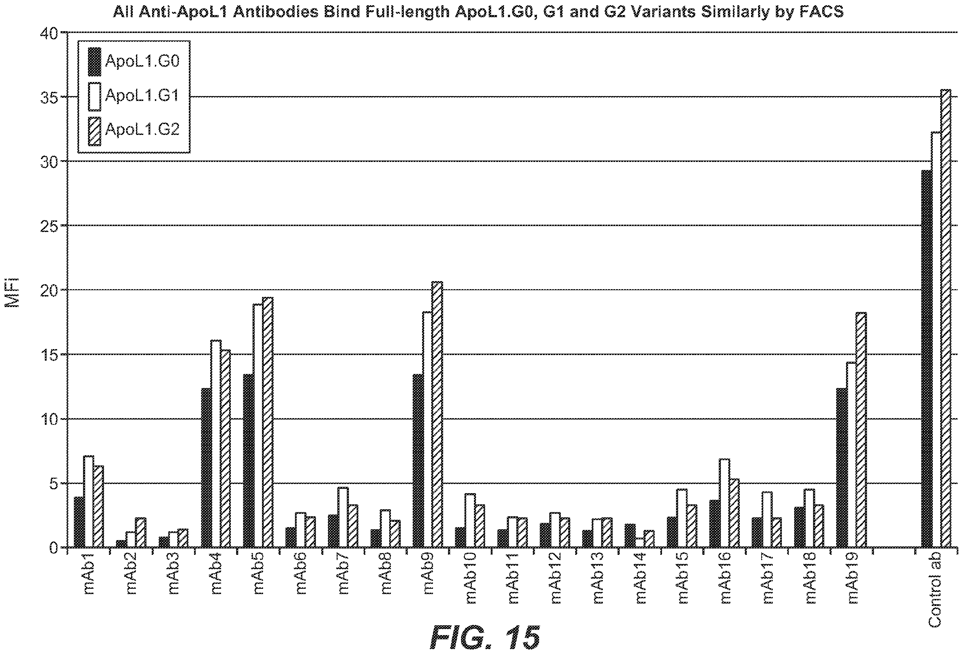

**Figure 15** depicts cross reactivity of anti-ApoL1 antibodies analyzed by FACS on CHO cells expressing G0 variant of APOL1 (Black), G1 variant of APOL1 (Grey) or G2 variant of APOL1 (hatched). Antibodies were used at 1 µg/ml concentration. As a positive control, a commercially available polyclonal antibody was used which binds non-specifically to more than one member of the apolipoprotein L family. Mean Fluorescence intensities (MFI) are plotted on the y axis.

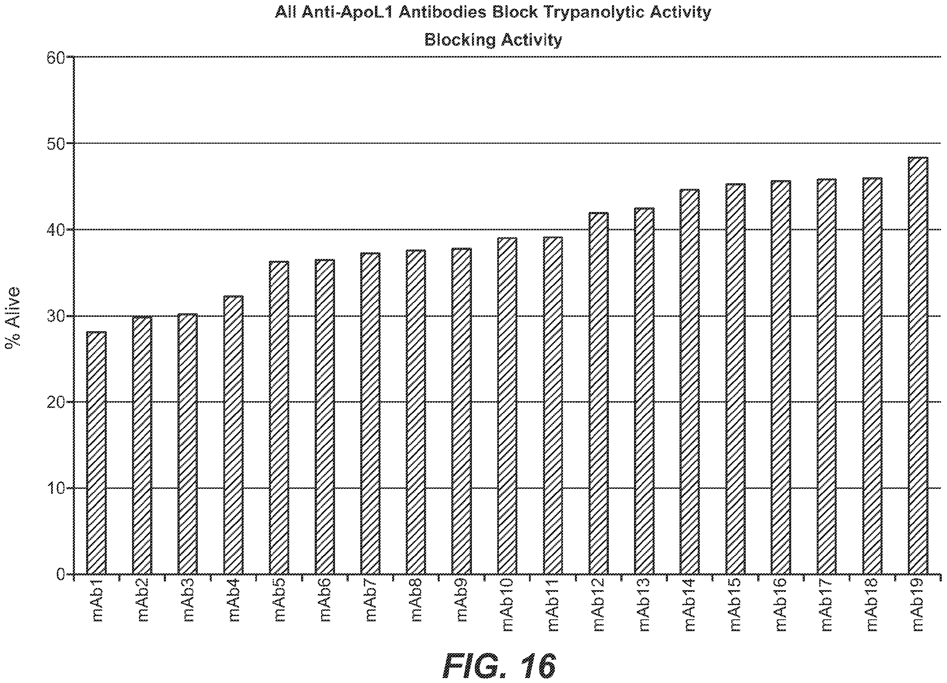

**Figure 16** shows the results of the *in vitro* blocking of Trypanolytic activity. Monoclonal anti-APOL1 antibodies generated in mice were added to *Trpanosoma brucei brucei* in the presence of 1% normal human serum (NHS) for 20 hr. Number of alive trypanosomes was measured in terms of fluorescent activity due to the presence of a resazurin based dye (Alamar blue). Blocking activity is normalized to the no-antibody control and plotted as % of alive trypanosomes.

### DETAILED DESCRIPTION OF EMBODIMENTS OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### I. DEFINITIONS

For purposes of interpreting this specification, the following definitions will apply and whenever appropriate, terms used in the singular will also include the plural and vice versa. In the event that any definition set forth below conflicts with any document cited herein the definition set forth below shall control.

As used in this specification and the appended claims, the singular forms "a," "an" and "the" include plural referents unless the context clearly dictates otherwise. Thus, for example, reference to "a protein" or an "antibody" includes a plurality of proteins or antibodies, respectively; reference to "a cell" includes a purality of cells, and the like.

The term "ApoL1" or "human ApoL1" refers to human apolipoprotein L1, a polypeptide that is the only member in a 6-gene family including a signal peptide. Three different variants are present in the population, namely the G0 variant of ApoL1, the G1 variant of ApoL1 and the G2 variant of ApoL1. The G0 variant of ApoL1 refers to the wild-type human ApoL1 protein (herein also referred to as "wt") having the amino acid sequence of SEQ ID NO:01. The G1 variant of ApoL1 refers to a variant of human ApoL1 protein that has two amino acid substitutions (S342G, I384M), having the amino acid sequence of SEQ ID NO:02. The G2 variant of ApoL1 refers to a variant of human ApoL1 protein that has a two amino acid deletion (N388 and Y389), having the amino acid sequence of SEQ ID NO:03.

The term "detecting" is used herein in the broadest sense to include both qualitative and/or quantitative measurements of a target molecule, i.e. detecting includes identifying the mere presence of the target molecule in a sample as well as determining the levels of the target molecule in the sample.

The term "doxorubicin" as used herein refers to the chemical compound with the CAS number 23214-92-8. Doxorubicin is herein also referred to as Adriamycin® or ADR.

The term "nephropathy" as used herein refers to a physiological condition wherein damage of the kidney occurs that disrupts its ability to properly regulate solute concentrations in the blood and urine. This can be assessed by a number of methods that commonly include: serum creatinine concentration, urinary protein concentration, urinary protein to creatinine ratio or through the use of tracer compounds such as phthalates. Nephropathy is often classified into apparently distinct clinical conditions, for example focal segmental glomerular sclerosis, HIV-Infection, sickle cell anemia, allograft loss following transplantation, hypertension, lupus nephritis, diabetes, and non diabetic chronic kidney disease. A nephropathy can also be classified histologically as characterized by pathological changes selected from one or more of: glomerular size, lobulation or adhesions, fibrosis of the tufts, fibrosis of Bowman's capsule, dilatation, narrowing of capillaries, thickening of basement membranes, protein in Bowman's space, increased cellularity (mesangial or endothelial), infiltration by leukocytes, capillary thrombi, tubules- atrophy, necrosis, vacuolar and hyaline droplet changes, basement membrane thickening, dilatation, inflammatory cells and casts in the lumen, interstitium- fibrosis, edema, acute and chronic leukocyte infiltration, arterioles- fibrosis, thrombosis, hyaline change and narrowing.

The term "ApoL1-mediated nephropathy" as used herein refers to a nephropathy as defined above, wherein the progression of the nephropathy is increased by the presence of one or more of G0 variant of Apo L1, G1 variant of Apo L1 and G2 variant of Apo L1.

The terms "label" or "detectable label" refers to any chemical group or moiety that can be linked to a substance that is to be detected or quantitated, e.g., an antibody. Typically, a label is a detectable label that is suitable for the sensitive detection or quantification of a substance. Examples of detectable labels include, but are not limited to, luminescent labels, e.g., fluorescent, phosphorescent, chemiluminescent, bioluminescent and electrochemiluminescent labels, radioactive labels, enzymes, particles, magnetic substances, electroactive species and the like. Alternatively, a detectable label may signal its presence by participating in specific binding reactions. Examples of such labels include haptens, antibodies, biotin, streptavidin, his-tag, nitrilotriacetic acid, glutathione S-transferase, glutathione and the like.

The terms "polypeptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. The terms "polypeptide" and "protein" as used herein specifically encompass antibodies.

"Purified" polypeptide (*e.g.,* antibody or immunoadhesin) means that the polypeptide has been increased in purity, such that it exists in a form that is more pure than it exists in its natural environment and/or when initially synthesized and/or amplified under laboratory conditions. Purity is a relative term and does not necessarily mean absolute purity.

An antibody "which binds" an antigen of interest, is one that binds the antigen with sufficient affinity such that the antibody is useful as a therapeutic agent or an assay reagent, e.g., as a capture antibody or as a detection antibody. Typically, such an antibody does not significantly cross-react with other antigens. The terms "antibody which binds to X" and "anti-X antibody" shall have the same meaning (wherein X is the name of the antigen of interest, e.g. a protein). Consequently, the terms "antibody which binds to ApoL1" can be used herein interchangeably with "anti-ApoL1 antibody".

With regard to the binding of a polypeptide to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a target molecule compared to binding of a control molecule, which generally is a molecule of similar structure that does not have binding activity.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

Antibodies are naturally occurring immunoglobulin molecules which have varying structures, all based upon the immunoglobulin fold. For example, IgG antibodies have two "heavy" chains and two "light" chains that are disulphide-bonded to form a functional antibody. Each heavy and light chain itself comprises a "constant" (C) and a "variable" (V) region. The V regions determine the antigen binding specificity of the antibody, and the C regions provide structural support and function in non-antigen-specific interactions with immune effectors. The antigen binding specificity of an antibody or antigen-binding fragment of an antibody is the ability of an antibody to specifically bind to a particular antigen.

The antigen binding specificity of an antibody is determined by the structural characteristics of the variable or V region. The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region may comprise amino acid residues from a "complementarity determining region" or "CDR" (*e.g.,* around about residues 24-34 (L1), 50-56 (L2) and 89-97 (L3) in the V_{L}, and around about 31-35B (H1), 50-65 (H2) and 95-102 (H3) in the V_{H} (Kabat et al., Sequences of Proteins of Immunological Interest, 5th Ed. Public Health Service, National Institutes of Health, Bethesda, Md. (1991)) and/or those residues from a "hypervariable loop" (*e.g.* residues 26-32 (L1), 50-52 (L2) and 91-96 (L3) in the V_{L}, and 26-32 (H1), 52A-55 (H2) and 96-101 (H3) in the V_{H} (Chothia and Lesk J. Mol. Biol. 196:901-917 (1987)).

"Framework" or "FR" residues are those variable domain residues other than the hypervariable region residues as herein defined.

"Antibody fragments" comprise a portion of an intact antibody, preferably comprising the antigen binding region thereof. Examples of antibody fragments include Fab, Fab', F(ab')₂, and Fv fragments; diabodies; tandem diabodies (taDb), linear antibodies(*e.g.*, U.S. Patent No. 5,641,870, Example 2; Zapata et al., Protein Eng. 8(10):1057-1062 (1995)); one-armed antibodies, single variable domain antibodies, minibodies, single-chain antibody molecules; multispecific antibodies formed from antibody fragments (*e.g.,* including but not limited to, Db-Fc, taDb-Fc, taDb-CH3, (scFV)4-Fc, di-scFv, bi-scFv, or tandem (di,tri)-scFv); and Bi-specific T-cell engagers (BiTEs).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g.,* IgG1, IgG2, IgG3, IgG4, IgA, and IgA2. The heavy chain constant domains that correspond to the different classes of antibodies are called α, δ, ε, γ, and µ, respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. In some embodiments, the Fv polypeptide further comprises a polypeptide linker between the V_{H} and V_{L} domains that enables the scFv to form the desired structure for antigen binding. For a review of scFv *see* Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "multispecific antibody" is used in the broadest sense and specifically covers an antibody that has polyepitopic specificity. Such multispecific antibodies include, but are not limited to, an antibody comprising a heavy chain variable domain (V_{H}) and a light chain variable domain (V_{L}), where the V_{H}V_{L} unit has polyepitopic specificity, antibodies having two or more V_{L} and V_{H} domains with each V_{H}V_{L} unit binding to a different epitope, antibodies having two or more single variable domains with each single variable domain binding to a different epitope, full length antibodies, antibody fragments such as Fab, Fv, dsFv, scFv, diabodies, bispecific diabodies, triabodies, tri-functional antibodies, antibody fragments that have been linked covalently or non-covalently. "Polyepitopic specificity" refers to the ability to specifically bind to two or more different epitopes on the same or different target(s). "Monospecific" refers to the ability to bind only one epitope. According to one embodiment the multispecific antibody is an IgG antibody which binds to each epitope with an affinity of 5 µM to 0.001 pM, 3 µM to 0.001 pM, 1 µM to 0.001 pM, 0.5 µM to 0.001 pM, or 0.1 µM to 0.001 pM.

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (*e.g.,* nurse sharks) and those derived from recombinant methods from humans and mouse antibodies (Nature (1989) 341:544-546; Dev Comp Immunol (2006) 30:43-56; Trend Biochem Sci (2001) 26:230-235; Trends Biotechnol (2003):21:484-490; WO 2005/035572; WO 03/035694; Febs Lett (1994) 339:285-290; WO00/29004; WO 02/051870).

The term "monoclonal antibody" as used herein refers to an antibody obtained from a population of substantially homogeneous antibodies, *i.e.,* the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the methods provided herein may be made by the hybridoma method first described by Kohler et al., Nature 256:495 (1975), or may be made by recombinant DNA methods (*see, e.g.,* U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA 81:6851-6855 (1984)). Chimeric antibodies of interest herein include "primatized" antibodies comprising variable domain antigen-binding sequences derived from a non-human primate *(e.g.* Old World Monkey, such as baboon, rhesus or cynomolgus monkey) and human constant region sequences (US Pat No. 5,693,780).

For the purposes herein, an "intact antibody" is one comprising heavy and light variable domains as well as an Fc region. The constant domains may be native sequence constant domains (*e.g.* human native sequence constant domains) or amino acid sequence variant thereof. Preferably, the intact antibody has one or more effector functions.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

A "naked antibody" is an antibody (as herein defined) that is not conjugated to a heterologous molecule, such as a detection moiety or label.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody which binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

Reference to "about" a value or parameter herein includes (and describes) variations that are directed to that value or parameter per se. For example, description referring to "about X" includes description of "X".

### II. ANIMAL MODEL, COMPOSITIONS AND METHODS

### A. Animal model for nephropathy

Provided herein is an animal model for nephropathy. As ApoL1 is only present in humans, African green monkeys and gorillas, we established an animal model on the basis of a transgenic animal. Upon generation, the non-human animals only express either of G0, G1 or G2. However, by crossing the animals it is possible to generate transgenic animals expressing a combination of the three variants. Thus, in one aspect, a non-human transgenic animal expressing human ApoL1 is provided herein. In certain embodiments, the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1. In certain embodiments, the non-human transgenic animal is a rodent. In certain embodiments, the non-human transgenic animal is selected from the group consisting of mouse, rat, hamster, guinea pig, rabbit, dog, cat, pig, cow and goat. In certain embodiments, the non-human transgenic animal is a mouse. In order to provide a model for nephropathy, the animal expresses ApoL1 and nephropathy is induced in the non-human transgenic animal. Therefore, in certain embodiments, the human transgenic animal has a nephropathy. There are different ways of inducing a nephropathy in the non-human transgenic animal. One possibility is to express in the non-human transgenic animal in addition to ApoL1 a transgene containing a portion of the human immunodeficiency virus (HIV). Expressing the HIV transgene leads to the development of an acute nephropathy. In a certain embodiment, the nephropathy is therefore an HIV-associated nephropathy. Another possibility to induce a nephropathy in the non-human transgenic animal is by chemical exposure. Doxorubicin is usually used in humans as a drug in chemotherapy. However, the substance is also known to induce nephropathy in animals. Therefore, in a certain embodiment, the nephropathy described herein is a doxorubicin-induced nephropathy.

In another aspect, the present description refers to a cell or a tissue derived from the non-human transgenic animal described above.

As shown herein, circulating ApoL1, including G0 variant of ApoL1, G1 variant of ApoL1 and G2 variant of ApoL1, can mediate the progression of nephropathy. Therefore, one possibility to reduce the progression of an ApoL1-mediated nephropathy is to remove circulating ApoL1. By binding of an antibody to ApoL1 the clearance of ApoL1 can be increased leading to a reduced serum concentration of ApoL1 and thus to an attenuated effect on the progression of an ApoL1-mediated nephropathy. Thus, in one aspect, a method of determining whether an agent is capable of reducing the serum concentration of human ApoL1 is provided, the method comprising the steps of measuring the serum concentration of human ApoL1 in the non-human transgenic animal described herein, administering the agent to the non-human transgenic animal, and measuring the serum concentration of human ApoL1 in the non-human transgenic animal, wherein a reduction in the serum concentration of human ApoL1 in the non-human transgenic animal indicates that the agent is capable of reducing the serum concentration of human ApoL1.

Alternatively, the binding of an agent, such as an antibody to ApoL1, can lead to a deactivation of ApoL1, i.e. the antibody-ApoL1-complex can not have the same physiological effect as compared to ApoL1 alone, thereby attenuating the effect on the progression of an ApoL1-mediated nephropathy. Yet another possibility is that the bound antibody can prevent multimerization of ApoL1 and attenuate the effect on the progression of an ApoL1-mediated nephropathy.

Therefore, identifying an agent capable of reducing the progression of an ApoL1 mediated nephropathy would be advantageous by assessing the effect of the agent on the progression on the ApoL1 mediated nephropathy based on the pathological phenotype of the kidneys of the non-human transgenic animal. Thus, in another aspect, provided is a method of identifying an agent capable of reducing the progression of an ApoL1 mediated nephropathy the method comprising the steps of inducing a nephropathy in a non-human transgenic animal as disclosed herein, administering the agent to the non-human transgenic animal, and assessing the progression of the ApoL1 mediated nephropathy based on the pathological phenotype of the kidneys of the non-human transgenic animal, wherein a less advanced ApoL1 mediated nephropathy as compared to non-human transgenic animals not administered with the agent identifies the agent to be capable of reducing the progression of the ApoL1 mediated nephropathy. As already mentioned, there are different ways of establishing a nephropathy in the non-human transgenic animal. In one embodiment, the nephropathy is induced by administration of doxorubicin. In another embodiment, the nephropathy is induced by expressing a transgene containing a portion of the human immunodeficiency virus in the non-human transgenic animal. The ability of the agent to reduce the progression of an ApoL1 mediated nephropathy may be based on its binding to one or more variants of human ApoL1. In a certain embodiment, the agent binds to i) human G0 variant of ApoL1 (SEQ ID NO:01), ii) human G0 variant of ApoL1 and human G1 variant of ApoL1 (SEQ ID NO:02), iii) human G0 variant of ApoL1 and human G2 variant of ApoL1 (SEQ ID NO:03), or iv) human G0 variant of ApoL1, human G1 variant of ApoL1 and human G2 variant of ApoL1. In a certain embodiment, the agent is an antibody. In a certain embodiment, the antibody is a monoclonal antibody. In a certain embodiment, the antibody is a human, humanized, or chimeric antibody. In a certain embodiment, the antibody is a full length IgG1 antibody.

Provided herein is furthermore a method for generation of an animal model for nephropathy. Thus, in one aspect, provided is a method of generating an animal model for nephropathy, the method comprising inducing a nephropathy in a non-human transgenic animal expressing human ApoL1. In a certain embodiment, the non-human animal is the non-human transgenic animal as described herein above. In a certain embodiment, the nephropathy is induced by expressing a transgene containing a portion of the human immunodeficiency virus in the non-human animal. In a certain embodiment, the nephropathy is induced by administration of doxorubicin. In a certain embodiment, doxorubicin is administered at a concentration from 10 mg/kg to 50 mg/kg. In a certain embodiment, doxorubicin is administered at a concentration from 15 mg/kg to 40 mg/kg. In a certain embodiment, doxorubicin is administered at a concentration from 20 mg/kg to 30 mg/kg. In a certain embodiment, doxorubicin is administered at a concentration from 24 mg/kg to 26 mg/kg. In a certain embodiment, doxorubicin is administered at a concentration of at least 25 mg/kg. In a certain embodiment, doxorubicin is administered at a concentration of 25 mg/kg. The measurement mg/kg refers to the mass of administered doxorubicin in mg per mass of bodyweight of the animal in kg. In a certain embodiment, doxorubicin is administered in multiple doses. In a certain embodiment, doxorubicin is administered in a single dose. In a certain embodiment, doxorubicin is administered intraveneous. In a certain embodiment, doxorubicin is administered into the tail vein.

Treatment of the animals with doxorubicin leads to dehydration. Thus, in a certain embodiment, the non-human animal is treated daily with subcutaneous fluids to prevent dehydration. In a certain embodiment, the subcutaneous fluid is administered at a volume of 0.5 to 5 ml. In a certain embodiment, the subcutaneous fluid is administered at a volume of 1 to 4 ml. In a certain embodiment, the subcutaneous fluid is administered at a volume of 1.5 to 3 ml. In a certain embodiment, the subcutaneous fluid is administered at a volume of 2 ml. In a certain embodiment, the subcutaneous fluid is lactated ringer's solution.

### B. Exemplary Anti-ApoL1 Antibodies

In order to reduce the progression of an ApoL1 mediated nephropathy, an antibody is generated which binds ApoL1. Thus, in one aspect, provided is an isolated antibody which binds to the human G0 variant of ApoL1 (SEQ ID NO:01) and to one or both of the human G1 variant of ApoL1 (SEQ ID NO:02) and the human G2 variant of ApoL1 (SEQ ID NO:03). In a certain embodiment, the antibody is a monoclonal antibody. In a certain embodiment, the antibody is a human, humanized, or chimeric antibody. In a certain embodiment, the the antibody is a full length IgG1 antibody. In a certain embodiment, the antibody is capable of blocking multimerization of ApoL1 variants. In a certain embodiment, the antibody is capable of reducing the serum concentration of human ApoL1. In a certain embodiment, the antibody is capable of reducing the progression of a nephropathy. In a certain embodiment, the nephropathy is an ApoL1 mediated nephropathy. In a certain embodiment, the ApoL1 mediated nephropathy is selected from the group consisting of HIV-associated nephropathy, focal segmental glomerular sclerosis associated nephropathy, sickle cell nephropathy, nephropathy associated with allograft loss following transplantation, and lupus nephritis associated nephropathy. In some embodiments, the ApoL1 mediated nephropathy is hypertension associated nephropathy. In some embodiments, the ApoL1 mediated nephropathy is diabetic nephropathy.

Furthermore, in one aspect, provided is an isolated nucleic acid encoding the antibody as described herein. In another aspect, provided is a host cell comprising the nucleic acid as described above. In another aspect, provided is a method of producing an antibody comprising culturing the host cell described above so that the antibody is produced. Furthermore, in one aspect, provided is pharmaceutical formulation comprising the antibody as described herein and a pharmaceutically acceptable carrier. Furthermore, in one aspect, provided is the antibody as described herein for use as a medicament. Furthermore, in one aspect, provided is the use of an antibody as described herein in the manufacture of a medicament. Furthermore, in one aspect, provided is a method for treating an individual having a nephropathy by administering to the individual the antibody as described herein. Furthermore, in one aspect, provided is a method of reducing the progression of a nephropathy in a subject comprising administering to the subject an effective amount of the antibody as described herein.

In a further aspect of the disclosure, an anti-ApoL1 antibody according to any of the above embodiments is a monoclonal antibody, including a chimeric, humanized or human antibody. In
one embodiment, an anti-ApoL1 antibody is an antibody fragment, e.g., a Fv, Fab, Fab', scFv, diabody, or F(ab')₂ fragment. In another embodiment, the antibody is a full length antibody, e.g., an intact IgG1 antibody or other antibody class or isotype as defined herein.

In a further aspect, an anti-ApoL1 antibody according to any of the above embodiments may incorporate any of the features, singly or in combination, as described in Sections 1-7 below:

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to 5 h at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 h) to ensure that equilibrium is reached. Thereafter, the mixtures are transferred to the capture plate for incubation at room temperature (e.g., for 1 h). The solution is then removed and the plate washed eight times with 0.1% polysorbate 20 (TWEEN-20®) in PBS. When the plates have dried, 150 µl/well of scintillant (MICROSCINT-20 ™; Packard) is added, and the plates are counted on a TOPCOUNT ™ gamma counter (Packard) for ten minutes. Concentrations of each Fab that give less than or equal to 20% of maximal binding are chosen for use in competitive binding assays.

According to another embodiment, Kd is measured using a BIACORE® surface plasmon resonance assay. For example, an assay using a BIACORE®-2000 or a BIACORE ®-3000 (BIAcore, Inc., Piscataway, NJ) is performed at 25°C with immobilized antigen CM5 chips at -10 response units (RU). In one embodiment, carboxymethylated dextran biosensor chips (CM5, BIACORE, Inc.) are activated with *N*-ethyl-*N'*-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio k_{off}/kₒₙ. See, e.g., Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the disclosure may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for ApoL1 and the other is for any other antigen. In a certain embodiment, one of the binding specificities is for ApoL1 and the second binding specificity triggers endocytosis of ApoL1 containing HDL particles. In certain embodiments, bispecific antibodies may bind to two different epitopes of ApoL1. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site which binds to ApoL1 as well as another, different antigen (see, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the disclosure may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (*e.g.* a substitution) at one or more amino acid positions.

In certain embodiments, the disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc
receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, e.g., as described in U.S. Patent No. 4,816,567. In one embodiment, isolated nucleic acid encoding an anti-ApoLl antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-ApoL1 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-ApoL1 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### C. Assays

Anti-ApoL1 antibodies provided herein may be identified, screened for, or characterized for their physical/chemical properties and/or biological activities by various assays known in the art.

### 1. Binding assays and other assays

In one aspect, an antibody of the disclosure is tested for its antigen binding activity, e.g., by known methods such as ELISA, Western blot, etc., or competition with trypanosome Serum Resistance Antigen (SRA) binding. In another aspect, competition assays may be used to identify an antibody that competes with an antibody as described herein for binding to ApoL1. In certain embodiments, such a competing antibody binds to the same epitope (e.g., a linear or a conformational epitope) that is bound by an antibody as described herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized ApoL1 is incubated in a solution comprising a first labeled antibody which binds to ApoL1 (e.g., an antibody as described herein) and a second unlabeled antibody that is being tested for its ability to compete with the first antibody for binding to ApoL1. The second antibody may be present in a hybridoma supernatant. As a control, immobilized ApoL1 is incubated in a solution comprising the first labeled antibody but not the second unlabeled antibody. After incubation under conditions permissive for binding of the first antibody to ApoL1, excess unbound antibody is removed, and the amount of label associated with immobilized ApoL1 is measured. If the amount of label associated with immobilized ApoL1 is substantially reduced in the test sample relative to the control sample, then that indicates that the second antibody is competing with the first antibody for binding to ApoL1. See Harlow and Lane (1988) Antibodies: A Laboratory Manual ch.14 (Cold Spring Harbor Laboratory, Cold Spring Harbor, NY).

### 2. Activity assays

In one aspect, assays are provided for identifying anti-ApoL1 antibodies thereof having biological activity. Biological activity may include, e.g., binding to ApoL1 thereby reducing the serum concentration of ApoL1 and/or reducing the progression of an ApoL1 mediated nephropathy. Antibodies having such biological activity in vivo and/or in vitro are also provided.

In certain embodiments, an antibody of the disclosure is tested for such biological activity. In certain embodiments, screening for antibodies is performed that reduce the trypanolytic activity of ApoL1. In certain embodiments, screening for antibodies is performed that reduce toxicity of ApoL1 in an in vitro model of podocyte toxicity. In certain embodiments, assays are used as described in the Examples.

### D. Pharmaceutical Formulations

Pharmaceutical formulations of an anti-ApoL1 antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide standard of care. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

### E. Therapeutic Methods and Compositions

Any of the anti-ApoL1 antibodies provided herein may be used in therapeutic methods.

In one aspect, an anti-ApoL1 antibody for use as a medicament is provided. In further aspects, an anti-ApoL1 antibody for use in treating nephropathy is provided. In certain embodiments, an anti-ApoL1 antibody for use in a method of treatment is provided. In certain embodiments, the disclosure provides an anti-ApoL1 antibody for use in a method of treating an individual having a nephropathy comprising administering to the individual an effective amount of the anti-ApoL1 antibody. In one such embodiment, the method further comprises
administering to the individual an effective amount of at least one additional therapeutic agent, e.g., as described below. In further embodiments, the disclosure provides an anti-ApoL1 antibody for use in reducing the serum concentration of ApoL1 and/or reducing the progression of an ApoL1 mediated nephropathy. In certain embodiments, the disclosure provides an anti-ApoL1 antibody for use in a method of reducing the serum concentration of ApoL1 and/or reducing the progression of an ApoL1 mediated nephropathy in an individual comprising administering to the individual an effective amount of the anti-ApoL1 antibody to reduce the serum concentration of ApoL1 and/or reduce the progression of an ApoL1 mediated nephropathy. An "individual" according to any of the above embodiments is preferably a human.

In a further aspect, the disclosure provides for the use of an anti-ApoL1 antibody in the manufacture or preparation of a medicament. In one embodiment, the medicament is for treatment of a nephropathy. In a further embodiment, the medicament is for use in a method of treating nephropathy comprising administering to an individual having a nephropathy an effective amount of the medicament. In one such embodiment, the method further comprises administering to the individual an effective amount of at least one additional therapeutic agent, e.g., as described below. In a further embodiment, the medicament is for reducing the serum concentration of ApoL1 and/or reducing the progression of an ApoL1 mediated nephropathy. In a further embodiment, the medicament is for use in a method of reducing the serum concentration of ApoL1 and/or reducing the progression of an ApoL1 mediated nephropathy in an individual comprising administering to the individual an effective amount of the medicament to reduce the serum concentration of ApoL1 and/or reduce the progression of an ApoL1 mediated nephropathy. An "individual" according to any of the above embodiments may be a human.

In a further aspect, the disclosure provides a method for treating a nephropathy. In one embodiment, the method comprises administering to an individual having a nephropathy an effective amount of an anti-ApoL1 antibody. In one such embodiment, the method further comprises administering to the individual an effective amount of at least one additional therapeutic agent, as described below. An "individual" according to any of the above embodiments may be a human.

In a further aspect, the disclosure provides a method for reducing the serum concentration of ApoL1 and/or reducing the progression of an ApoL1 mediated nephropathy in an individual. In one embodiment, the method comprises administering to the individual an effective amount of an anti-ApoL1 antibody to reduce the serum concentration of ApoL1 and/or reduce the
progression of an ApoL1 mediated nephropathy. In one embodiment, an "individual" is a human.

In a further aspect, the disclosure provides pharmaceutical formulations comprising any of the anti-ApoL1 antibodies provided herein, e.g., for use in any of the above therapeutic methods. In one embodiment, a pharmaceutical formulation comprises any of the anti-ApoL1 antibodies provided herein and a pharmaceutically acceptable carrier. In another embodiment, a pharmaceutical formulation comprises any of the anti-ApoL1 antibodies provided herein and at least one additional therapeutic agent, e.g., as described below.

Antibodies of the disclosure can be used either alone or in combination with other agents in a therapy. For instance, an antibody of the disclosure may be co-administered with at least one additional therapeutic agent.

Such combination therapies noted above encompass combined administration (where two or more therapeutic agents are included in the same or separate formulations), and separate administration, in which case, administration of the antibody of the disclosure can occur prior to, simultaneously, and/or following, administration of the additional therapeutic agent or agents. In one embodiment, administration of the anti-ApoL1 antibody and administration of an additional therapeutic agent occur within about one month, or within about one, two or three weeks, or within about one, two, three, four, five, or six days, of each other.

An antibody of the disclosure (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the disclosure would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the disclosure (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1mg/kg-10mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the antibody). An initial higher loading dose, followed by one or more lower doses may be administered. The progress of this therapy is easily monitored by conventional techniques and assays.

It is understood that any of the above formulations or therapeutic methods may be carried out using an immunoconjugate of the disclosure in place of or in addition to an anti-ApoL1 antibody.

### F. Articles of Manufacture

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, intravenous solution bags, etc. The containers may be formed from a variety of materials such as glass or
plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the disclosure. The label or package insert indicates that the composition is used for treating the condition of choice. Moreover, the article of manufacture may comprise (a) a first container with a composition contained therein, wherein the composition comprises an antibody of the disclosure; and (b) a second container with a composition contained therein, wherein the composition comprises a further cytotoxic or otherwise therapeutic agent. The article of manufacture in this embodiment of the disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, lactated ringer's solution and dextrose solution. It may further include other materials desirable from a commercial and user standpoint, including other buffers, diluents, filters, needles, and syringes.
It is understood that any of the above articles of manufacture may include an immunoconjugate of the disclosure in place of or in addition to an anti-ApoL1 antibody.

### EXAMPLES

### Ethics

All animals were handled in accordance with protocols approved by the Genentech Institutional Animal Care and Use Committee. Mouse colonies were maintained in a barrier facility at Genentech, conforming to California State legal and ethical standards of animal care.

### EXAMPLE 1: Generation and genotyping of APOL1 transgenic mice

To identify the role of apolipoprotein L1 (ApoL1) in the progression of kidney disease, we used a 44kb portion of a Bacterial Artificial Chromosome (BAC) containing the human ApoL1 gene to generate three series of transgenic mice that express G0 variant of ApoL1 as well as to the two common sequence variants (G1, G2) that provide resistance to trypanosomal
infection and increase kidney disease risk in African populations (Kao et al., 2008; Kopp et al., 2008).

### Generation of the transgenes

A BAC (Bacterial Artificial Chromosome) containing the human APOL1 gene (RP11-826P13) was modified using established recombineering methods and galK positive and counterselection, essentially as described in Warming et al, Nucleic Acids Res 2005, v33, e36 . After transformation into SW102 E.coli and after each modification step, BAC DNA was characterized by finger-printing using SpeI-digested BAC miniprep DNA. Dual-step cassettes for both positive and negative selection were synthesized by Blue Heron Biotech/OriGene. Each cassette has about 200 bp of homology on either side of the modification. The cassettes were designed to allow for insertion of the galK selection marker using EcoRI and SpeI/BamHI sites to generate the positive selection cassettes for BAC modification and for subsequent seamless removal of galK using BfuAI Type IIs restriction followed by self-ligation, to generate the counter-selection cassettes for BAC modification. The targeting cassettes were released from the pUC vector backbone by NotI digestion. Sequences of the two cassettes used in this work are included in table 2 (recognition sequences for AscI, NotI, EcoRI, BamHI, SpeI, and BfUAI (upper or lower strand for type IIs enzymes) are underlined; in the G1 cassette, the DNA changes encoding the two G1 mutations (S342G and I384M) are in bold).

The G2 mutation is a deletion of amino acids N388 and Y389 (base pairs AATTAT). The G1 mutation consists of two substitutions, S342G and I384M (AGC to GGC and ATT to ATG, respectively). After removal of the galK cassette from the BAC, the modified genomic region of the APOL1 BAC was retrieved (subcloned) into pBR322 using recombineering, giving rise to a 44 kb transgene. In addition, a control transgene (wt) was generated by retrieval from the unmodified APOL1 BAC. The transgenes were linearized by NotI digestion, purified and microinjected into zygotes from the C57BL/6N mouse strain, to generate transgenic animals, using established methods. Schematic drawings of the transgenes for ApoL1 wt, G1 and G2, respectively, are depicted in Fig. 1, A-C. Mice were maintained on a C57BL/6 background.

**Table 2**

| Name | Sequence |
|---|---|
| G0 variant ApoL1 (SEQ ID No:01) | |
| | |
| G1 variant ApoL1 (SEQ ID No:02) | |
| G2 variant ApoL1 (SEQ ID No:03) | |
| ApoL1 G1 cassette (SEQ ID No:04) | |
| ApoL1 G2 cassette (SEQ ID No:05) | |
| Retrieval oligo 1 (SEQ ID No:06) | |
| Retrieval oligo 2 (SEQ ID No:07) | |

### Genotyping of mice

Genotyping of ApoL1 transgenic mice was performed by standard PCR or Taqman analysis. Genotyping for the G0 variant of ApoL1 and G2 variant of ApoL1 uses standard PCR and primers directed against ApoL1 (Forward 5'- TTTCTTGTGCTGGATGTAG -3' (SEQ ID No:08) and Reverse 5' - ATATCTCTCCTGGTGGCT -3'(SEQ ID No:09)) as well as an internal control to Taci (Forward 5'- TGGGTGTCAGGTTCTTGCTTCAGC -3'(SEQ ID No:10) and Reverse 5' - CAGTGGATGCGCGCAGGAC -3'(SEQ ID No: 11)). The reaction conditions are 94°C, 4 min followed by 30 cycles at 94°C, 60 s (denaturing), 55°C, 30 s (annealing), and 72°C, 1 min (extension). A tagman assay is used for the G1 variant of ApoL1 using Type-it Fast SNP PCR master mix (Qiagen PN 206042), 0.5 µM of each primer and 0.15 µM of probe (Applied Biosystems). ApoL1 uses forward primer 5'-TGAGCAGAGGAGTCAAG -3'(SEQ ID No: 12), reverse primer 5'-TGTGGTCACAGTTCTTG -3'(SEQ ID No:13)), and probe 5'-6FAM-AGCTAAACATGCTCAAC -NFQ-MGB-3' (SEQ ID No:14). A positive control to Apo uses forward primer 5'- CACGTGGGCTCCAGCATT-3' (SEQ ID No:15), reverse primer 5'TCACCAGTCATTTCTGCCTTTG-3' (SEQ ID No:16), and probe 5'-VIC-CCAATGGTCGGGCACTGCTCAA-3' (SEQ ID No:17). The reaction conditions are 94°C, 4 min followed by 35 cycles of 94°C, 60 s (denaturing), 55°C, 30 s (annealing), and 72°C, 1 min (extension). End-point PCRs using FAM and VIC probes are read on an AB7900 (Applied Biosystems) to discern alleles from cluster plots.

### EXAMPLE 2: Analysis of serological ApoL1 in transgenic mice and humans

### Ultracentrifugal density gradients to fractionate major lipoprotein classes from serum

KBr/NaCl solutions with densities at 1.006, 1.019, 1.063, and 1.24 g/ml, were prepared according to McPherson, *et al* (McPherson et al., 2007). All salt solutions contained 0.1% NaN₃ and 0.04% EDTA. Densities were confirmed at 25°C with a Densito 30PX density meter (Mettler Toledo). Diluted serum solution with a density of 1.21 g/ml was prepared by the addition of 0.923 g KBr to 0.284 ml serum and 2.556 ml H₂O to a final volume of 3 ml. The gradient column was prepared and packed according to Chapman MJ, *et al* using a peristaltic pump (Chapman et al., 1981). Centrifugation was performed on a Beckman SW41Ti rotor at 40,000 rpm for > 48 h at 15°C. Fractions were collected in 1ml volumes with a peristaltic pump and then dialyzed against Tris buffer (0.04% EDTA, 5 mM Tris and 50 mM NaCl, pH 7.4) overnight at 4°C. For Western blots, 5 ml of each fraction were loaded onto a 4-20% Tris-Glycine gel, probed with antibodies specific to ApoL1 (Proteintech 11486-2-AP), ApoA1 (Rockland 600-101-196), and IgG (GE NA931V). Immunoreactivity was detected using ECL prime (GE healthcare).

### ApoL1 ELISA

An ApoL1 sandwich ELISA was developed as follows: 0.5 µg/ml of anti-ApoL1 polyclonal antibody (Proteintech 11486-2-AP) was coated onto a Maxisorp® plate (Thermo Fisher Scientific, 464718) in carbonate/bicarbonate buffer (1.59 g Na₂CO₃, 2.93 g NaHCO₃, in water qc to 1L) at 4°C overnight. The coated plate was blocked with 5% milk in Tris-buffered saline containing 0.05% Tween20. To prepare the assay standard curve, purified ApoL1 protein, whose concentration was measured at OD₂₈₀, was diluted in magic buffer (0.5% BSA, 0.05% Tween 20, 5 mM EDTA pH 8, 0.25% CHAPS, 0.2% BgG, 15 ppm Proclin in PBS) and spiked with 10% mouse serum (Invitrogen, 10410). A 4x serial dilution was subsequently performed to yield a standard curve ranging from 10 µg/ml to 0.153 ng/ml. Experimental serum samples were diluted 1:10 in magic buffer. The pre-coated and pre-blocked plate was sequentially incubated with either the standard curve or the experimental samples, 0.5 µg/ml of a biotinylated antibody directed against G0 variant of ApoL1 raised in rabbit using a recombinant fusion protein containing amino acids 61 to 398 of human APOL1 isoform 1(generated and purified at Genentech), and 1:10,000 of a peroxide conjugated streptavidin (GE, RPN4401V). The plate was washed between each incubation step on a 405™ microplate washer (Biotek) using phosphate buffered saline containing 0.05% Tween 20. ApoL1 was detected using the colorimetric BioFX® TMB substrate (Surmodics, TMBS 1000-01), stopped with 1M phosphoric acid and read on a SpectraMax250 spectrophotometer (Molecular Devices).

### Comparison of serological ApoL1 in transgenic mice and Human in fractionation studies and ELISAs

In order to determine if serological ApoL1 in transgenic mice associates with HDL particles similarly to human ApoL1, we fractionated the major lipoprotein classes from human serum (Fig. 2A) and compared them to transgenic serum (Fig. 2E) using the above described ultracentrifugal density gradient methods. We found that ApoA1 fractionates precisely as expected from human serum (Chapman et al., 1981; Davidson et al., 2009), indicating that the method is working well. It is principally present as HDL3 in Band IV and IF-c (fractions 7, 8 and 9); lower levels are present as HDL2 in Band III (fraction 6); significant quantities are found in IF-d as VHDL (fraction 9 and 10); and ApoA1 is pelleted with free protein such as IgG (fractions 11 and 12) (Fig. 2C, D). ApoL1 in human serum is present in a subset of the fractions where ApoA1 is present. A small amount is present as HDL3 in Band IV and IF-c (fractions 8 and 9) while the majority is present as VHDL in IF-d and Band V (fractions 9 and 10) as well as with free protein in BandV and the pellet (fractions 11, 12) (Fig. 2B, D). Consistent with human apolipoproteins, ApoA1 from serum of transgenic mice is present as HDL2 and HDL3 in Bands III and IV (fractions 6, 7, 8, and 9), while relatively smaller amounts are present as VHDL in IF-d (fraction 10) and with unbound protein such as IgG (fraction 12) (Fig. 2G, H). ApoL1 in transgenic mice is primarily present as HDL3 in Band IV (fractions 8 and 9) and VHDL (IF-d, fraction 10), while a relatively smaller amount is in Band V with un-lipidated proteins (fraction 11) in both G0 variant of ApoL1 and G2 variant of ApoL1 alike (Fig. 2F). ApoL1 and ApoA1 from both transgenic lines fractionate similarly.

In order to determine if our transgenic mouse lines express concentrations of ApoL1 similar to humans, circulating ApoL1 levels in transgenic mice were quantified and compared to human serological ApoL1 using a specific sandwich ELISA developed in-house as described above. We compared human sera from African American donors to mice expressing the G0 variant of ApoL1 or G2 variant of ApoL1 and transgenic negative littermate controls. While others report the concentration of circulating ApoL1 ranges from 386 - 15,743 ng/ml (Duchateau et al., 2000; Page et al., 2006), we find that ApoL1 in serum of animals expressing G0 variant of ApoL1 is 53-70 ng/ml (±10), the concentration in animals expressing the G2 variant is 93-117 (±14), and ApoL1 in human sera is 99-125 (±32). The range for each sera is shown in Fig. 3 and reflects the use of a standard curve with either G0 variant of ApoL1 (Fig. 3A) or G2 variant of ApoL1 (Fig. 3B). In conclusion we found that serological ApoL1 in transgenic mice behaves similarly to human ApoL1 in fractionation studies and ELISAs.

### EXAMPLE 3: Expression of ApoL1 in transgenic mice

### Quantitative PCR

Total RNA was extracted from organs of adult mice (RNeasy kit, Qiagen), and included a DNase treatment (Qiagen). RT-PCR was performed using an RT-PCR kit (High Capacity cDNA Reverse Transcription; Applied Biosystems) and quantitative PCR was performed for ApoL1 and mGapdH using primer/probe sets purchased from Applied Biosystems.

### Western Blots

Transient transfection of CHO.K1 cells was performed as per manufacturer's instructions using FuGene6 (Roche) and cDNAs to ApoLl (DNA370081, NM_003661) and ApoL2 (DNA369843, NM_145637) (Origene). Cells were quickly rinsed with PBS and immediately lysed in RIPA buffer plus protease inhibitor cocktail. For perfused tissue lysates, mice were anesthetized using 0.1-0.2 ml IP per 20-30 g body weight of a cocktail of Xylazine (1 mg/ml) and Ketamine (100 mg/ml) and perfused with 40 ml of phosphate-buffered saline through the heart. The liver and lung were removed, minced into 1-mm3 pieces, homogenized in HBSS, pelleted and lysed in RIPA buffer plus protease inhibitor cocktail. Protein concentrations were determined using bicinchoninic acid (BCA) reagent (Pierce). 30 µg of podocyte or tissue homogenates, 15 µg of over-expressed lysates, and 0.1 µl of ApoL1 transgenic mouse serum or human serum were loaded per lane and separated by electrophoresis using 4-20% Tris-Glycine or 4-12% Bis-Tris sodium dodecyl sulfate-polyacrylamide gels. Proteins were transferred to nitrocellulose membranes (Invitrogen). Blots were blocked in 5% milk in PBS with 0.05% Tween-20, then probed with ApoL1 antibodies (Sigma-Aldrich HPA018885 at 1:500, or Proteintech 11486-2AP at 1µg/ml). Signals were detected by chemiluminescence detection of primary antibodies with horse radish peroxidase-conjugated secondary antibodies followed by visualization with ECL-prime reagent (GE).

### Podocyte culture and differentiation

A conditionally immortalized human SV40^{tsA58T}/hTERT podocyte cell line was obtained from the University of Bristol (Bristol, UK). Undifferentiated podocytes were kept at 33°C in RPMI-1640 medium supplemented with 10% FBS (Sigma) and Insulin-Transferrin-Selenium (Invitrogen) for proliferation. Podocyte differentiation was achieved as described in Saleem et al (Saleem et al., 2002) essentially by thermoswitching the undifferentiated podocytes at 40-60% confluency to 37°C and maintaining them for an additional 14 days, with medium changes 3 times per week.

### Determination of ApoL1 expression using quantitative PCR and western blot analysis

In order to determine if transgenic mice expressing ApoL1 have a similar pattern of gene expression to humans, we performed quantitative PCR as described above on several organs reported to highly express ApoL1, namely the kidney, lung and liver (Genecards, www.genecards.org). Our data indicate that expression in transgenic mice is consistent with the human expression pattern. There is a relatively low expression in the whole kidney, with the greatest expression in the liver (88-fold over kidney) and lung (10-fold over kidney) (Fig. 4A, A'). Western analysis (as described above) of lung and liver lysates from mice perfused with PBS to remove circulating ApoL1 show that expressed protein correlates with the gene expression data. ApoL1 was detected using a polyclonal antibody (Proteintech, 11486-2AP), raised in rabbit using a recombinant fusion protein containing N-terminal 238 amino acids of human APOL1 isoform 1(Fig. 4B). ApoL1 was detected in undifferentiated or differentiated human cultured podocytes and in serum from transgenic mice expressing G0 variant of ApoL1 using a rabbit polyclonal antibody (Sigma, HPA018885). Lysates of ApoL1, ApoL2, or control transient transfections in CHO-1K cells indicate that both ApoL1 and ApoL2 are detected with the Sigma antibody (Fig. 4C).

### EXAMPLE 4:

### Chemical Induced Nephropathy

Male mice weighing 20 to 25 g and aged eight to twelve weeks were obtained. Doxorubicin (resuspended in warmed PBS to 0.5 mg/ml; Sigma D1515) was injected once via the tail vein of each non-anesthetized mouse. Age-matched male mice were injected with same volume of phosphate buffered saline. All control and experimental mice were housed individually and hydrated daily with 2 ml of lactated ringer's (Baxter). Proteinuria was assessed weekly from spontaneously voided urine from each animal. Urinary albumin was measured by enzyme-linked immunosorbent assay (ELISA) using mouse albumin as a standard (Innovative Research) and normalized to creatinine measured by colorimetric assay (Enzo Life Sciences). At day 21, animals were weighed, euthanized and kidneys were harvested for histology.

### Immunohistochemical Analysis

For all histological analyses, mice were sacrificed by CO₂ inhalation to effect, kidneys were immediately dissected, fixed by immersion in 4% paraformaldehyde in PBS, cut sagittally and processed for paraffin sections. Regressive Haematoxylin and Eosin (H&E) stains were performed as per standard protocols on 3 µm sections.

### Transmission Electron Microscopy (TEM)

The tissues were fixed in 1/2-strength Karnovsky's fixative (2% paraformaldehyde and 2.5% glutaraldehyde in 0.1 M sodium cacodylate buffer, pH 7.2), washed in the same buffer, and postfixed in 1% aqueous osmium textroxide for 1 h. The samples were then dehydrated through a series of ethanol followed by propylene oxide and embedded in Eponate 12 (Ted Pella). Thin sections were cut on a microtome (Ultracut E; Reichert), stained with uranyl acetate and lead citrate, and examined in a microscope (CM12; Philips). Images were captured with a retractable digital camera (MultiScan; Gatan).

### ApoL1 accelerates the progression of kidney disease in a mouse model

ApoL1 expression alone does not induce the progression to end stage renal disease (ESRD). By 25 weeks of age urinary protein levels are normal in transgenic mice expressing G0 variant of ApoL1 or G2 variant of ApoL1 and albumin to creatinine ratios fall below a value of five (albumin concentrations are normalized to creatinine levels; n = 3-9 per genotype and means are ± SEM; Fig. 5). Doxorubicin induced nephropathy is a well established rodent model of chronic kidney disease that is characterized by podocyte injury followed by glomerulosclerosis, tubulointerstitial inflammation and fibrosis (Lee and Harris, 2011). Therefore, we chemically induced nephropathy in our transgenic models as described above. In a series of preliminary experiments, we administered doxorubicin by a single tail-vein injection in C57BL/6 mice at eight to twelve weeks of age. Doses of 12, or 15 mg/kg per body weight had no effect on urinary protein (albumin to creatinine ratios range between 5 and 15) (Fig. 6) and kidney histology is indistinguishable in doxorubicin treated mice and littermate controls treated with PBS (data not shown). A median dose of 20 mg/kg induces proteinuria by Day 11, but the presence of ApoL1 has no effect (Fig. 6).

After a single injection of 25 mg/kg doxorubicin, urinary protein abruptly elevated by Day 2 in all mice treated with doxorubicin, but there was no significant difference between the genotypes (Fig. 7A). H&E stained kidney tissue indicates a variable nephropathy phenotype. Transgenic negative animals treated with doxorubicin (Fig. 7B) or PBS (Fig. 7C) are indistinguishable and do not exhibit a nephropathy. However, transgenic mice expressing G0 variant of ApoL1 (Fig. 7D) or G2 variant of ApoL1 (Fig. 7F, G) exhibit tubular ectasia, tubular necrosis, proteinaceous casts and tubular cell atrophy. However, not all transgenic positive mice display nephropathies (Fig. 7E) nor is any overt damage to glomeruli observed. In these studies we observed significant weight loss and mortality in the treated groups and particularly in those positive for ApoL1 expression.

Since reduced hydration and blood flow can lead to more rapid deterioration of the kidney and to early mortality, we repeated the higher dose of doxorubicin while additionally administering subcutaneous fluids daily. This allowed all of the mice to survive until the end of study at day 21. At the end of study, mice treated with doxorubicin have significant weight loss of 17% as compared to G0 variant of ApoL1 ApoL1 transgenic mice (10%) or transgenic negative animals (11%) (Fig. 8). Urinary albumin is also significantly elevated at day 11 in transgenic mice expressing the G2 variant with an average albumin to creatinine ratio of 6609, 5.2-fold higher than transgenic negative mice (*p*=0.002). Proteinuria remains high in these mice at day18 with an average albumin to creatinine ratio of 6146, 3.9-fold higher than treated transgenic negative animals (*p*=0.02). Urinary albumin is not significantly higher in doxorubicin -treated transgenic mice expressing G0 variant of ApoL1 but values are intermediate between doxorubicin-treated transgenic negative animals and mice expressing the G2 variant (average albumin to creatinine ratios in transgenics expressing G0 variant of ApoL1 are 2768 at day 11 and 2880 at day 18). PBS-treated controls remain normal (Fig. 9). Furthermore, TEM analysis reveals an elaboration of podocytes into regularly spaced foot processes in PBS-treated mice while animals treated with doxorubicin display podocyte foot process effacement, which is an invariable feature of proteinuric glomerular disease (Fig. 10). TEM analysis was performed as described above.

To assess if elevated urinary albumin values correlate with histopathology in doxorubicin-treated kidneys, mice were taken down at day 21 after doxorubicin treatment and stained with Haematoxylin and eosin (H&E), Periodic-acid Schiff (PAS) and Masson's trichrome stain. Qualitatively, H&E stains protein and nuclei, PAS stains basement membranes, whereas trichrome stains both interstitial fibrosis and basement membranes. While all doxorubicin-treated animals display some lesions, the difference between genotypes is in the severity and distribution of lesions, but not the lesions themselves, and the doxorubicin -treated transgenic mice expressing G0 variant of ApoL1 display an intermediate pathological phenotype to the transgenic negative and ApoL1 G2 transgenic mice. Low magnification images of H&E stained kidneys show a graded progression in damage relative to PBS treated mice (Fig. 11A) with the least amount of damage in non-transgenic mice (Fig. 11B), moderate in transgenic mice expressing G0 variant of ApoL1 (Fig. 11C), and most severe in transgenic mice expressing G2 variant of ApoL1 (Fig. 11D) in a protocol in which a single dose of doxorubicin is delivered intravenous at day 0, take down is at day 21, and subcutaneous fluids are administered daily to prevent dehydration (Fig. 11E). Higher magnifications of doxorubicin-treated kidneys stained with H&E (Fig. 11F-J), periodic-acid schiff stain (Fig. 11K-O), and Masson's Trichrome stain (Fig. 11P-S) demonstrate that compared to normal glomeruli (Fig. 11F, K, P), doxorubicin-treated animals present with FSGS (Fig. 11G-J, L-O, Q-S). Bowman's space is dilated and filled with proteinaceous fluid (Fig. 11H, star); there are mild tubulointerstitial infiltrates comprised primarily of lymphocytes (Fig. 11I, white asterix); protein is accumulated in dilated proximal tubules (Fig. 11O, cross); and there is an accumulation of hyaline (protein) droplets in the cytoplasm of proximal epithelial cells (Fig. 11O, black arrowhead). Pathology scores show that there is a significantly greater damage in doxorubicin-treated mice expressing ApoL1 G2 (*p*=0.00034) relative to transgenic negative controls or mice expressing G0 variant of ApoL1 (*p*=0.03) (Fig. 11T).

In all doxorubicin-treated mice, proteinaceous fluid is present and is expanding Bowman's space. There is focal segmental glomerular sclerosis, which variably affects the glomerular tufts within the whole kidney. Proximal tubules are dilated and filled with proteinaceous fluid and protein casts and the cytoplasm of proximal epithelial cells is filled with protein globules. Multifocally throughout the kidney there is mild tubulointerstitial infiltrate comprised primarily of lymphocytes. The following grading system was applied to determine the pathological phenotype of the kidneys in order to assess the progression of the ApoL1 mediated nephropathy. Four major structures of the kidney were graded on a 0-4 scale. 0 represents normal and 4 severe changes. At least 10 glomeruli were examined for each animal. The overall grade is based on a general impression of all the changes and takes into account the proportion of the appropriate structures affected in the entire section. Changes were graded for each component as follows:
1. Glomeruli- size, lobulation, adhesions, fibrosis of the tufts, fibrosis of Bowman's capsule, dilatation, narrowing of capillaries, thickening of basement membranes, protein in Bowman's space, increased cellularity (mesangial or endothelial), infiltration by leukocytes, capillary thrombi
2. Tubules- atrophy, necrosis, vacuolar and hyaline droplet changes, basement membrane thickening, dilatation, inflammatory cells and casts in the lumen
3. Interstitium- fibrosis, edema, acute and chronic leukocyte infiltration
4. Arterioles- fibrosis, thrombosis, hyaline change and narrowing

A separate study was performed with a larger cohort of animals. A single dose of doxorubicin was delivered intravenous (IV) at day 0, take down was at day 28, and subcutaneous fluids were administered daily to prevent dehydration (Fig. 12A). Urinary albumin normalized to creatinine levels was elevated in ApoL1 G2 variant and as compared with transgenic negative controls (p=0.05, Fig. 12B). Histopathology correlated with elevated urinary albumin values and disease progression of mice expressing G0 variant of ApoL1 was intermediate to mice expressing G2 variant of ApoL1 and as compared with transgenic negative controls. Pathology scores indicated there was a significantly greater damage in doxorubicin-treated mice expressing either G0 variant of ApoL1 (p=0.003) or G2 variant of ApoL1 (p=0.0005) relative to transgenic negative controls (Fig. 12C). Consistent with the previous study, urinary albumin was significantly elevated at day 18 in transgenic mice expressing the G2 variant of ApoL1 with an average albumin to creatinine ratio of 5964, 2-fold higher than transgenic negative mice (*p*=0.05). Though the elevation in urinary proteinuria was not significant in the mice expressing G0 variant of ApoL1 in comparison to non-transgenic animals, the pathology scores at the end of the 28-day study indicated a significant progression of disease relative to the transgenic negative that is intermediate to the transgenic negative and transgenic mice expressing G2 variant of ApoL1.

### EXAMPLE 5: ApoL1 adenovirus delivery

G0 variant of ApoL1, G1 variant of ApoL1 and G2 variant of ApoL1 were expressed using an adenovirus. ApoL1 variants were cloned into an adenoviral vector, produced and titerd by Vector BioLabs. Adenovirus delivery was achieved by tail-vein injection at D(-)2 and D14 in each mouse with 1 x 10⁸ Pfu brought to 100 µl in sterile PBS. Doxorubicin-induced nephropathy was performed as described above at D0 (see Example 4). Subcutaneous fluids were administered daily to prevent dehydration, urine was collected at D11 and D18 and take down was at day 28 (Fig. 13A). Urinary albumin normalized to creatinine levels was significantly elevated in G0 variant of ApoL1 (p=0.05) and G1 (p=0.03) variants and as compared with transgenic negative controls (Fig.13B). Histopathology correlated with elevated urinary albumin values and pathology scores revealed significantly greater damage observed in mice expressing G0 variant of ApoL1 (p=0.004), G1 variant of ApoL1 (p=0.000085), and G2 variant of ApoL1 (p=0.03) relative to transgenic negative controls (Fig. 13C). Levels of ApoL1 were high in the majority of mice injected with adenoviral constructs. Only animals expressing ApoL1 after the first injection were included in the urine analysis and pathology scoring (Fig. 13D). The effect of the ApoL1 variants was shown relative to mice expressing haptoglobin related protein (HPR) as a control. HPR is a serum secreted protein specific to humans and usually not expressed in mice. Conclusively, adenovirus expression of G0 variant of ApoL1, G1 variant of ApoL1 and G2 variant of ApoL1 delivered ApoL1 to the liver confirmed that serological expression of ApoL1 accelerates the progression of kidney disease.

### EXAMPLE 6: Making of antibodies recognizing ApoL1

Monoclonal antibodies are raised to G0, G1 and G2 variants by any standard hybridoma methodologies, including but not limited to, injection of mice or hamsters with recombinant ApoL1 protein (G0, G1 and G2). Additionally mice are subjected to hydrodynamic delivery of the DNA sequence encoding ApoL1 or its G1 and G2 variants in a suitable delivery vector (pCAGGS).

Antibody screening is performed as described as follows. Standard ELISA is used to on the original antigen to select positive hybridomas. Therefore, Maxisorp® plates coated with ApoL1 G0, G1 or G2 (amino acids D61-L398 with an N-terminal his or FLAG epitope tag) are blocked, bound to hybridoma supernatants and detected with HRP-conjugated anti-mouse. Then, an SRA (serum resistance associated protein) blocking ELISA is performed to identify antibodies that prevent binding of ApoL1's SRA-interacting domain with SRA, since this leucine zipper region might also mediate binding to other yet-to-be-identified proteins to mediate kidney malfunction. For the blocking ELISA, ApoLl-FLAG coated plates are blocked and incubated with SRA-his pre-bound to antibody supernatants. Any antibody binding in the presence of SRA-his (i.e. non-SRA-ID blocking antibodies) is detected with HRP-conjugated anti-his tag antibodies and TMB substrate.

Subsequently, an ApoAl-ApoLl sandwich ELISA of human serum is used to identify antibodies capable of recognizing ApoL1 within HDL particles in serum. For this purpose, Maxisorp® plates are coated with polyclonal goat anti-ApoAl antibodies, blocked and incubated in detergent-free buffer (to maintain HDL particle integrity) with human serum to capture HDL particles. Anti-ApoLl monoclonals are then incubated and any bound antibodies detected with HRP anti-mouse. Positive antibodies can be further tested on human ApoL1 G1 and/or G2 serum if necessary.

Next, epitope binning by standard cross-blocking ELISA is performed. For that purpose, any standard method can be used, such as binding of a primary antibody to recombinant ApoL1, followed by incubation with biotinylated seconday antibody and detecting with streptavidin-HRP to determine which antibodies compete for the same ApoL1 epitope.

For epitope mapping CHO cells stably expressing inducible ApoL1, or fragments thereof, on their surface via a C-terminally engineered glycosylphosphatidylinositol anchor are incubated with hybridoma supernatants, followed by Alexa647 anti-mouse to identify which domains they bind to. ApoL1 (with an N-terminal Herpes Simplex Virus glycoprotein D (gD) epitope tag including its signal sequence and a C-terminal glycosylphosphatidylinositol anchor to anchor it to the cell surface) lentiviruses are used to make CHO cells stably expressing ApoL1 G0, G1 or G2 full length or domain fragments in a doxycycline-inducible fashion to overcome toxicity issues. The following constructs are used: Full length = D610-L398; pore forming domain + membrane addressing domain = aa D61-E308; membrane addressing domain + SRA-interacting domain = G231- L398; SRA-ID only = R305-L398.
Cell based assays for ApoL1 activity include the following:
a) Membrane potential assays using doxycycline-inducible TRPC6 expressing stable 293 cells stimulated with carbachol. Any effect of ApoL1 (in the presence or absence of podocin expression) is assayed in the presence of anti-ApoLl antibodies to identify any that inhibit the effect of ApoL1 on TRPC6 signaling.
b) Any effect of recombinant ApoL1 on the actin cytoskeleton (Alexa phalloidin stained) of human immortalized podocytes is assayed in the presence of anti-ApoLl antibodies to determine if any inhibit the effect of ApoL1.
c) Adenovirally expressed ApoL1 (especially G1 variant) disrupts lysosomal integrity of human immortalized podocytes as assessed using a pinocytic dye (Lucifer yellow CH) and an acid tracer, Lysotracker Red. Ideally conditioned media from infected podocytes is used so as to enable pre-incubation with anti-ApoLl antibodies to identify any that inhibit the effect on lysosomes.

The potential candidate antibodies to various domains are tested in an *in vivo* efficacy model, namely for their ability to inhibit the ApoLl-enhanced doxorubicin-induced proteinuria in ApoL1 (G0, G1 and G2) transgenic mice. The candidate antibodies from various epitopes, preferably those that block SRA binding or one of the cell-based assays, are tested *in vivo.* Transgenic mice expressing G0 variant of ApoL1, G1 variant of ApoL1 and G2 variant of ApoL1 are dosed with doxorubicin to induce nephropathy as described above, as assessed by proteinuria (increased albumin:creatinine ratio in urine). As shown herein, G0 variant of ApoL1 enhances proteinuria compared to non-transgenic mice, and G2 variant of ApoL1 has an even greater effect. The ability of antibodies to ApoL1 to reverse this ApoLl-mediated enhancement of proteinuria is monitored.

PD marker for *in vivo* activity also includes monitoring serum ApoL1 levels to determine if ApoLl-containing HDL particles are depleted. Therefore, using the ApoL1 transgenic mouse models described herein, blood is drawn at appropriate intervals following antibody dosing for assessment of ApoL1 levels by sandwich ELISA to determine if ApoL1 is being depleted from the circulation. Rabbit polyclonal ApoL1 is coated on the plate, blocked and incubated with mouse serum in the presence of detergent and detected with biotinylated rabbit polyclonal ApoL1 to a non-competing epitope. Binding is detected with streptavidin-HRP and compared to recombinant ApoL1 standards to determine relative serum levels.

### EXAMPLE 7: Generating and characterizing ApoL1 antibodies

Ribi adjuvant was from Sigma (Cat# S6322), Complete Freund' Adjuvant and Incomplete Freund's Adjuvant (CFA/IFA) were from Becton Dickinson (BD, Cat# 231141/263910), adjuvants used in Toll Like Receptor (TLR) -cocktail were CpG from Invivogen (Cat#tlrl-1826-1), R848 from Invivogen (Cat# tlrl-r848), Poly I:C from Invivogen (Cat# vac-pic) and monophosphoryl lipid A (MPL) from Sigma (Cat#L6895), ClonaCell-HY Medium B (Cat# 03802), Medium C (Cat# 03803), Medium D (Cat# 03804) and Medium E (Cat# 03805) were from StemCell Technologies. Cytofusion Medium C (Cat# LCM-C) used for electrofusion was from Cyto Pulse Sciences. Goat anti-mouse IgG Fc horseradish peroxidase conjugated antibody was from Sigma (Cat# A2554). 3, 3', 5, 5'-tetramethylbenzidine (TMB) Conductivity one component HRP microwell substrate (Cat# TMBW-1000-01) and TMB stop reagent (Cat# BSTP-1000-01) were from BioFx Laboratories.

### In vivo immunization

For the hydrodynamic tail veil injection (HTV) immunization, BALB/c mice were immunized with 50 µg/mouse of ApoL1 G1 or G2 DNA construct (sequences are depicted in table 3 below) mixed with 1µg GM-CSF by tail veil injection in a total volume of 2.0 ml in phosphate buffered saline (PBS). The injections were performed every two weeks for a total of 6 injections.

For the protein immunization, BALB/c mice were immunized with either 10 or 50 µg/injection per mouse with recombinant human ApoL1 G0 (amino acids D61-L398) depending on the adjuvant. For the Ribi adjuvant (Sigma) and the TLR-cocktail adjuvant (10 µg CpG plus 10 µg poly I:C plus 20 µg R848 plus 50 µg MPL), a total of 10 µg of the antigen in 100 µl adjuvant/mouse was injected intraperitoneally (IP), subcutaneously (SC), or at the bottom of the tail (BOT) alternatively at 3 to 4 day intervals for a total of 16 injections. For the CFA/IFA adjuvant, 50 µg of antigen in 100 µl adjuvant/mouse was injected intraperitoneally (IP) every two weeks for a total 7 boosts. Three days after the final pre-fusion injection, lymphocytes from
mice spleens and lymph nodes were harvested.

### Fusion and ELISA screening

Isolated mouse lymphocytes were fused with PU-1 myeloma cells (American Type

Culture Collection) by using the Cyto Pulse CEEF-50 apparatus (Cyto Pulse Sciences). After two washes with Cytofusion Medium C the isolated lymphocytes and PU-1 cells were mixed at a 1:1 ratio and then resuspended at 10 million cells/ml in Cytofusion Medium C. Electrofusion was performed according to the manufacturer's instructions. Fused cells were cultured in ClonaCell-HY Medium C overnight at 37°C in a 7% CO₂ incubator. The next day, the fused cells were centrifuged and then resuspended in 10 ml ClonaCell-HY Medium C and then gently mixed with 90 ml Methylcellulose-based ClonaCell-HY Medium D. The cells were plated into Omni dishes (Cat#10026, Nunc) and allowed to grow in 37°C in a 7% CO₂ incubator. After a 6-7 day incubation, single hybridoma clones were picked by ClonePix FL (Genetix, United Kingdom) and transferred into 96-well cell culture plates (#353075, Becton Dickinson) with 200 µl/well ClonaCell-HY Medium E. After 6-7 days in culture, hybridoma supernatants were screened by ELISA. The supernatants of all ELISA positive clones against ApoL1 G0 were also collected for flow cytometry assays.

ELISA assay was performed as follows. 96-well microtiter ELISA plates (Greiner, Germany) were coated with ApoL1 G0, G1 or G2 at 1 µg/ml in 0.05 M carbonate buffer (pH 9.6) in a final volume of 100 µl/well at 4°C overnight. After washing three times with wash buffer (0.05% Tween 20 in PBS, Sigma), plates were blocked with 200 µl ELISA assay diluents containing BSA. 100 µl of cultured supernatants or diluted purified mAbs were added and incubated for 1 hour at room temperature. The plates were washed three times and incubated with HRP conjugated goat anti-mouse IgG Fc for 1 hour. After washing three times, bound HRP was detected by addition of TMB substrate (BioFX Laboratories, MD, USA) in a final volume of 100 µl/well for 5 min. The reactions were stopped by adding 100 µl/well of stop reagent (BioFX, Laboratories, MD, USA). OD 630 was detected on a Sunrise Tecan plate reader.

### mAbs Purification and Isotyping

The hybridoma supernatants were purified by Protein A affinity chromatography, then sterile-filtered (0.2 µm pore size, Nalge Nunc International, NY, USA) and stored at 4°C in PBS. Binding of the purified mAbs to APOL1 was confirmed by ELISA prior to further testing in functional assays.

The isotype of purified mAbs was determined by the mouse monoclonal antibody isotyping kit (Roche Diagnostics).

### Cell lines and constructs

Full length APOL1 protein can be subdivided into four domains (Fig. 14): the signal sequence, 1-27 aminoacids (aa), the Pore Forming Domain (PFD), 60-235 aa, the Membrane Addressing Domain (MAD), 240-303 aa, and the SRA-Interacting Domain (SRA-ID) 339-398aa (Fig. 14A). Since APOL1 overexpression is often toxic, stable doxycycline-inducible APOL1 expressing CHO cells were generated by viral infection. In order to mimic the physiologically secreted APOL1, CHO cells expressing and secreting either full length G0 variant of APOL1 (WT), or G1 variant of APOL1, or G2 variant of APOL1 were generated.

Since it was uncertain if these cells would be useful for assessing antibody binding by flow cytometry (FACS) due APOL1 secretion, GPI-anchored APOL1 expressing cells were also generated (Figure 14B) to ensure cell surface expression of the protein, albeit likely in a different conformation than native APOL1. Additionally, the GPI anchored constructs had the first 60 aa of APOL1 (which are not present in other ApoL family members and are not essential for ApoL1 activity) replaced with a gD (HSV glycoprotein D) epitope tag (and HSV signal sequence) to confirm expression and enable normalization of antibody binding. Furthermore, in order to map the antibodies to the three APOL1 domains (PFD, MAD or SRA-ID) by FACS, CHO cells expressing only one or two of the APOL1 domains, attached to the cell surface by a GPI anchor, with a gD at the N-terminal, were also generated (Fig. 14C-E).

Cell lines were generated as follows. APOL1 constructs for the G0 variant of APOL1 (WT, isoform a, NM_003661.3), the G1 variant of APOL1 (S342G, I384M) and the G2 variant of APOL1 (Δ N388, Y389) were generated by site directed mutagenesis (full length and truncated) were subcloned into a Gateway entry vector using the pENTR Directional TOPO cloning kit (K2400-20). Non-GPI anchored and herpes simplex virus glycoprotein D anchor (gD)-tagged and GPI-anchored constructs were generated and subcloned into the doxycycline-inducible lentiviral expression plasmid pInducer20 using Gateway LRII recombination. Constructs were verified by DNA sequencing and used to generate Lentiviruses encoding full length or truncated ApoL1 using the pInducer system. To generate stable inducible ApoL1 expressing CHO cells, lentiviruses were inoculated onto CHO cells, cultured for 72 h and then selected using G418. Viral P24 protein in the culture medium was periodically estimated by ELISA until it was no longer detectable. Expression of ApoL1 was induced with 5µg/ml doxycycline for 48 h prior to experimental analysis.

### Flow cytometery

19 monoclonal anti-APOLl antibodies were analyzed by FACS for binding to full length G0 variant of APOL1 (WT), G1 variant of APOL1 or G2 variant of APOL1 to estimate cross-reactivity. All antibodies bound to the G0 variant of APOL1 and both the G1 variant of APOL1 and the G2 variant of APOL1, as indicated by Mean Fluorescence Intensities (MFI) (Fig. 15).

To map the binding domains of each of the antibodies, FACS analysis was performed on cells expressing truncated versions of G0 variant of APOL1. Of the 19 anti-APOLl antibodies generated, 12 were PFD-specific, 1 was MAD-specific, 5 were SRA-ID specific, and 1 was considered a confirmation-sensitive binder, i.e. it binds to a non-linear epitope.

Flow cytometry analysis was performed as described herein below. ApoLl-CHO cells induced with 5 µg/ml doxycycline for 48 h were harvested with 5mM EDTA in PBS, washed with CHO media (spinning at 1200 rpm in a G6-SR centrifuge for 10 minutes) and washed again in PBS. Approximately 0.5x10⁵ cells per sample were incubated with 1µg/mL of antibody in PBS for 60 minutes, on ice. Cells were then collected by centrifugation, as above, washed 3 times with FACS buffer (PBS +3% Fetal bovine serum) and then resuspended and incubated for 60 minutes on ice with 2 µg/mL of Alexa Fluor® 488-conjugated goat anti-mouse/anti rabbit (H+L) IgG in PBS. Cells were then washed as above and resuspended in PBS with propidium iodide (PI) and analyzed using a fluorescence-activated cell sorting (FACS) flow cytometer (FACSCalibur™, BD Biosciences). Mean fluorescence intensities (MFI) were measured for each sample. Data was analyzed using FlowJo software (v8.4.5). MFIs were then exported into MS Excel and plotted, after subtraction of background signals from the secondary antibody alone.

### Trypanosome assay

A trypanosome-based functional assay was generated based on the hypothesis that the mechanism of ApoL1 mediated progression of kidney disease may be related to the ability of APOL1 to lyse trypanosomes. *Trypanosoma brucei brucei* is a human serum sensitive species of trypanosome lysed by APOL1. The monoclonal anti-APOLl antibodies described herein were therefore screened for their ability to block the APOL1-mediated lysis of normal human serum (NHS). Blocking activity was measured in terms of cell viability in the presence of 1% NHS. The anti-ApoLl antibodies have a blocking activity ranging from 28% to 49% in this assay at a concentration of 1 µg/ml (Fig. 16).

The Trypanosome Assay was performed as described below. *Trypanosoma brucei brucei* (Lister 427 VSG 221) was obtained under the appropriate permit from ATCC and grown in Modified HMI-9 media (ATCC# PRA-383), passaging at least 3 times a week. Trypanosomes were never allowed to grow to full confluency to ensure they remained in the proliferative rather than stumpy form. For blocking assays approximately 0.5x10⁵ *Trypanosoma brucei brucei* were incubated with 1% Normal Human serum (NHS) in the presence or absence of 1.0 µg/ml anti-ApoL1 antibodies in a 96 well clear plate for 20 h at 37C. Subsequenctly, 10 µl of AlamarBlue® (Invitrogen catalog #DAL1025) was added and after 4-6 hrs cell viability was read in a fluorimeter at 530ex/590em. Data were analyzed using SoftmaxPro. Relative Fluorescence Unit (RFU) values were exported into MS Excel for graph plotting.

**Table 3**

| Name | Sequence |
|---|---|
| ApoL1 G0 DNA sequence from Open Reading Frame (SEQ ID No:18) | |
| Entire sequence of ApoL1 G0 DNA construct (SEQ ID No:19) | |
| | |
| | |
| | |
| ApoL1 G1 DNA sequence from Open Reading Frame (SEQ ID No:20) | |
| Entire sequence of ApoL1 G1 DNA construct (SEQ ID No:21) | |
| | |
| | |
| ApoL1 G2 DNA sequence from Open | |
| Reading Frame (SEQ ID No:22) | |
| Entire sequence of ApoL1 G2 DNA construct (SEQ ID No:23) | |
| | |
| | |
| ApoL1 G0 amino acid sequence for protein immunization (SEQ ID No:24) | |

### REFERENCES

Chapman, M.J., Goldstein, S., Lagrange, D., Laplaud, P.M., 1981. A density gradient ultracentrifugal procedure for the isolation of the major lipoprotein classes from human serum. J Lipid Res 22, 339-358.
Davidson, W.S., Silva, R.A., Chantepie, S., Lagor, W.R., Chapman, M.J., Kontush, A., 2009. Proteomic analysis of defined HDL subpopulations reveals particle-specific protein clusters: relevance to antioxidative function. Arterioscler Thromb Vasc Biol 29, 870-876.
Duchateau, P.N., Movsesyan, I., Yamashita, S., Sakai, N., Hirano, K., Schoenhaus, S.A., O'Connor-Kearns, P.M., Spencer, S.J., Jaffe, R.B., Redberg, R.F., Ishida, B.Y., Matsuzawa, Y., Kane, J.P., Malloy, M.J., 2000. Plasma apolipoprotein L concentrations correlate with plasma triglycerides and cholesterol levels in normolipidemic, hyperlipidemic, and diabetic subjects. J Lipid Res 41, 1231-1236.
Duchateau, P.N., Pullinger, C.R., Orellana, R.E., Kunitake, S.T., Naya-Vigne, J., O'Connor, P.M., Malloy, M.J., Kane, J.P., 1997. Apolipoprotein L, a new human high density lipoprotein apolipoprotein expressed by the pancreas. Identification, cloning, characterization, and plasma distribution of apolipoprotein L. The Journal of biological chemistry 272, 25576-25582.
Freedman, B.I., Kopp, J.B., Langefeld, C.D., Genovese, G., Friedman, D.J., Nelson, G.W., Winkler, C.A., Bowden, D.W., Pollak, M.R., 2010. The apolipoprotein L1 (APOL1) gene and nondiabetic nephropathy in African Americans. J Am Soc Nephrol 21, 1422-1426.
Freedman, B.I., Langefeld, C.D., Lu, L., Divers, J., Comeau, M.E., Kopp, J.B., Winkler, C.A., Nelson, G.W., Johnson, R.C., Palmer, N.D., Hicks, P.J., Bostrom, M.A., Cooke, J.N., McDonough, C.W., Bowden, D.W., 2011. Differential effects of MYH9 and APOL1 risk variants on FRMD3 Association with Diabetic ESRD in African Americans. PLoS Genet 7, e1002150.
Hsu, C.Y., Lin, F., Vittinghoff, E., Shlipak, M.G., 2003. Racial differences in the progression from chronic renal insufficiency to end-stage renal disease in the United States. J Am Soc Nephrol 14, 2902-2907.
Kao, W.H., Klag, M.J., Meoni, L.A., Reich, D., Berthier-Schaad, Y., Li, M., Coresh, J., Patterson, N., Tandon, A., Powe, N.R., Fink, N.E., Sadler, J.H., Weir, M.R., Abboud, H.E., Adler, S.G., Divers, J., Iyengar, S.K., Freedman, B.I., Kimmel, P.L., Knowler, W.C., Kohn, O.F., Kramp, K., Leehey, D.J., Nicholas, S.B., Pahl, M.V., Schelling, J.R., Sedor, J.R., Thornley-Brown, D., Winkler, C.A., Smith, M.W., Parekh, R.S., 2008. MYH9 is associated with nondiabetic end-stage renal disease in African Americans. Nature genetics 40, 1185-1192.
Kopp, J.B., Nelson, G.W., Sampath, K., Johnson, R.C., Genovese, G., An, P., Friedman, D., Briggs, W., Dart, R., Korbet, S., Mokrzycki, M.H., Kimmel, P.L., Limou, S., Ahuja, T.S., Berns, J.S., Fryc, J., Simon, E.E., Smith, M.C., Trachtman, H., Michel, D.M., Schelling, J.R., Vlahov, D., Pollak, M., Winkler, C.A., 2011. APOL1 genetic variants in focal segmental glomerulosclerosis and HIV-associated nephropathy. J Am Soc Nephrol 22, 2129-2137.
Kopp, J.B., Smith, M.W., Nelson, G.W., Johnson, R.C., Freedman, B.I., Bowden, D.W., Oleksyk, T., McKenzie, L.M., Kajiyama, H., Ahuja, T.S., Berns, J.S., Briggs, W., Cho, M.E., Dart, R.A., Kimmel, P.L., Korbet, S.M., Michel, D.M., Mokrzycki, M.H., Schelling, J.R., Simon, E., Trachtman, H., Vlahov, D., Winkler, C.A., 2008. MYH9 is a major-effect risk gene for focal segmental glomerulosclerosis. Nature genetics 40, 1175-1184.
Lee, V.W., Harris, D.C., 2011. Adriamycin nephropathy: a model of focal segmental glomerulosclerosis. Nephrology (Carlton) 16, 30-38.
Madhavan, S.M., O'Toole, J.F., Konieczkowski, M., Ganesan, S., Bruggeman, L.A., Sedor, J.R., 2011. APOL1 localization in normal kidney and nondiabetic kidney disease. J Am Soc Nephrol 22,2119-2128.
McPherson, P.A., Young, I.S., McKibben, B., McEneny, J., 2007. High density lipoprotein subfractions: isolation, composition, and their duplicitous role in oxidation. J Lipid Res 48, 86-95.
Page, N.M., Olano-Martin, E., Lanaway, C., Turner, R., Minihane, A.M., 2006. Polymorphisms in the Apolipoprotein L1 gene and their effects on blood lipid and glucose levels in middle age males. Genes Nutr 1, 133-135.
Parsa, A., Kao, W.H., Xie, D., Astor, B.C., Li, M., Hsu, C.Y., Feldman, H.I., Parekh, R.S., Kusek, J.W., Greene, T.H., Fink, J.C., Anderson, A.H., Choi, M.J., Wright, J.T., Jr., Lash, J.P., Freedman, B.I., Ojo, A., Winkler, C.A., Raj, D.S., Kopp, J.B., He, J., Jensvold, N.G., Tao, K., Lipkowitz, M.S., Appel, L.J., 2013. APOL1 risk variants, race, and progression of chronic kidney disease. N Engl J Med 369, 2183-2196.
Saleem, M.A., O'Hare, M.J., Reiser, J., Coward, R.J., Inward, C.D., Farren, T., Xing, C.Y., Ni, L., Mathieson, P.W., Mundel, P., 2002. A conditionally immortalized human podocyte cell line demonstrating nephrin and podocin expression. J Am Soc Nephrol 13, 630-638.

## Claims

1. A non-human transgenic animal expressing human ApoL1, wherein the non-human transgenic animal is a rodent and wherein the non-human transgenic animal has a nephropathy.

2. The non-human transgenic animal of claim 1, wherein the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1.

3. The non-human transgenic animal of claim 1, wherein the rodent is a mouse.

4. The non-human transgenic animal of claim 1, wherein the nephropathy is an induced nephropathy.

5. The non-human transgenic animal of claim 1, wherein the nephropathy is a HIV-associated nephropathy.

6. The non-human transgenic animal of claim 1, wherein the nephropathy is a doxorubicin-induced nephropathy.

7. A method of determining whether an agent is capable of reducing the serum concentration of human ApoL1 the method comprising:
a) measuring the serum concentration of human ApoL1 in the non-human transgenic animal of any one of claims 1-6;
b) administering the agent to the non-human transgenic animal; and
c) measuring the serum concentration of human ApoL1 in the non-human transgenic animal;
wherein a reduction in the serum concentration of human ApoL1 in the non-human transgenic animal indicates that the agent is capable of reducing the serum concentration of human ApoL1.

8. A method of identifying an agent capable of reducing the progression of nephropathy the method comprising:
a) inducing a nephropathy in a non-human transgenic animal expressing human ApoL1, wherein the non-human transgenic animal is a rodent, optionally wherein the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1 or wherein the rodent is a mouse.
b) administering the agent to the non-human transgenic animal; and
c) assessing the progression of nephropathy based on the pathological phenotype of the kidneys of the non-human transgenic animal;
wherein a less advanced nephropathy as compared to non-human transgenic animals not administered with the agent identifies the agent to be capable of reducing the progression of nephropathy.

9. The method of claim 8, wherein the nephropathy is induced by administration of doxorubicin.

10. The method of claim 8, wherein the nephropathy is induced by expressing a transgene containing a portion of the human immunodeficiency virus in the non-human transgenic animal.

11. The method of any one of claims 7-10, wherein the agent binds to i) human G0 variant of ApoL1 (SEQ ID NO:01), ii) human G0 variant of ApoL1 and human G1 variant of ApoL1 (SEQ ID NO:02), iii) human G0 variant of ApoL1 and human G2 variant of ApoL1 (SEQ ID NO:03), or iv) human G0 variant of ApoL1, human G1 variant of ApoL1 and human G2 variant of ApoL1.

12. The method of any of claims 7-10, wherein the agent is an antibody.

13. A method of generating an animal model for nephropathy, the method comprising inducing a nephropathy in a non-human transgenic animal expressing human ApoL1, wherein the non-human transgenic animal is a rodent, optionally wherein the non-human transgenic animal expresses i) G0 variant of ApoL1 (SEQ ID NO:01), ii) G1 variant of ApoL1 (SEQ ID NO:02), iii) G2 variant of ApoL1 (SEQ ID NO:03), iv) G0 variant of ApoL1 and G1 variant of ApoL1, v) G0 variant of ApoL1 and G2 variant of ApoL1, vi) G1 variant of ApoL1 and G2 variant of ApoL1, or vii) G0 variant of ApoL1, G1 variant of ApoL1 and G2 variant of ApoL1 or wherein the rodent is a mouse.

14. The method of claim 13, wherein the nephropathy is induced by expressing a transgene containing a portion of the human immunodeficiency virus in the non-human animal.

15. The method of claim 13, wherein the nephropathy is induced by administration of doxorubicin.

16. The method of claim 15, wherein doxorubicin is administered (i) at a concentration from 15 mg/kg to 40 mg/kg, (ii) at a concentration from 20 mg/kg to 30 mg/kg, (iii) at a concentration from 24 mg/kg to 26 mg/kg, (iv) at a concentration of 25 mg/kg, (v) in a single dose, or (vi) into the tail vein.

17. The method of claim 16, wherein the non-human animal is treated daily with subcutaneous fluids to prevent dehydration.

## Patentansprüche

1. Nicht humanes transgenes Tier, das humanes ApoL1 exprimiert, wobei das nicht humane transgene Tier ein Nagetier ist und wobei das nicht humane transgene Tier eine Nephropathie aufweist.

2. Nicht humanes transgenes Tier nach Anspruch 1, wobei das nicht humane transgene Tier die i) G0-Variante von ApoL1 (SEQ ID NO: 01), ii) G1-Variante von ApoL1 (SEQ ID NO: 02), iii) G2-Variante von ApoL1 (SEQ ID NO: 03), iv) G0-Variante von ApoL1 und G1-Variante von ApoL1, v) G0-Variante von ApoL1 und G2-Variante von ApoL1, vi) G1-Variante von ApoL1 und G2-Variante von ApoL1 oder vii) G0-Variante von ApoL1, G1-Variante von ApoL1 und G2-Variante von ApoL1 exprimiert.

3. Nicht humanes transgenes Tier nach Anspruch 1, wobei das Nagetier eine Maus ist.

4. Nicht humanes transgenes Tier nach Anspruch 1, wobei die Nephropathie eine induzierte Nephropathie ist.

5. Nicht humanes transgenes Tier nach Anspruch 1, wobei die Nephropathie eine HIVassoziierte Nephropathie ist.

6. Nicht humanes transgenes Tier nach Anspruch 1, wobei die Nephropathie eine durch Doxorubicin induzierte Nephropathie ist.

7. Verfahren zum Bestimmen, ob ein Mittel in der Lage ist, die Serumkonzentration von humanem ApoL1 zu reduzieren, wobei das Verfahren Folgendes umfasst:
a) Messen der Serumkonzentration von humanem ApoL1 im nicht humanen transgenen Tier nach einem der Ansprüche 1-6;
b) Verabreichen des Mittels an das nicht humane transgene Tier; und
c) Messen der Serumkonzentration von humanem ApoL1 im nicht humanen transgenen Tier;
wobei eine Reduktion in der Serumkonzentration von humanem ApoL1 im nicht humanen transgenen Tier darauf hinweist, dass das Mittel in der Lage ist, die Serumkonzentration von humanem ApoL1 zu reduzieren.

8. Verfahren zum Identifizieren eines Mittels, das in der Lage ist, das Fortschreiten einer Nephropathie zu reduzieren, wobei das Verfahren Folgendes umfasst:
a) Induzieren einer Nephropathie in einem nicht humanen transgenen Tier, das humanes ApoL1 exprimiert, wobei das nicht humane transgene Tier ein Nagetier ist, gegebenenfalls wobei das nicht humane transgene Tier die i) G0-Variante von ApoL1 (SEQ ID NO: 01), ii) G1-Variante von ApoL1 (SEQ ID NO: 02), iii) G2-Variante von ApoL1 (SEQ ID NO: 03), iv) G0-Variante von ApoL1 und G1-Variante von ApoL1, v) G0-Variante von ApoL1 und G2-Variante von ApoL1, vi) G1-Variante von ApoL1 und G2-Variante von ApoL1 oder vii) G0-Variante von ApoL1, G1-Variante von ApoL1 und G2-Variante von ApoL1 exprimiert oder wobei das Nagetier eine Maus ist;
b) Verabreichen des Mittels an das nicht humane transgene Tier; und
c) Beurteilen des Fortschreitens der Nephropathie auf der Basis des pathologischen Phänotyps der Nieren des nicht humanen transgenen Tiers;
wobei eine weniger fortgeschrittene Nephropathie im Vergleich zu nicht humanen transgenen Tieren, denen das Mittel nicht verabreicht wurde, das Mittel als ein Mittel identifiziert, das in der Lage ist, das Fortschreiten einer Nephropathie zu reduzieren.

9. Verfahren nach Anspruch 8, wobei die Nephropathie durch die Verabreichung von Doxorubicin induziert wird.

10. Verfahren nach Anspruch 8, wobei die Nephropathie durch die Expression eines Transgens, enthaltend einen Abschnitt des humanen Immundefizienzvirus, im nicht humanen transgenen Tier induziert wird.

11. Verfahren nach einem der Ansprüche 7-10, wobei das Mittel an die i) humane G0-Variante von ApoL1 (SEQ ID NO: 01), ii) humane G0-Variante von ApoL1 und humane G1-Variante von ApoL1 (SEQ ID NO: 02), iii) humane G0-Variante von ApoL1 und humane G2-Variante von ApoL1 (SEQ ID NO: 03) oder iv) humane G0-Variante von ApoL1, humane G1-Variante von ApoL1 und humane G2-Variante von ApoL1 bindet.

12. Verfahren nach einem der Ansprüche 7-10, wobei das Mittel ein Antikörper ist.

13. Verfahren zum Erzeugen eines Tiermodells für Nephropathie, wobei das Verfahren das Induzieren einer Nephropathie in einem nicht humanen transgenen Tier, das humanes ApoL1 exprimiert, umfasst, wobei das nicht humane transgene Tier ein Nagetier ist, gegebenenfalls wobei das nicht humane transgene Tier die i) G0-Variante von ApoL1 (SEQ ID NO: 01), ii) G1-Variante von ApoL1 (SEQ ID NO: 02), iii) G2-Variante von ApoL1 (SEQ ID NO: 03), iv) G0-Variante von ApoL1 und G1-Variante von ApoL1, v) G0-Variante von ApoL1 und G2-Variante von ApoL1, vi) G1-Variante von ApoL1 und G2-Variante von ApoL1 oder vii) G0-Variante von ApoL1, G1-Variante von ApoL1 und G2-Variante von ApoL1 exprimiert oder wobei das Nagetier eine Maus ist.

14. Verfahren nach Anspruch 13, wobei die Nephropathie durch die Expression eines Transgens, enthaltend einen Abschnitt des humanen Immundefizienzvirus, im nicht humanen Tier induziert wird.

15. Verfahren nach Anspruch 13, wobei die Nephropathie durch die Verabreichung von Doxorubicin induziert wird.

16. Verfahren nach Anspruch 15, wobei Doxorubicin (i) in einer Konzentration von 15 mg/kg bis 40 mg/kg, (ii) in einer Konzentration von 20 mg/kg bis 30 mg/kg, (iii) in einer Konzentration von 24 mg/kg bis 26 mg/kg, (iv) in einer Konzentration von 25 mg/kg, (v) in einer Einzeldosis oder (vi) in die Schwanzvene verabreicht wird.

17. Verfahren nach Anspruch 16, wobei das nicht humane Tier täglich mit subkutanen Flüssigkeiten behandelt wird, um eine Dehydrierung zu verhindern.

## Revendications

1. Animal transgénique non humain exprimant l'ApoL1 humaine, dans lequel l'animal transgénique non humain est un rongeur et dans lequel l'animal transgénique non humain a une néphropathie.

2. Animal transgénique non humain selon la revendication 1, dans lequel l'animal transgénique non humain exprime i) le variant G0 de l'ApoL1 (SEQ ID NO: 01), ii) le variant G1 de l'ApoL1 (SEQ ID NO: 02), iii) le variant G2 de l'ApoL1 (SEQ ID NO: 03), iv) le variant G0 de l'ApoL1 et le variant G1 de l'ApoL1, v) le variant G0 de l'ApoL1 et le variant G2 de l'ApoL1, vi) le variant G1 de l'ApoL1 et le variant G2 de l'ApoL1 ou vii) le variant G0 de l'ApoL1, le variant G1 de l'ApoL1 et le variant G2 de l'ApoL1.

3. Animal transgénique non humain selon la revendication 1, dans lequel le rongeur est une souris.

4. Animal transgénique non humain selon la revendication 1, dans lequel la néphropathie est une néphropathie induite.

5. Animal transgénique non humain selon la revendication 1, dans lequel la néphropathie est une néphropathie associée au VIH.

6. Animal transgénique non humain selon la revendication 1, dans lequel la néphropathie est une néphropathie induite par la doxorubicine.

7. Procédé pour déterminer si un agent est capable de réduire la concentration sérique d'ApoL1 humaine, le procédé comprenant :
a) la mesure de la concentration sérique d'ApoL1 humaine chez l'animal transgénique non humain selon l'une quelconque des revendications 1 à 6 ;
b) l'administration de l'agent à l'animal transgénique non humain ; et
c) la mesure de la concentration sérique d'ApoL1 humaine chez l'animal transgénique non humain ;
dans lequel une réduction de la concentration sérique d'ApoL1 humaine chez l'animal transgénique non humain indique que l'agent est capable de réduire la concentration sérique d'ApoL1 humaine.

8. Procédé pour identifier un agent capable de réduire la progression de la néphropathie, le procédé comprenant :
a) l'induction d'une néphropathie chez un animal transgénique non humain exprimant l'ApoL1 humaine, dans lequel l'animal transgénique non humain est un rongeur, éventuellement dans lequel l'animal transgénique non humain exprime i) le variant G0 de l'ApoL1 (SEQ ID NO: 01), ii) le variant G1 de l'ApoL1 (SEQ ID NO: 02), iii) le variant G2 de l'ApoL1 (SEQ ID NO: 03), iv) le variant G0 de l'ApoL1 et le variant G1 de l'ApoL1, v) le variant G0 de l'ApoL1 et le variant G2 de l'ApoL1, vi) le variant G1 de l'ApoL1 et le variant G2 de l'ApoL1 ou vii) le variant G0 de l'ApoL1, le variant G1 de l'ApoL1 et le variant G2 de l'ApoL1 ou dans lequel le rongeur est une souris ;
b) l'administration de l'agent à l'animal transgénique non humain ; et
c) l'évaluation de la progression de la néphropathie basée sur le phénotype pathologique des reins de l'animal transgénique non humain ;
dans lequel une néphropathie moins avancée par comparaison à des animaux transgéniques non humains auxquels l'agent n'a pas été administré identifie que l'agent est capable de réduire la progression de la néphropathie.

9. Procédé selon la revendication 8, dans lequel la néphropathie est induite par administration de doxorubicine.

10. Procédé selon la revendication 8, dans lequel la néphropathie est induite par l'expression d'un transgène contenant une partie du virus de l'immunodéficience humaine chez l'animal transgénique non humain.

11. Procédé selon l'une quelconque des revendications 7 à 10, dans lequel l'agent se lie i) au variant G0 humain de l'ApoL1 (SEQ ID NO: 01), ii) au variant G0 humain de l'ApoL1 et au variant G1 humain de l'ApoL1 (SEQ ID NO: 02), iii) au variant G0 humain de l'ApoL1 et au variant G2 humain de l'ApoL1 (SEQ ID NO: 03) ou iv) au variant G0 humain de l'ApoL1, au variant G1 humain de l'ApoL1 et au variant G2 humain de l'ApoL1.

12. Procédé selon l'une quelconque des revendications 7 à 10, dans lequel l'agent est un anticorps.

13. Procédé pour générer un modèle animal de néphropathie, le procédé comprenant l'induction d'une néphropathie chez un animal transgénique non humain exprimant l'ApoL1 humaine, dans lequel l'animal transgénique non humain est un rongeur, éventuellement dans lequel l'animal transgénique non humain exprime i) le variant G0 de l'ApoL1 (SEQ ID NO: 01), ii) le variant G1 de l'ApoL1 (SEQ ID NO: 02), iii) le variant G2 de l'ApoL1 (SEQ ID NO: 03), iv) le variant G0 de l'ApoL1 et le variant G1 de l'ApoL1, v) le variant G0 de l'ApoL1 et le variant G2 de l'ApoL1, vi) le variant G1 de l'ApoL1 et le variant G2 de l'ApoL1 ou vii) le variant G0 de l'ApoL1, le variant G1 de l'ApoL1 et le variant G2 de l'ApoL1 ou dans lequel le rongeur est une souris.

14. Procédé selon la revendication 13, dans lequel la néphropathie est induite en exprimant un transgène contenant une partie du virus de l'immunodéficience humaine chez l'animal non humain.

15. Procédé selon la revendication 13, dans lequel la néphropathie est induite par administration de doxorubicine.

16. Procédé selon la revendication 15, dans lequel la doxorubicine est administrée (i) à une concentration de 15 mg/kg à 40 mg/kg, (ii) à une concentration de 20 mg/kg à 30 mg/kg, (iii) à une concentration de 24 mg/kg à 26 mg/kg, (iv) à une concentration de 25 mg/kg, (v) en une dose unique ou (vi) dans la veine de la queue.

17. Procédé selon la revendication 16, dans lequel l'animal non humain est traité quotidiennement avec des fluides sous-cutanés pour prévenir une déshydratation.


### SEQUENCE LISTING

<110> GENENTECH, INC. ET AL.
<120> ANIMAL MODEL FOR NEPHROPATHY AND AGENTS FOR TREATING THE SAME
<130> P32407-WO
<140>
   <141>
<150> 62/077,774
   <151> 2014-11-10
<160> 24
<170> PatentIn version 3.5
<210> 1
   <211> 398
   <212> PRT
   <213> Artificial Sequence
<220>
   <221> source
   <223> /note="Description of Artificial Sequence: Synthetic polypeptide"
<400> 1
   Met Glu Gly Ala Ala Leu Leu Arg Val Ser Val Leu Cys Ile Trp Met
   1               5                   10                  15
   Ser Ala Leu Phe Leu Gly Val Gly Val Arg Ala Glu Glu Ala Gly Ala
               20                  25                  30
   Arg Val Gln Gln Asn Val Pro Ser Gly Thr Asp Thr Gly Asp Pro Gln
           35                  40                  45
   Ser Lys Pro Leu Gly Asp Trp Ala Ala Gly Thr Met Asp Pro Glu Ser
       50                  55                  60
   Ser Ile Phe Ile Glu Asp Ala Ile Lys Tyr Phe Lys Glu Lys Val Ser
   65                  70                  75                  80
   Thr Gln Asn Leu Leu Leu Leu Leu Thr Asp Asn Glu Ala Trp Asn Gly
                   85                  90                  95
   Phe Val Ala Ala Ala Glu Leu Pro Arg Asn Glu Ala Asp Glu Leu Arg
               100                 105                 110
   Lys Ala Leu Asp Asn Leu Ala Arg Gln Met Ile Met Lys Asp Lys Asn
           115                 120                 125
   Trp His Asp Lys Gly Gln Gln Tyr Arg Asn Trp Phe Leu Lys Glu Phe
       130                 135                 140
   Pro Arg Leu Lys Ser Glu Leu Glu Asp Asn Ile Arg Arg Leu Arg Ala
   145                 150                 155                 160
   Leu Ala Asp Gly Val Gln Lys Val His Lys Gly Thr Thr Ile Ala Asn
                   165                 170                 175
   Val Val Ser Gly Ser Leu Ser Ile Ser Ser Gly Ile Leu Thr Leu Val
               180                 185                 190
   Gly Met Gly Leu Ala Pro Phe Thr Glu Gly Gly Ser Leu Val Leu Leu
           195                 200                 205
   Glu Pro Gly Met Glu Leu Gly Ile Thr Ala Ala Leu Thr Gly Ile Thr
       210                 215                 220
   Ser Ser Thr Met Asp Tyr Gly Lys Lys Trp Trp Thr Gln Ala Gln Ala
   225                 230                 235                 240
   His Asp Leu Val Ile Lys Ser Leu Asp Lys Leu Lys Glu Val Arg Glu
                   245                 250                 255
   Phe Leu Gly Glu Asn Ile Ser Asn Phe Leu Ser Leu Ala Gly Asn Thr
               260                 265                 270
   Tyr Gln Leu Thr Arg Gly Ile Gly Lys Asp Ile Arg Ala Leu Arg Arg
           275                 280                 285
   Ala Arg Ala Asn Leu Gln Ser Val Pro His Ala Ser Ala Ser Arg Pro
       290                 295                 300
   Arg Val Thr Glu Pro Ile Ser Ala Glu Ser Gly Glu Gln Val Glu Arg
   305                 310                 315                 320
   Val Asn Glu Pro Ser Ile Leu Glu Met Ser Arg Gly Val Lys Leu Thr
                   325                 330                 335
   Asp Val Ala Pro Val Ser Phe Phe Leu Val Leu Asp Val Val Tyr Leu
               340                 345                 350
   Val Tyr Glu Ser Lys His Leu His Glu Gly Ala Lys Ser Glu Thr Ala
           355                 360                 365
   Glu Glu Leu Lys Lys Val Ala Gln Glu Leu Glu Glu Lys Leu Asn Ile
       370                 375                 380
   Leu Asn Asn Asn Tyr Lys Ile Leu Gln Ala Asp Gln Glu Leu
   385 390 395
<210> 2
   <211> 398
   <212> PRT
   <213> Artificial Sequence
<220>
   <221> source
   <223> /note="Description of Artificial Sequence: Synthetic polypeptide"
<400> 2
   Met Glu Gly Ala Ala Leu Leu Arg Val Ser Val Leu Cys Ile Trp Met
   1               5                   10                  15
   Ser Ala Leu Phe Leu Gly Val Gly Val Arg Ala Glu Glu Ala Gly Ala
               20                  25                  30
   Arg Val Gln Gln Asn Val Pro Ser Gly Thr Asp Thr Gly Asp Pro Gln
           35                  40                  45
   Ser Lys Pro Leu Gly Asp Trp Ala Ala Gly Thr Met Asp Pro Glu Ser
       50                  55                  60
   Ser Ile Phe Ile Glu Asp Ala Ile Lys Tyr Phe Lys Glu Lys Val Ser
   65                  70                  75                  80
   Thr Gln Asn Leu Leu Leu Leu Leu Thr Asp Asn Glu Ala Trp Asn Gly
                   85                  90                  95
   Phe Val Ala Ala Ala Glu Leu Pro Arg Asn Glu Ala Asp Glu Leu Arg
               100                 105                 110
   Lys Ala Leu Asp Asn Leu Ala Arg Gln Met Ile Met Lys Asp Lys Asn
           115                 120                 125
   Trp His Asp Lys Gly Gln Gln Tyr Arg Asn Trp Phe Leu Lys Glu Phe
       130                 135                 140
   Pro Arg Leu Lys Ser Glu Leu Glu Asp Asn Ile Arg Arg Leu Arg Ala
   145                 150                 155                 160
   Leu Ala Asp Gly Val Gln Lys Val His Lys Gly Thr Thr Ile Ala Asn
                   165                 170                 175
   Val Val Ser Gly Ser Leu Ser Ile Ser Ser Gly Ile Leu Thr Leu Val
               180                 185                 190
   Gly Met Gly Leu Ala Pro Phe Thr Glu Gly Gly Ser Leu Val Leu Leu
                   195                 200                 205
   Glu Pro Gly Met Glu Leu Gly Ile Thr Ala Ala Leu Thr Gly Ile Thr
       210                 215                 220
   Ser Ser Thr Met Asp Tyr Gly Lys Lys Trp Trp Thr Gln Ala Gln Ala
   225                 230                 235                 240
   His Asp Leu Val Ile Lys Ser Leu Asp Lys Leu Lys Glu Val Arg Glu
                   245                 250                 255
   Phe Leu Gly Glu Asn Ile Ser Asn Phe Leu Ser Leu Ala Gly Asn Thr
               260                 265                 270
   Tyr Gln Leu Thr Arg Gly Ile Gly Lys Asp Ile Arg Ala Leu Arg Arg
           275                 280                 285
   Ala Arg Ala Asn Leu Gln Ser Val Pro His Ala Ser Ala Ser Arg Pro
       290                 295                 300
   Arg Val Thr Glu Pro Ile Ser Ala Glu Ser Gly Glu Gln Val Glu Arg
   305                 310                 315                 320
   Val Asn Glu Pro Ser Ile Leu Glu Met Ser Arg Gly Val Lys Leu Thr
                   325                 330                 335
   Asp Val Ala Pro Val Gly Phe Phe Leu Val Leu Asp Val Val Tyr Leu
               340                 345                 350
   Val Tyr Glu Ser Lys His Leu His Glu Gly Ala Lys Ser Glu Thr Ala
           355                 360                 365
   Glu Glu Leu Lys Lys Val Ala Gln Glu Leu Glu Glu Lys Leu Asn Met
       370                 375                 380
   Leu Asn Asn Asn Tyr Lys Ile Leu Gln Ala Asp Gln Glu Leu
   385                 390                 395
<210> 3
   <211> 396
   <212> PRT
   <213> Artificial Sequence
<220>
   <221> source
   <223> /note="Description of Artificial Sequence: Synthetic polypeptide"
<400> 3
   Met Glu Gly Ala Ala Leu Leu Arg Val Ser Val Leu Cys Ile Trp Met
   1               5                   10                  15
   Ser Ala Leu Phe Leu Gly Val Gly Val Arg Ala Glu Glu Ala Gly Ala
               20                  25                  30
   Arg Val Gln Gln Asn Val Pro Ser Gly Thr Asp Thr Gly Asp Pro Gln
           35                  40                  45
   Ser Lys Pro Leu Gly Asp Trp Ala Ala Gly Thr Met Asp Pro Glu Ser
       50                  55                  60
   Ser Ile Phe Ile Glu Asp Ala Ile Lys Tyr Phe Lys Glu Lys Val Ser
   65                  70                  75                  80
   Thr Gln Asn Leu Leu Leu Leu Leu Thr Asp Asn Glu Ala Trp Asn Gly
                   85                  90                  95
   Phe Val Ala Ala Ala Glu Leu Pro Arg Asn Glu Ala Asp Glu Leu Arg
               100                 105                 110
   Lys Ala Leu Asp Asn Leu Ala Arg Gln Met Ile Met Lys Asp Lys Asn
           115                 120                 125
   Trp His Asp Lys Gly Gln Gln Tyr Arg Asn Trp Phe Leu Lys Glu Phe
       130                 135                 140
   Pro Arg Leu Lys Ser Glu Leu Glu Asp Asn Ile Arg Arg Leu Arg Ala
   145                 150                 155                 160
   Leu Ala Asp Gly Val Gln Lys Val His Lys Gly Thr Thr Ile Ala Asn
                   165                 170                 175
   Val Val Ser Gly Ser Leu Ser Ile Ser Ser Gly Ile Leu Thr Leu Val
               180                 185                 190
   Gly Met Gly Leu Ala Pro Phe Thr Glu Gly Gly Ser Leu Val Leu Leu
           195                 200                 205
   Glu Pro Gly Met Glu Leu Gly Ile Thr Ala Ala Leu Thr Gly Ile Thr
       210                 215                 220
   Ser Ser Thr Met Asp Tyr Gly Lys Lys Trp Trp Thr Gln Ala Gln Ala
   225                 230                 235                 240
   His Asp Leu Val Ile Lys Ser Leu Asp Lys Leu Lys Glu Val Arg Glu
                   245                 250                 255
   Phe Leu Gly Glu Asn Ile Ser Asn Phe Leu Ser Leu Ala Gly Asn Thr
   260 265 270
   Tyr Gln Leu Thr Arg Gly Ile Gly Lys Asp Ile Arg Ala Leu Arg Arg
   275 280 285
   Ala Arg Ala Asn Leu Gln Ser Val Pro His Ala Ser Ala Ser Arg Pro
   290 295 300
   Arg Val Thr Glu Pro Ile Ser Ala Glu Ser Gly Glu Gln Val Glu Arg
   305 310 315 320
   Val Asn Glu Pro Ser Ile Leu Glu Met Ser Arg Gly Val Lys Leu Thr
   325 330 335
   Asp Val Ala Pro Val Ser Phe Phe Leu Val Leu Asp Val Val Tyr Leu
   340 345 350
   Val Tyr Glu Ser Lys His Leu His Glu Gly Ala Lys Ser Glu Thr Ala
   355 360 365
   Glu Glu Leu Lys Lys Val Ala Gln Glu Leu Glu Glu Lys Leu Asn Ile
   370 375 380
   Leu Asn Asn Lys Ile Leu Gln Ala Asp Gln Glu Leu
   385 390 395
<210> 4
   <211> 593
   <212> DNA
   <213> Artificial Sequence
<220>
   <221> source
   <223> /note="Description of Artificial Sequence: Synthetic polynucleotide"
<400> 4
   gcggccgctc acacgaggca ttgggaagga catccgtgcc ctcagacgag ccagagccaa 60
   tcttcagtca gtaccgcatg cctcagcctc acgcccccgg gtcactgagc caatctcagc 120
   tgaaagcggt gaacaggtgg agagggttaa tgaacccagc atcctggaaa tgagcagagg 180
   agtcaagctc acggatgtgg cccctgtagg cttctttctt gtgctggatg tagtctacct 240
   cgtgtacgaa tcaaagcact tacatgaggg ggcaaagaat gcaggtgaat tcaataaact 300
   cggcggatcc acctgcattc caaagtcaga gacagctgag gagctgaaga aggtggctca 360
   ggagctggag gagaagctaa acatgctcaa caataattat aagattctgc aggcggacca 420
   agaactgtga ccacagggca gggcagccac caggagagat atgcctggca ggggccagga 480
   caaaatgcaa actttttttt ttttctgaga cagagtcttg ctctgtcgcc aagttggagt 540
   gcaatggtgc gatctcagct cactgcaagc tctgcctccc gtgttgcggc cgc 593
<210> 5
   <211> 464
   <212> DNA
   <213> Artificial Sequence
<220>
   <221> source
   <223> /note="Description of Artificial Sequence: Synthetic polynucleotide"
<400> 5
   gcggccgctt aatgaaccca gcatcctgga aatgagcaga ggagtcaagc tcacggatgt 60
   ggcccctgta agcttctttc ttgtgctgga tgtagtctac ctcgtgtacg aatcaaagca 120
   cttacatgag ggggcaaagt cagagacagc tgaggagctg aagaaggtgg ctcaggagct 180
   ggaggagaag ctaaacattc tcaacaataa gactgtgcag gtgaattcgg atccattaaa 240
   actagtacct gcattcaaga ttctgcaggc ggaccaagaa ctgtgaccac agggcagggc 300
   agccaccagg agagatatgc ctggcagggg ccaggacaaa atgcaaactt tttttttttt 360
   ctgagacaga gtcttgctct gtcgccaagt tggagtgcaa tggtgcgatc tcagctcact 420
   gcaagctctg cctcccgtgt tcaagcgatt ctcctggcgg ccgc 464
<210> 6
   <211> 78
   <212> DNA
   <213> Artificial Sequence
<220>
   <221> source
   <223> /note="Description of Artificial Sequence: Synthetic oligonucleotide"
<400> 6
   tagtcctgtc cagggccccc tggccgcaga caaatgctac agacacggct ggcgcgccga 60
   tacgcgagcg aacgtgaa 78
<210> 7
   <211> 78
   <212> DNA
   <213> Artificial Sequence
<220>
   <221> source
   <223> /note="Description of Artificial Sequence: Synthetic oligonucleotide"
<400> 7
   gtcagtggca ggtctttctt gagctcagaa aggttaggta actagttcag gcggccgcct 60
   tagacgtcag gtggcact 78
<210> 8
   <211> 19
   <212> DNA
   <213> Artificial Sequence
<220>
   <221> source
   <223> /note="Description of Artificial Sequence: Synthetic primer"
<400> 8
   tttcttgtgc tggatgtag 19
<210> 9
   <211> 18
   <212> DNA
   <213> Artificial Sequence
<220>
   <221> source
   <223> /note="Description of Artificial Sequence: Synthetic primer"
<400> 9
   atatctctcc tggtggct 18
<210> 10
   <211> 24
   <212> DNA
   <213> Artificial Sequence
<220>
   <221> source
   <223> /note="Description of Artificial Sequence: Synthetic primer"
<400> 10
   tgggtgtcag gttcttgctt cagc 24
<210> 11
   <211> 19
   <212> DNA
   <213> Artificial Sequence
<220>
   <221> source
   <223> /note="Description of Artificial Sequence: Synthetic primer"
<400> 11
   cagtggatgc gcgcaggac 19
<210> 12
   <211> 17
   <212> DNA
   <213> Artificial Sequence
<220>
   <221> source
   <223> /note="Description of Artificial Sequence: Synthetic primer"
<400> 12
   tgagcagagg agtcaag 17
<210> 13
   <211> 17
   <212> DNA
   <213> Artificial Sequence
<220>
   <221> source
   <223> /note="Description of Artificial Sequence: Synthetic primer"
<400> 13
   tgtggtcaca gttcttg 17
<210> 14
   <211> 17
   <212> DNA
   <213> Artificial Sequence
<220>
   <221> source
   <223> /note="Description of Artificial Sequence: Synthetic probe"
<400> 14
   agctaaacat gctcaac 17
<210> 15
   <211> 18
   <212> DNA
   <213> Artificial Sequence
<220>
   <221> source
   <223> /note="Description of Artificial Sequence: Synthetic primer"
<400> 15
   cacgtgggct ccagcatt 18
<210> 16
   <211> 22
   <212> DNA
   <213> Artificial Sequence
<220>
   <221> source
   <223> /note="Description of Artificial Sequence: Synthetic primer"
<400> 16
   tcaccagtca tttctgcctt tg 22
<210> 17
   <211> 22
   <212> DNA
   <213> Artificial Sequence
<220>
   <221> source
   <223> /note="Description of Artificial Sequence: Synthetic probe"
<400> 17
   ccaatggtcg ggcactgctc aa 22
<210> 18
   <211> 1194
   <212> DNA
   <213> Artificial Sequence
<220>
   <221> source
   <223> /note="Description of Artificial Sequence: Synthetic polynucleotide"
<400> 18
   atggagggag ctgctttgct gagagtctct gtcctctgca tctggatgag tgcacttttc      60
   cttggtgtgg gagtgagggc agaggaagct ggagcgaggg tgcaacaaaa cgttccaagt     120
   gggacagata ctggagatcc tcaaagtaag cccctcggtg actgggctgc tggcaccatg     180
   gacccagaga gcagtatctt tattgaggat gccattaagt atttcaagga aaaagtgagc     240
   acacagaatc tgctactcct gctgactgat aatgaggcct ggaacggatt cgtggctgct     300
   gctgaactgc ccaggaatga ggcagatgag ctccgtaaag ctctggacaa ccttgcaaga     360
   caaatgatca tgaaagacaa aaactggcac gataaaggcc agcagtacag aaactggttt     420
   ctgaaagagt ttcctcggtt gaaaagtaag cttgaggata acataagaag gctccgtgcc     480
   cttgcagatg gggttcagaa ggtccacaaa ggcaccacca tcgccaatgt ggtgtctggc     540
   tctctcagca tttcctctgg catcctgacc ctcgtcggca tgggtctggc acccttcaca     600
   gagggaggca gccttgtact cttggaacct gggatggagt tgggaatcac agcagctttg     660
   accgggatta ccagtagtac catagactac ggaaagaagt ggtggacaca agcccaagcc     720
   cacgacctgg tcatcaaaag ccttgacaaa ttgaaggagg tgaaggagtt tttgggtgag     780
   aacatatcca actttctttc cttagctggc aatacttacc aactcacacg aggcattggg     840
   aaggacatcc gtgccctcag acgagccaga gccaatcttc agtcagtacc gcatgcctca     900
   gcctcacgcc cccgggtcac tgagccaatc tcagctgaaa gcggtgaaca ggtggagaga     960
   gttaatgaac ccagcatcct ggaaatgagc agaggagtca agctcacgga tgtggcccct    1020
   gtaagcttct ttcttgtgct ggatgtagtc tacctcgtgt acgaatcaaa gcacttacat    1080
   gagggggcaa agtcagagac agctgaggag ctgaagaagg tggctcagga gctggaggag    1140
   aagctaaaca ttctcaacaa taattataag attctgcagg cggaccaaga actg          1194
<210> 19
   <211> 7154
   <212> DNA
   <213> Artificial Sequence
<220>
   <221> source
   <223> /note="Description of Artificial Sequence: Synthetic polynucleotide"
<400> 19
   gtcgacattg attattgact agttattaat agtaatcaat tacggggtca ttagttcata      60
   gcccatatat ggagttccgc gttacataac ttacggtaaa tggcccgcct ggctgaccgc     120
   ccaacgaccc ccgcccattg acgtcaataa tgacgtatgt tcccatagta acgccaatag     180
   ggactttcca ttgacgtcaa tgggtggagt atttacggta aactgcccac ttggcagtac     240
   atcaagtgta tcatatgcca agtacgcccc ctattgacgt caatgacggt aaatggcccg     300
   cctggcatta tgcccagtac atgaccttat gggactttcc tacttggcag tacatctacg     360
   tattagtcat cgctattacc atggtcgagg tgagccccac gttctgcttc actctcccca     420
   tctccccccc ctccccaccc ccaattttgt atttatttat tttttaatta ttttgtgcag     480
   cgatgggggc gggggggggg ggggggcgcg cgccaggcgg ggcggggcgg ggcgaggggc     540
   ggggcggggc gaggcggaga ggtgcggcgg cagccaatca gagcggcgcg ctccgaaagt     600
   ttccttttat ggcgaggcgg cggcggcggc ggccctataa aaagcgaagc gcgcggcggg     660
   cgggagtcgc tgcgcgctgc cttcgccccg tgccccgctc cgccgccgcc tcgcgccgcc     720
   cgccccggct ctgactgacc gcgttactcc cacaggtgag cgggcgggac ggcccttctc     780
   ctccgggctg taattagcgc ttggtttaat gacggcttgt ttcttttctg tggctgcgtg     840
   aaagccttga ggggctccgg gagggccctt tgtgcggggg gagcggctcg gggggtgcgt     900
   gcgtgtgtgt gtgcgtgggg agcgccgcgt gcggctccgc gctgcccggc ggctgtgagc     960
   gctgcgggcg cggcgcgggg ctttgtgcgc tccgcagtgt gcgcgagggg agcgcggccg    1020
   ggggcggtgc cccgcggtgc ggggggggct gcgaggggaa caaaggctgc gtgcggggtg    1080
   tgtgcgtggg ggggtgagca gggggtgtgg gcgcgtcggt cgggctgcaa ccccccctgc    1140
   acccccctcc ccgagttgct gagcacggcc cggcttcggg tgcggggctc cgtacggggc    1200
   gtggcgcggg gctcgccgtg ccgggcgggg ggtggcggca ggtgggggtg ccgggcgggg    1260
   cggggccgcc tcgggccggg gagggctcgg gggaggggcg cggcggcccc cggagcgccg    1320
   gcggctgtcg aggcgcggcg agccgcagcc attgcctttt atggtaatcg tgcgagaggg    1380
   cgcagggact tcctttgtcc caaatctgtg cggagccgaa atctgggagg cgccgccgca    1440
   ccccctctag cgggcgcggg gcgaagcggt gcggcgccgg caggaaggaa atgggcgggg 1500
   agggccttcg tgcgtcgccg cgccgccgtc cccttctccc tctccagcct cggggctgtc 1560
   cgcgggggga cggctgcctt cgggggggac ggggcagggc ggggttcggc ttctggcgtg 1620
   tgaccggcgg ctctagagcc tctgctaacc atgttcatgc cttcttcttt ttcctacagc 1680
   tcctgggcaa cgtgctggtt gttgtgctgt ctcatcattt tggcaaagac ttcggtaccg 1740
   cgggcccggg atccaccatg gagggagctg ctttgctgag agtctctgtc ctctgcatct 1800
   ggatgagtgc acttttcctt ggtgtgggag tgagggcaga ggaagctgga gcgagggtgc 1860
   aacaaaacgt tccaagtggg acagatactg gagatcctca aagtaagccc ctcggtgact 1920
   gggctgctgg caccatggac ccagagagca gtatctttat tgaggatgcc attaagtatt 1980
   tcaaggaaaa agtgagcaca cagaatctgc tactcctgct gactgataat gaggcctgga 2040
   acggattcgt ggctgctgct gaactgccca ggaatgaggc agatgagctc cgtaaagctc 2100
   tggacaacct tgcaagacaa atgatcatga aagacaaaaa ctggcacgat aaaggccagc 2160
   agtacagaaa ctggtttctg aaagagtttc ctcggttgaa aagtaagctt gaggataaca 2220
   taagaaggct ccgtgccctt gcagatgggg ttcagaaggt ccacaaaggc accaccatcg 2280
   ccaatgtggt gtctggctct ctcagcattt cctctggcat cctgaccctc gtcggcatgg 2340
   gtctggcacc cttcacagag ggaggcagcc ttgtactctt ggaacctggg atggagttgg 2400
   gaatcacagc agctttgacc gggattacca gtagtaccat agactacgga aagaagtggt 2460
   ggacacaagc ccaagcccac gacctggtca tcaaaagcct tgacaaattg aaggaggtga 2520
   aggagttttt gggtgagaac atatccaact ttctttcctt agctggcaat acttaccaac 2580
   tcacacgagg cattgggaag gacatccgtg ccctcagacg agccagagcc aatcttcagt 2640
   cagtaccgca tgcctcagcc tcacgccccc gggtcactga gccaatctca gctgaaagcg 2700
   gtgaacaggt ggagagagtt aatgaaccca gcatcctgga aatgagcaga ggagtcaagc 2760
   tcacggatgt ggcccctgta agcttctttc ttgtgctgga tgtagtctac ctcgtgtacg 2820
   aatcaaagca cttacatgag ggggcaaagt cagagacagc tgaggagctg aagaaggtgg 2880
   ctcaggagct ggaggagaag ctaaacattc tcaacaataa ttataagatt ctgcaggcgg 2940
   accaagaact gtgagggaat tcgtgagcgg ccgcatgatc agctggatgc atcgatcacg 3000
   cgtaccggtg ctcgaggtac cgatgaatag ctaaggtcga ggccgcaggt aagtatcaag 3060
   gttacaagac aggtttaagg agaccaatag aaactgggct tgtcgagaca gagaagactc 3120
   ttgcgtttct gataggcacc tattggtctt actgacatcc actttgcctt tctctccaca 3180
   ggtgtcgaca atcaacctct ggattacaaa atttgtgaaa gattgactgg tattcttaac 3240
   tatgttgctc cttttacgct atgtggatac gctgctttaa tgcctttgta tcatgctatt 3300
   gcttcccgta tggctttcat tttctcctcc ttgtataaat cctggttgct gtctctttat 3360
   gaggagttgt ggcccgttgt caggcaacgt ggcgtggtgt gcactgtgtt tgctgacgca 3420
   acccccactg gttggggcat tgccaccacc tgtcagctcc tttccgggac tttcgctttc 3480
   cccctcccta ttgccacggc ggaactcatc gccgcctgcc ttgcccgctg ctggacaggg 3540
   gctcggctgt tgggcactga caattccgtg gtgttgtcgg ggaagctgac gtcctttcca 3600
   tggctgctcg cctgtgttgc cacctggatt ctgcgcggga cgtccttctg ctacgtccct 3660
   tcggccctca atccagcgga ccttccttcc cgcggcctgc tgccggctct gcggcctctt 3720
   ccgcgtcttc gccttcgccc tcagacgagt cggatctccc tttgggccgc ctccccgcct 3780
   ggacttcgag ctcggtacga tcagcctcga ctgtgccttc tagttgccag ccatctgttg 3840
   tttgcccctc ccccgtgcct tccttgaccc tggaaggtgc cactcccact gtcctttcct 3900
   aataaaatga ggaaattgca tcgcattgtc tgagtaggtg tcattctatt ctggggggtg 3960
   gggtggggca ggacagcaag ggggaggatt gggaagacaa tagcccagct tttgttccct 4020
   ttagtgaggg ttaattgcgc gcttggcgta atcatggtca tagctgtttc ctgtgtgaaa 4080
   ttgttatccg ctaattcact cctcaggtgc aggctgccta tcagaaggtg gtggctggtg 4140
   tggccaatgc cctggctcac aaataccact gagatctttt tccctctgcc aaaaattatg 4200
   gggacatcat gaagcccctt gagcatctga cttctggcta ataaaggaaa tttattttca 4260
   ttgcaatagt gtgttggaat tttttgtgtc tctcactcgg aaggacatat gggagggcaa 4320
   atcatttaaa acatcagaat gagtatttgg tttagagttt ggcaacatat gcccatatgc 4380
   tggctgccat gaacaaaggt tggctataaa gaggtcatca gtatatgaaa cagccccctg 4440
   ctgtccattc cttattccat agaaaagcct tgacttgagg ttagattttt tttatatttt 4500
   gttttgtgtt atttttttct ttaacatccc taaaattttc cttacatgtt ttactagcca 4560
   gatttttcct cctctcctga ctactcccag tcatagctgt ccctcttctc ttatggagat 4620
   ccctcgacct gcagcccaag cttggcgtaa tcatggtcat agctgtttcc tgtgtgaaat 4680
   tgttatccgc tcacaattcc acacaacata cgagccggaa gcataaagtg taaagcctgg 4740
   ggtgcctaat gagtgagcta actcacatta attgcgttgc gctcactgcc cgctttccag 4800
   tcgggaaacc tgtcgtgcca gcggaaccgc atctcaatta gtcagcaacc atagtcccgc 4860
   ccctaactcc gcccatcccg cccctaactc cgcccagttc cgcccattct ccgccccatg 4920
   gctgactaat tttttttatt tatgcagagg ccgaggccgc ctcggcctct gagctattcc 4980
   agaagtagtg aggaggcttt tttggaggcc taggcttttg caaaaagcta acttgtttat 5040
   tgcagcttat aatggttaca aataaagcaa tagcatcaca aatttcacaa ataaagcatt 5100
   tttttcactg cattctagtt gtggtttgtc caaactcatc aatgtatctt atcatgtctg 5160
   gaaccgctgc attaatgaat cggccaacgc gcggggagag gcggtttgcg tattgggcgc 5220
   tcttccgctt cctcgctcac tgactcgctg cgctcggtcg ttcggctgcg gcgagcggta 5280
   tcagctcact caaaggcggt aatacggtta tccacagaat caggggataa cgcaggaaag 5340
   aacatgtgag caaaaggcca gcaaaaggcc aggaaccgta aaaaggccgc gttgctggcg 5400
   tttttccata ggctccgccc ccctgacgag catcacaaaa atcgacgctc aagtcagagg 5460
   tggcgaaacc cgacaggact ataaagatac caggcgtttc cccctggaag ctccctcgtg 5520
   cgctctcctg ttccgaccct gccgcttacc ggatacctgt ccgcctttct cccttcggga 5580
   agcgtggcgc tttctcatag ctcacgctgt aggtatctca gttcggtgta ggtcgttcgc 5640
   tccaagctgg gctgtgtgca cgaacccccc gttcagcccg accgctgcgc cttatccggt 5700
   aactatcgtc ttgagtccaa cccggtaaga cacgacttat cgccactggc agcagccact 5760
   ggtaacagga ttagcagagc gaggtatgta ggcggtgcta cagagttctt gaagtggtgg 5820
   cctaactacg gctacactag aagaacagta tttggtatct gcgctctgct gaagccagtt 5880
   accttcggaa aaagagttgg tagctcttga tccggcaaac aaaccaccgc tggtagcggt 5940
   ggtttttttg tttgcaagca gcagattacg cgcagaaaaa aaggatctca agaagatcct 6000
   ttgatctttt ctacggggtc tgacgctcag tggaacgaaa actcacgtta agggattttg 6060
   gtcatgagat tatcaaaaag gatcttcacc tagatccttt taaattaaaa atgaagtttt 6120
   aaatcaatct aaagtatata tgagtaaact tggtctgaca gttaccaatg cttaatcagt 6180
   gaggcaccta tctcagcgat ctgtctattt cgttcatcca tagttgcctg actccccgtc 6240
   gtgtagataa ctacgatacg ggagggctta ccatctggcc ccagtgctgc aatgataccg 6300
   cgagacccac gctcaccggc tccagattta tcagcaataa accagccagc cggaagggcc 6360
   gagcgcagaa gtggtcctgc aactttatcc gcctccatcc agtctattaa ttgttgccgg 6420
   gaagctagag taagtagttc gccagttaat agtttgcgca acgttgttgc cattgctaca 6480
   ggcatcgtgg tgtcacgctc gtcgtttggt atggcttcat tcagctccgg ttcccaacga 6540
   tcaaggcgag ttacatgatc ccccatgttg tgcaaaaaag cggttagctc cttcggtcct 6600
   ccgatcgttg tcagaagtaa gttggccgca gtgttatcac tcatggttat ggcagcactg 6660
   cataattctc ttactgtcat gccatccgta agatgctttt ctgtgactgg tgagtactca 6720
   accaagtcat tctgagaata gtgtatgcgg cgaccgagtt gctcttgccc ggcgtcaata 6780
   cgggataata ccgcgccaca tagcagaact ttaaaagtgc tcatcattgg aaaacgttct 6840
   tcggggcgaa aactctcaag gatcttaccg ctgttgagat ccagttcgat gtaacccact 6900
   cgtgcaccca actgatcttc agcatctttt actttcacca gcgtttctgg gtgagcaaaa 6960
   acaggaaggc aaaatgccgc aaaaaaggga ataagggcga cacggaaatg ttgaatactc 7020
   atactcttcc tttttcaata ttattgaagc atttatcagg gttattgtct catgagcgga 7080
   tacatatttg aatgtattta gaaaaataaa caaatagggg ttccgcgcac atttccccga 7140
   aaagtgccac ctgg 7154
<210> 20
   <211> 1194
   <212> DNA
   <213> Artificial Sequence
<220>
   <221> source
   <223> /note="Description of Artificial Sequence: Synthetic polynucleotide"
<400> 20
   atggagggag ctgctttgct gagagtctct gtcctctgca tctggatgag tgcacttttc 60
   cttggtgtgg gagtgagggc agaggaagct ggagcgaggg tgcaacaaaa cgttccaagt 120
   gggacagata ctggagatcc tcaaagtaag cccctcggtg actgggctgc tggcaccatg 180
   gacccagaga gcagtatctt tattgaggat gccattaagt atttcaagga aaaagtgagc 240
   acacagaatc tgctactcct gctgactgat aatgaggcct ggaacggatt cgtggctgct 300
   gctgaactgc ccaggaatga ggcagatgag ctccgtaaag ctctggacaa ccttgcaaga 360
   caaatgatca tgaaagacaa aaactggcac gataaaggcc agcagtacag aaactggttt 420
   ctgaaagagt ttcctcggtt gaaaagtaag cttgaggata acataagaag gctccgtgcc 480
   cttgcagatg gggttcagaa ggtccacaaa ggcaccacca tcgccaatgt ggtgtctggc 540
   tctctcagca tttcctctgg catcctgacc ctcgtcggca tgggtctggc acccttcaca 600
   gagggaggca gccttgtact cttggaacct gggatggagt tgggaatcac agcagctttg 660
   accgggatta ccagtagtac catagactac ggaaagaagt ggtggacaca agcccaagcc 720
   cacgacctgg tcatcaaaag ccttgacaaa ttgaaggagg tgaaggagtt tttgggtgag 780
   aacatatcca actttctttc cttagctggc aatacttacc aactcacacg aggcattggg 840
   aaggacatcc gtgccctcag acgagccaga gccaatcttc agtcagtacc gcatgcctca 900
   gcctcacgcc cccgggtcac tgagccaatc tcagctgaaa gcggtgaaca ggtggagaga 960
   gttaatgaac ccagcatcct ggaaatgagc agaggagtca agctcacgga tgtggcccct 1020
   gtaggcttct ttcttgtgct ggatgtagtc tacctcgtgt acgaatcaaa gcacttacat 1080
   gagggggcaa agtcagagac agctgaggag ctgaagaagg tggctcagga gctggaggag 1140
   aagctaaaca tgctcaacaa taattataag attctgcagg cggaccaaga actg 1194
<210> 21
   <211> 7154
   <212> DNA
   <213> Artificial Sequence
<220>
   <221> source
   <223> /note="Description of Artificial Sequence: Synthetic polynucleotide"
<400> 21
   gtcgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtacgcccc ctattgacgt caatgacggt aaatggcccg 300
   cctggcatta tgcccagtac atgaccttat gggactttcc tacttggcag tacatctacg 360
   tattagtcat cgctattacc atggtcgagg tgagccccac gttctgcttc actctcccca 420
   tctccccccc ctccccaccc ccaattttgt atttatttat tttttaatta ttttgtgcag 480
   cgatgggggc gggggggggg ggggggcgcg cgccaggcgg ggcggggcgg ggcgaggggc 540
   ggggcggggc gaggcggaga ggtgcggcgg cagccaatca gagcggcgcg ctccgaaagt 600
   ttccttttat ggcgaggcgg cggcggcggc ggccctataa aaagcgaagc gcgcggcggg 660
   cgggagtcgc tgcgcgctgc cttcgccccg tgccccgctc cgccgccgcc tcgcgccgcc 720
   cgccccggct ctgactgacc gcgttactcc cacaggtgag cgggcgggac ggcccttctc 780
   ctccgggctg taattagcgc ttggtttaat gacggcttgt ttcttttctg tggctgcgtg 840
   aaagccttga ggggctccgg gagggccctt tgtgcggggg gagcggctcg gggggtgcgt 900
   gcgtgtgtgt gtgcgtgggg agcgccgcgt gcggctccgc gctgcccggc ggctgtgagc 960
   gctgcgggcg cggcgcgggg ctttgtgcgc tccgcagtgt gcgcgagggg agcgcggccg 1020
   ggggcggtgc cccgcggtgc ggggggggct gcgaggggaa caaaggctgc gtgcggggtg 1080
   tgtgcgtggg ggggtgagca gggggtgtgg gcgcgtcggt cgggctgcaa ccccccctgc 1140
   acccccctcc ccgagttgct gagcacggcc cggcttcggg tgcggggctc cgtacggggc 1200
   gtggcgcggg gctcgccgtg ccgggcgggg ggtggcggca ggtgggggtg ccgggcgggg 1260
   cggggccgcc tcgggccggg gagggctcgg gggaggggcg cggcggcccc cggagcgccg 1320
   gcggctgtcg aggcgcggcg agccgcagcc attgcctttt atggtaatcg tgcgagaggg 1380
   cgcagggact tcctttgtcc caaatctgtg cggagccgaa atctgggagg cgccgccgca 1440
   ccccctctag cgggcgcggg gcgaagcggt gcggcgccgg caggaaggaa atgggcgggg 1500
   agggccttcg tgcgtcgccg cgccgccgtc cccttctccc tctccagcct cggggctgtc 1560
   cgcgggggga cggctgcctt cgggggggac ggggcagggc ggggttcggc ttctggcgtg 1620
   tgaccggcgg ctctagagcc tctgctaacc atgttcatgc cttcttcttt ttcctacagc 1680
   tcctgggcaa cgtgctggtt gttgtgctgt ctcatcattt tggcaaagac ttcggtaccg 1740
   cgggcccggg atccaccatg gagggagctg ctttgctgag agtctctgtc ctctgcatct 1800
   ggatgagtgc acttttcctt ggtgtgggag tgagggcaga ggaagctgga gcgagggtgc 1860
   aacaaaacgt tccaagtggg acagatactg gagatcctca aagtaagccc ctcggtgact 1920
   gggctgctgg caccatggac ccagagagca gtatctttat tgaggatgcc attaagtatt 1980
   tcaaggaaaa agtgagcaca cagaatctgc tactcctgct gactgataat gaggcctgga 2040
   acggattcgt ggctgctgct gaactgccca ggaatgaggc agatgagctc cgtaaagctc 2100
   tggacaacct tgcaagacaa atgatcatga aagacaaaaa ctggcacgat aaaggccagc 2160
   agtacagaaa ctggtttctg aaagagtttc ctcggttgaa aagtaagctt gaggataaca 2220
   taagaaggct ccgtgccctt gcagatgggg ttcagaaggt ccacaaaggc accaccatcg 2280
   ccaatgtggt gtctggctct ctcagcattt cctctggcat cctgaccctc gtcggcatgg 2340
   gtctggcacc cttcacagag ggaggcagcc ttgtactctt ggaacctggg atggagttgg 2400
   gaatcacagc agctttgacc gggattacca gtagtaccat agactacgga aagaagtggt 2460
   ggacacaagc ccaagcccac gacctggtca tcaaaagcct tgacaaattg aaggaggtga 2520
   aggagttttt gggtgagaac atatccaact ttctttcctt agctggcaat acttaccaac 2580
   tcacacgagg cattgggaag gacatccgtg ccctcagacg agccagagcc aatcttcagt 2640
   cagtaccgca tgcctcagcc tcacgccccc gggtcactga gccaatctca gctgaaagcg 2700
   gtgaacaggt ggagagagtt aatgaaccca gcatcctgga aatgagcaga ggagtcaagc 2760
   tcacggatgt ggcccctgta ggcttctttc ttgtgctgga tgtagtctac ctcgtgtacg 2820
   aatcaaagca cttacatgag ggggcaaagt cagagacagc tgaggagctg aagaaggtgg 2880
   ctcaggagct ggaggagaag ctaaacatgc tcaacaataa ttataagatt ctgcaggcgg 2940
   accaagaact gtgagggaat tcgtgagcgg ccgcatgatc agctggatgc atcgatcacg 3000
   cgtaccggtg ctcgaggtac cgatgaatag ctaaggtcga ggccgcaggt aagtatcaag 3060
   gttacaagac aggtttaagg agaccaatag aaactgggct tgtcgagaca gagaagactc 3120
   ttgcgtttct gataggcacc tattggtctt actgacatcc actttgcctt tctctccaca 3180
   ggtgtcgaca atcaacctct ggattacaaa atttgtgaaa gattgactgg tattcttaac 3240
   tatgttgctc cttttacgct atgtggatac gctgctttaa tgcctttgta tcatgctatt 3300
   gcttcccgta tggctttcat tttctcctcc ttgtataaat cctggttgct gtctctttat 3360
   gaggagttgt ggcccgttgt caggcaacgt ggcgtggtgt gcactgtgtt tgctgacgca 3420
   acccccactg gttggggcat tgccaccacc tgtcagctcc tttccgggac tttcgctttc 3480
   cccctcccta ttgccacggc ggaactcatc gccgcctgcc ttgcccgctg ctggacaggg 3540
   gctcggctgt tgggcactga caattccgtg gtgttgtcgg ggaagctgac gtcctttcca 3600
   tggctgctcg cctgtgttgc cacctggatt ctgcgcggga cgtccttctg ctacgtccct 3660
   tcggccctca atccagcgga ccttccttcc cgcggcctgc tgccggctct gcggcctctt 3720
   ccgcgtcttc gccttcgccc tcagacgagt cggatctccc tttgggccgc ctccccgcct 3780
   ggacttcgag ctcggtacga tcagcctcga ctgtgccttc tagttgccag ccatctgttg 3840
   tttgcccctc ccccgtgcct tccttgaccc tggaaggtgc cactcccact gtcctttcct 3900
   aataaaatga ggaaattgca tcgcattgtc tgagtaggtg tcattctatt ctggggggtg 3960
   gggtggggca ggacagcaag ggggaggatt gggaagacaa tagcccagct tttgttccct 4020
   ttagtgaggg ttaattgcgc gcttggcgta atcatggtca tagctgtttc ctgtgtgaaa 4080
   ttgttatccg ctaattcact cctcaggtgc aggctgccta tcagaaggtg gtggctggtg 4140
   tggccaatgc cctggctcac aaataccact gagatctttt tccctctgcc aaaaattatg 4200
   gggacatcat gaagcccctt gagcatctga cttctggcta ataaaggaaa tttattttca 4260
   ttgcaatagt gtgttggaat tttttgtgtc tctcactcgg aaggacatat gggagggcaa 4320
   atcatttaaa acatcagaat gagtatttgg tttagagttt ggcaacatat gcccatatgc 4380
   tggctgccat gaacaaaggt tggctataaa gaggtcatca gtatatgaaa cagccccctg 4440
   ctgtccattc cttattccat agaaaagcct tgacttgagg ttagattttt tttatatttt 4500
   gttttgtgtt atttttttct ttaacatccc taaaattttc cttacatgtt ttactagcca 4560
   gatttttcct cctctcctga ctactcccag tcatagctgt ccctcttctc ttatggagat 4620
   ccctcgacct gcagcccaag cttggcgtaa tcatggtcat agctgtttcc tgtgtgaaat 4680
   tgttatccgc tcacaattcc acacaacata cgagccggaa gcataaagtg taaagcctgg 4740
   ggtgcctaat gagtgagcta actcacatta attgcgttgc gctcactgcc cgctttccag 4800
   tcgggaaacc tgtcgtgcca gcggaaccgc atctcaatta gtcagcaacc atagtcccgc 4860
   ccctaactcc gcccatcccg cccctaactc cgcccagttc cgcccattct ccgccccatg 4920
   gctgactaat tttttttatt tatgcagagg ccgaggccgc ctcggcctct gagctattcc 4980
   agaagtagtg aggaggcttt tttggaggcc taggcttttg caaaaagcta acttgtttat 5040
   tgcagcttat aatggttaca aataaagcaa tagcatcaca aatttcacaa ataaagcatt 5100
   tttttcactg cattctagtt gtggtttgtc caaactcatc aatgtatctt atcatgtctg 5160
   gaaccgctgc attaatgaat cggccaacgc gcggggagag gcggtttgcg tattgggcgc 5220
   tcttccgctt cctcgctcac tgactcgctg cgctcggtcg ttcggctgcg gcgagcggta 5280
   tcagctcact caaaggcggt aatacggtta tccacagaat caggggataa cgcaggaaag 5340
   aacatgtgag caaaaggcca gcaaaaggcc aggaaccgta aaaaggccgc gttgctggcg 5400
   tttttccata ggctccgccc ccctgacgag catcacaaaa atcgacgctc aagtcagagg 5460
   tggcgaaacc cgacaggact ataaagatac caggcgtttc cccctggaag ctccctcgtg 5520
   cgctctcctg ttccgaccct gccgcttacc ggatacctgt ccgcctttct cccttcggga 5580
   agcgtggcgc tttctcatag ctcacgctgt aggtatctca gttcggtgta ggtcgttcgc 5640
   tccaagctgg gctgtgtgca cgaacccccc gttcagcccg accgctgcgc cttatccggt 5700
   aactatcgtc ttgagtccaa cccggtaaga cacgacttat cgccactggc agcagccact 5760
   ggtaacagga ttagcagagc gaggtatgta ggcggtgcta cagagttctt gaagtggtgg 5820
   cctaactacg gctacactag aagaacagta tttggtatct gcgctctgct gaagccagtt 5880
   accttcggaa aaagagttgg tagctcttga tccggcaaac aaaccaccgc tggtagcggt 5940
   ggtttttttg tttgcaagca gcagattacg cgcagaaaaa aaggatctca agaagatcct 6000
   ttgatctttt ctacggggtc tgacgctcag tggaacgaaa actcacgtta agggattttg 6060
   gtcatgagat tatcaaaaag gatcttcacc tagatccttt taaattaaaa atgaagtttt 6120
   aaatcaatct aaagtatata tgagtaaact tggtctgaca gttaccaatg cttaatcagt 6180
   gaggcaccta tctcagcgat ctgtctattt cgttcatcca tagttgcctg actccccgtc 6240
   gtgtagataa ctacgatacg ggagggctta ccatctggcc ccagtgctgc aatgataccg 6300
   cgagacccac gctcaccggc tccagattta tcagcaataa accagccagc cggaagggcc 6360
   gagcgcagaa gtggtcctgc aactttatcc gcctccatcc agtctattaa ttgttgccgg 6420
   gaagctagag taagtagttc gccagttaat agtttgcgca acgttgttgc cattgctaca 6480
   ggcatcgtgg tgtcacgctc gtcgtttggt atggcttcat tcagctccgg ttcccaacga 6540
   tcaaggcgag ttacatgatc ccccatgttg tgcaaaaaag cggttagctc cttcggtcct 6600
   ccgatcgttg tcagaagtaa gttggccgca gtgttatcac tcatggttat ggcagcactg 6660
   cataattctc ttactgtcat gccatccgta agatgctttt ctgtgactgg tgagtactca 6720
   accaagtcat tctgagaata gtgtatgcgg cgaccgagtt gctcttgccc ggcgtcaata 6780
   cgggataata ccgcgccaca tagcagaact ttaaaagtgc tcatcattgg aaaacgttct 6840
   tcggggcgaa aactctcaag gatcttaccg ctgttgagat ccagttcgat gtaacccact 6900
   cgtgcaccca actgatcttc agcatctttt actttcacca gcgtttctgg gtgagcaaaa 6960
   acaggaaggc aaaatgccgc aaaaaaggga ataagggcga cacggaaatg ttgaatactc 7020
   atactcttcc tttttcaata ttattgaagc atttatcagg gttattgtct catgagcgga 7080
   tacatatttg aatgtattta gaaaaataaa caaatagggg ttccgcgcac atttccccga 7140
   aaagtgccac ctgg 7154
<210> 22
   <211> 1188
   <212> DNA
   <213> Artificial Sequence
<220>
   <221> source
   <223> /note="Description of Artificial Sequence: Synthetic polynucleotide"
<400> 22
   atggagggag ctgctttgct gagagtctct gtcctctgca tctggatgag tgcacttttc      60
   cttggtgtgg gagtgagggc agaggaagct ggagcgaggg tgcaacaaaa cgttccaagt     120
   gggacagata ctggagatcc tcaaagtaag cccctcggtg actgggctgc tggcaccatg     180
   gacccagaga gcagtatctt tattgaggat gccattaagt atttcaagga aaaagtgagc     240
   acacagaatc tgctactcct gctgactgat aatgaggcct ggaacggatt cgtggctgct     300
   gctgaactgc ccaggaatga ggcagatgag ctccgtaaag ctctggacaa ccttgcaaga     360
   caaatgatca tgaaagacaa aaactggcac gataaaggcc agcagtacag aaactggttt     420
   ctgaaagagt ttcctcggtt gaaaagtaag cttgaggata acataagaag gctccgtgcc     480
   cttgcagatg gggttcagaa ggtccacaaa ggcaccacca tcgccaatgt ggtgtctggc     540
   tctctcagca tttcctctgg catcctgacc ctcgtcggca tgggtctggc acccttcaca     600
   gagggaggca gccttgtact cttggaacct gggatggagt tgggaatcac agcagctttg     660
   accgggatta ccagtagtac catagactac ggaaagaagt ggtggacaca agcccaagcc     720
   cacgacctgg tcatcaaaag ccttgacaaa ttgaaggagg tgaaggagtt tttgggtgag     780
   aacatatcca actttctttc cttagctggc aatacttacc aactcacacg aggcattggg     840
   aaggacatcc gtgccctcag acgagccaga gccaatcttc agtcagtacc gcatgcctca     900
   gcctcacgcc cccgggtcac tgagccaatc tcagctgaaa gcggtgaaca ggtggagaga     960
   gttaatgaac ccagcatcct ggaaatgagc agaggagtca agctcacgga tgtggcccct    1020
   gtaagcttct ttcttgtgct ggatgtagtc tacctcgtgt acgaatcaaa gcacttacat    1080
   gagggggcaa agtcagagac agctgaggag ctgaagaagg tggctcagga gctggaggag    1140
   aagctaaaca ttctcaacaa taagattctg caggcggacc aagaactg                  1188
<210> 23
   <211> 7148
   <212> DNA
   <213> Artificial Sequence
<220>
   <221> source
   <223> /note="Description of Artificial Sequence: Synthetic polynucleotide"
<400> 23
<210> 24
   <211> 357
   <212> PRT
   <213> Artificial Sequence
<220>
   <221> source
   <223> /note="Description of Artificial Sequence: Synthetic polypeptide"
<400> 24
   Met His His His His His His Gly Glu Asn Leu Tyr Phe Gln Gly Ser
   1               5                   10                  15
   Asp Pro Glu Ser Ser Ile Phe Ile Glu Asp Ala Ile Lys Tyr Phe Lys
               20                  25                  30
   Glu Lys Val Ser Thr Gln Asn Leu Leu Leu Leu Leu Thr Asp Asn Glu
           35                  40                  45
   Ala Trp Asn Gly Phe Val Ala Ala Ala Glu Leu Pro Arg Asn Glu Ala
       50                  55                  60
   Asp Glu Leu Arg Lys Ala Leu Asp Asn Leu Ala Arg Gln Met Ile Met
   65                  70                  75                  80
   Lys Asp Lys Asn Trp His Asp Lys Gly Gln Gln Tyr Arg Asn Trp Phe
                   85                  90                  95
   Leu Lys Glu Phe Pro Arg Leu Lys Ser Glu Leu Glu Asp Asn Ile Arg
               100                 105                 110
   Arg Leu Arg Ala Leu Ala Asp Gly Val Gln Lys Val His Lys Gly Thr
           115                 120                 125
   Thr Ile Ala Asn Val Val Ser Gly Ser Leu Ser Ile Ser Ser Gly Ile
       130                 135                 140
   Leu Thr Leu Val Gly Met Gly Leu Ala Pro Phe Thr Glu Gly Gly Ser
   145                 150                 155                 160
   Leu Val Leu Leu Glu Pro Gly Met Glu Leu Gly Ile Thr Ala Ala Leu
                   165                 170                 175
   Thr Gly Ile Thr Ser Ser Thr Met Asp Tyr Gly Lys Lys Trp Trp Thr
               180                 185                 190
   Gln Ala Gln Ala His Asp Leu Val Ile Lys Ser Leu Asp Lys Leu Lys
           195                 200                 205
   Glu Val Arg Glu Phe Leu Gly Glu Asn Ile Ser Asn Phe Leu Ser Leu
       210                 215                 220
   Ala Gly Asn Thr Tyr Gln Leu Thr Arg Gly Ile Gly Lys Asp Ile Arg
   225                 230                 235                 240
   Ala Leu Arg Arg Ala Arg Ala Asn Leu Gln Ser Val Pro His Ala Ser
                   245                 250                 255
   Ala Ser Arg Pro Arg Val Thr Glu Pro Ile Ser Ala Glu Ser Gly Glu
               260                 265                 270
   Gln Val Glu Arg Val Asn Glu Pro Ser Ile Leu Glu Met Ser Arg Gly
                       275                 280                 285
   Val Lys Leu Thr Asp Val Ala Pro Val Ser Phe Phe Leu Val Leu Asp
       290                 295                 300
   Val Val Tyr Leu Val Tyr Glu Ser Lys His Leu His Glu Gly Ala Lys
   305                 310                 315                 320
   Ser Glu Thr Ala Glu Glu Leu Lys Lys Val Ala Gln Glu Leu Glu Glu
                   325                 330                 335
   Lys Leu Asn Ile Leu Asn Asn Asn Tyr Lys Ile Leu Gln Ala Asp Gln
               340                 345                 350
   Glu Leu Gly Asn Ser
           355